# EUROPEAN PATENT SPECIFICATION

(11) **EP 3 397 051 B1**
(45) Date of publication and mention of the grant of the patent: **13.04.2022**
(21) Application number: 16882638.6
(22) Date of filing: 29.12.2016
(51) Int. Cl.: A01K 67/033, C07K 14/82, G01N 33/50, A61K 31/405, A61K 31/519, C12N 9/16

(54) **FLY AVATARS FOR CANCER AND USES THEREOF**
FLIEGENAVATARE FÜR KREBS UND VERWENDUNGEN DAVON
AVATARS DE MOUCHE DESTINÉS AU TRAITEMENT DU CANCER ET LEURS UTILISATIONS

(30) Priority: 30.12.2015 US 201562273125 P
(43) Date of publication of application: 07.11.2018
(73) Proprietor: Icahn School of Medicine at Mount Sinai, New York, NY 10029 (US)
(72) Inventor: CAGAN, Ross, New York, NY 10075 (US); BANGI, Erdem, New York, NY 10029 (US)
(74) Representative: Potter Clarkson
(86) International application number: PCT/US2016/069104
(87) International publication number: WO 2017/117344

(56) References cited:
- WO-A1-2009/055461
- WO-A1-2015/026860
- WO-A2-2006/009903
- WO-A2-2014/178012
- US-A1- 2002 026 648
- US-A1- 2006 010 505
- US-A1- 2013 136 694
- US-A1- 2015 143 553
- US-B1- 6 531 644
- YUMI KASAI ET AL: "Drosophila as a tool for personalized medicine: a primer", PERSONALIZED MEDICINE, vol. 7, no. 6, 1 November 2010 (2010-11-01), pages 621-632, XP055396072, GB ISSN: 1741-0541, DOI: 10.2217/pme.10.65
- L. F. WILLOUGHBY ET AL: "An in vivo large-scale chemical screening platform using Drosophila for anti-cancer drug discovery", DISEASE MODELS & MECHANISMS, vol. 6, no. 2, 20 September 2012 (2012-09-20), pages 521-529, XP055584430, GB ISSN: 1754-8403, DOI: 10.1242/dmm.009985
- VIVEK A. RUDRAPATNA ET AL: "Drosophila cancer models : Drosophila Cancer Models", DEVELOPMENTAL DYNAMICS., vol. 241, no. 1, 1 January 2012 (2012-01-01), pages 107-118, XP055584436, US ISSN: 1058-8388, DOI: 10.1002/dvdy.22771
- ERDEM BANGI ET AL: "Functional exploration of colorectal cancer genomes using Drosophila", NATURE COMMUNICATIONS, vol. 7, no. 1, 29 November 2016 (2016-11-29), pages 1-15, XP055584432, DOI: 10.1038/ncomms13615
- BANGI ERDEM ET AL: "A Drosophila approach to personalized cancer therapeutics", CLINICAL CANCER RESEARCH, vol. 22, no. Suppl. 1, January 2016 (2016-01), page 34, XP002790935, & MEETING OF THE AMERICAN-ASSOCIATION-FOR-CANCER-RESEARCH (AACR) PRECISION MEDICINE SERIES - INTEGRATI; SALT LAKE, UT, USA; JUNE 13 -16, 2015
- KASAI ET AL.: 'Drosophila as a tool for personalized medicine: a primer' PERSONALIZED MEDICINE vol. 7, no. 6, 30 November 2010, pages 621 - 632, XP055396072

## Description

This invention was made with government support under grant nos. R01-CA109730, R01-CA170495 and R01-CA084309 awarded by the National Institutes of Health. The government has certain rights in the invention.

### 1. INTRODUCTION

The invention relates to improved methods for treating a human subject diagnosed with cancer using combination drug regimens tailored to the genome/proteome/phenome of the subject's tumor/cancer. The treatment regimens are selected and/or their efficacy is confirmed using an animal model "avatar," preferably a transgenic *Drosophila* avatar, of the genome/proteome/phenome of the subject. In certain embodiments, "avatar armies" representing the genomes/proteomes/phenomes of a population of patients diagnosed with a disease or disorder can be used to screen and select therapeutic regimens for treatment and/or to screen for new lead compounds and identify new therapeutics for the disease or disorder.

### 2. BACKGROUND OF THE INVENTION

Genetic techniques available in the fruit fly make it an attractive model to study genetics. (The terms "fruit fly," "fly" and *"Drosophila"* are used synonymously herein). The fly genome has been completely sequenced and a repertoire of genetic tools is available to generate transgenics. The fly's very rapid life cycle allows for the production of transgenic founder lines in approximately 6 weeks. Because the adult fly has organs that perform the equivalent functions of the mammalian heart, lung, kidney, gut and reproductive tract, as well as a sophisticated brain and visual system, transgenic flies have been developed as model systems for studying developmental biology as well as a variety of diseases. For example, in cancer, transgenic *Drosophila* engineered to misexpress suspected target gene(s) allowed the study of invasion and tumor growth in fly organs, such as the eye or wing (Pagliarini & Xu, 2003, Science 302:1227-1231; Vidal et al., 2006, Dev. Cell 10: 33-44; Vidal et al., 2007, Cancer Res. 67:10278-10285; Humbert et al., 2008, Oncogene 27: 6888-6907 (review); Wu et al., 2010, Nature 463:545-549; Das & Cagan, 2010, Thyroid 20: 689-695; Das & Cagan, 2012, Drug Discov. Today Technol. 10: e65-e71).

However, *Drosophila* lack certain vertebrate features that limit its use as a cancer model. For example, while *Drosophila* have innate immunity (the non-specific immune response used as a first line of defense against pathogens), they lack an adaptive/acquired immune system that vertebrates possess for developing immunological memory and the cellular responses involved in immune checkpoints that control or limit tumor growth. Additionally, *Drosophila* do not have a vascular circulatory system, which limits the study of factors affecting tumor angiogenesis and growth.

Because of these differences, the use of *Drosophila* has been limited to models for the study of tumorigenesis and metastasis in fly organs, such as the developing eye or wing (Pagliarini & Xu, 2003, *supra;* Vidal et al., 2006 and 2007, *supra;* Humbert et al., 2008 review, *supra;* Wu et al., 2010, *supra;* Das & Cagan, 2010 and 2012, *supra*); drug discovery programs using targeted expression of genes implicated in cancer to screen compounds that modify a "screenably distinct" feature in the targeted organ, such as retinal defects in the fly (Baranski & Cagan, US 2006/0010505); or *Drosophila* cell-culture assays used as part of drug screening programs (Perrimon et al., 2007, Drug Discovery Today 12: 28-33). For a review, *see* Pandey & Nichols, 2011, Pharmacological Reviews 63: 411-436.

Vertebrate animal models continue to be the mainstay for developing or testing regimens for the treatment of cancer in human subjects. But these models are also limited. For example, human tumor xenografts injected into nude mice do not recapitulate tumorigenesis or metastasis in the subject, and the nude mouse lacks the adaptive/acquired immune system that plays a role in controlling tumor growth.

Mouse models built to reflect commonly observed mutation combinations in human tumors have begun to shed light on interactions between individual mutations and pathways (Frese and Tuveson, 2007, "Maximizing mouse cancer models," Nat Rev Cancer, 7(9):645-58; Politi and Pao, 2011, "How genetically engineered mouse tumor models provide insights into human cancers," J Clin Oncol, 29(16):2273-8; Texidó, 2013, "Genetically engineered animal models for in vivo target identification and validation in oncology," Methods Mol. Biol.986: 281-305). However, cancer is a multigenic and highly heterogeneous disease: individual tumors typically carry mutations in dozens of genes. Genetically engineering mice is time consuming and difficult to do for the multiple mutations involved in a subject's cancer - time the human subject may not have. Indeed, recent genome-wide mutation profiling of human tumors has provided important insights into this genetic complexity/diversity (Stratton, 2011, "Exploring the genomes of cancer cells: progress and promise," Science, 331(6024):1553-8; Vogelstein, 2013, "Cancer genome landscapes," Science, 339(5127):1546-58), and partly explains the low success rate of candidate drugs that have entered clinical trials based on current preclinical models (Caponigro and Sellers, 2011, "Advances in the preclinical testing of cancer therapeutic hypotheses," Nat Rev Drug Discov, 10(3):179-87; Ocana et al., 2010, "Preclinical development of molecular-targeted agents for cancer," Nat Rev Clin Oncol, 8(4):200-9): for example, approval rates in colon cancer are approximately 3% of drugs that enter clinical trials (Hay et al, 13th BIO CEO & Investor Conference, 2011, New-York). The success rate of cancer clinical trials remains among the lowest of the major diseases (Ocana et al., 2010, "Preclinical development of molecular-targeted agents for cancer," Nat Rev Clin Oncol, 8(4):200-9).

Thus, there is a need for new therapeutics to treat cancer and new techniques to identify those therapeutics.

### 3. SUMMARY OF THE INVENTION

The invention provides a method for screening/selecting a therapeutically effective drug or combination of drugs for treating a subject diagnosed with cancer, wherein cancer cells from the subject exhibit increased activity of one or more oncogenes and/or reduced activity of one or more tumor suppressors, said method comprising:
providing a transgenic *Drosophila* larva avatar having an ortholog of an oncogene or a tumor suppressor of cancer cells of a subject diagnosed with cancer, the ortholog designed to be under inducible control such that, upon induction of the ortholog through an external factor, the activity of the ortholog is altered to correspond to the increased activity of the oncogene or reduced activity of the tumor suppressor, wherein the activity of the ortholog is in a larval tissue of the transgenic Drosophila larva avatar selected from the group consisting of the hindgut, midgut, imaginal disc, fat body, nephrocyte, heart, trachea, thorax, abdomen and malphigian tubules and the activity of the ortholog prevents the *Drosophila* larva avatar from surviving to pupation; and
screening a library of drugs, wherein said screening comprises feeding a culture of the transgenic *Drosophila* larva avatar a drug or combination of drugs,
wherein a therapeutically effective drug or combination of drugs is selected from the library of drugs when the transgenic *Drosophila* larva avatar survives to pupation.

The invention relates to improved methods for treating a human subject diagnosed with cancer using combination drug regimens tailored to the genome/proteome/phenome of the subject's tumor/cancer. Particularly useful disclosures involve methods for treating a human subject diagnosed with a solid tumor cancer who has failed standard of care therapy or for which there is no substantially effective standard of care therapy, using a therapeutically effective combination drug regimen tailored to the genome/proteome/phenome of the subject's tumor/cancer. The invention relates to screening methods that can be used to quickly identify and select therapeutically effective regimens for the subject, or to confirm the therapeutic efficacy of the selected regimen tailored to the patient. The regimens used in accordance with the invention are those most efficacious to treat the particular genomic/proteomic/phenomic profile of the subject's tumor/cancer.

The invention is based, in part, on the recognition that cancer is genetically complex - involving the accumulation of multiple mutations over time that obscures the culprits responsible for disease, as well as the identification of the targets within a patient's profile that are most beneficial for intervention. Attributing causal relationships to any one mutated target among a panoply of mutations in the subject introduces a bias that may render the selected treatment less than an ideal match for the patient.

The invention is also based, in part, on the use of a *Drosophila* "avatar" of the patient's genomic/proteomic/phenomic profile to test the therapeutic regimens selected or screened. The avatar can be used to screen different regimens to identify those most effective, or to confirm the efficacy of a selected regimen for the patient. In a related embodiment, "avatar armies" *(e.g., Drosophila* "avatar armies") reflecting the genomic/proteomic/phenomic profile of a population of patients diagnosed with a disease or disorder can be used to screen and select therapeutic regimens for treatment and/or to screen for new lead compounds and identify new therapeutics for the disease or disorder. In another embodiment, an avatar *(e.g., Drosophila* avatar) from an avatar army *(e.g.,* a *Drosophila* avatar army) that most closely resembles a patient's genomic/proteomic/phenomic profile may be selected and used to screen and identify therapeutic regimens for the patient's cancer.

While the disclosure is not limited to the use of the avatar to select the therapeutic regimen (other animal model avatars could be used for this purpose), the avatar model system as described herein offers the advantage of flexibility and speed - the genetic tools available for rapidly generating transgenic flies can be used to up- or down- regulate the activity of multiple orthologs of human gene products in the fly avatar to reflect the patient's profile. Moreover, the assay used to screen or test therapeutic regimens is rapid and does not require expensive equipment for read-outs. Thus saving precious time in selecting a regimen tailored to the patient - a factor extremely important for treating a cancer patient at late stages of disease and morbidity.

Briefly, tumor/cancer cells from the human subject are analyzed to characterize the patient's mutations, particularly those predicted to increase activity of one or more oncogene products and/or reduce activity of one or more tumor suppressor gene products. This information is used to design and construct a *Drosophila* avatar that recapitulates the patient's phenome. Similar information obtained from patients within a disease population can be used to design and construct *Drosophila* "avatar armies."

A *Drosophila* avatar is engineered by genetically modifying a fly to correspondingly increase the activity of an ortholog(s) of the human subject's oncogene product(s), and inhibit the activity of an ortholog(s) of the human subject's tumor suppressor gene product(s) in a tissue/organ vital/necessary for survival (for example, the hindgut of the larva). The activity of the engineered orthologs should be designed to be under inducible control so that, *e.g.,* upon induction, the untreated larva avatar *(e.g.,* untreated *Drosophila* larva avatar) does not survive to pupation or mature to an adult fly. This allows for the preferred activity to be controlled at will to facilitate screening.

The drug regimens to be screened or confirmed for therapeutic efficacy are added to the food supplied to the culture of *Drosophila* avatars. Embryos are placed on the food; they begin consuming the food as larvae, at which point the activity of the transgenic orthologs are or have been induced. Therapeutic efficacy of the regimen is indicated by survival of the larva. The assay does not require tumor visualization, expensive equipment or detection of markers not compatible with high through-put screening for a read-out. However, such assays are not precluded for follow up studies.

The therapeutic regimen arrived at or confirmed by the fly avatar assay can be communicated to the oncologist and ultimately to the patient for treatment. Where the therapeutic regimen involves combinations of known drugs where the toxicity and therapeutic indices are known, no further testing may be necessary. This could be especially important for patients with advanced disease where time is of the essence. Nevertheless, standard laboratory animal models (*e.g*., mice) could be used for further safety and efficacy testing of the regimen. Optionally, the regimen can be tested using cells from the patient to confirm efficacy prior to treatment.

In constructing the fly avatar (*e.g*., Drosophila avatar) assays, the following guiding principles should be followed:
(a) The exact mutation of the patient's tumor is not required to be engineered into the avatar (*e.g*., Drosophila avatar) for the invention to work. All that is required is to up-regulate the activity of orthologs of the patient's genes that demonstrate increased activity, and down-regulate the activity of orthologs of the patient's genes that exhibit decreased activity.
(b) Due to the lethality of the engineered phenotype, the expression of the orthologs should be placed under inducible control so that the lethal activity can be induced at will, *e.g.,* when the larva cultures are fed the drug regimens. (*N.B.,* during fly development, embryos hatch to progress through three larval stages followed by pupation and metamorphosis to adult flies).
(c) While the activity of the orthologs is required to be altered in a larval organ vital to survival, the altered activity need not be confined/targeted solely to that organ -- activity of the orthologs can also be altered in other tissues. However, it is critical for the assay that the altered expression/activity occur in an organ vital to survival of the larvae - in this way, one can rapidly test drugs, combinations of drugs and regimens for efficacy by incorporating the drugs into the larval food, using survival of the larvae as the readout.

The invention is illustrated by the examples, *infra,* which demonstrate methods for producing *Drosophila* avatars of a patient's cancer and methods for screening to identify therapeutic regimens for treating cancer utilizing such avatars. *See,* Sections 6-9, *infra.*

Provided herein is a method for treating a subject diagnosed with cancer, comprising administering a therapeutic regimen to a subject diagnosed with cancer, wherein the therapeutic regimen comprises a drug or a combination of drugs, wherein (a) cancer cells from the subject exhibit increased activity of one or more oncogenes and/or reduced activity of one or more tumor suppressors, and (b) the drug or combination of drugs, when fed to a culture of a *Drosophila* larva avatar, allows the *Drosophila* larva avatar to survive to pupation, and wherein the *Drosophila* larva avatar is genetically modified such that upon induction through an external factor there is an increase in the activity of one or more orthologs of the subject's one or more oncogenes and/or inhibition of one or more orthologs of the subject's one or more tumor suppressors in a larval tissue that is necessary for survival to pupation, which increase in activity and/or inhibition prevents an untreated *Drosophila* larva avatar from surviving to pupation. In a specific embodiment, the larval tissue that is necessary for survival is a hindgut or an imaginal disc. In another specific embodiment, the external factor is temperature. In another specific embodiment, the subject is human.

In certain embodiments, in accordance with the methods described herein the cancer is a solid tumor. In a specific embodiment, in accordance with the methods described herein the cancer is bladder cancer, bone cancer, breast cancer, cervical cancer, colorectal cancer, esophageal cancer, gallbladder cancer, head and neck cancer, kidney cancer, lung cancer, ocular cancer, oral cancer, ovarian cancer, pancreatic cancer, pelvic cancer, penile cancer, prostate cancer, skin cancer, throat cancer, thyroid cancer, such as medullary thyroid cancer, or uterine cancer.

In some embodiments, the activity of the one, two, three, four, five, six, seven, eight, nine, ten or more orthologs of the oncogenes is increased by at least 10%, 20%, 30%, 40%, 50%, 60%, 70%, 80%, 90%, 100%, 125%, 150%, 175%, 200%, 250%, or 300% or more as compared to the activity of the one or more orthologs of the oncogenes in a non-modified *Drosophila.*

In specific embodiments, in accordance with the methods described herein the one, two, three, four, five, six, seven, eight, nine, ten or more oncogenes are selected from the group consisting of abl, Af4/hrx, akt-2, alk, alk/npm, aml1, aml1/mtg8, axl, bcl-2, bcl-3, bcl-6, bcr/abl, c-myc, dbl, dek/can E2A/pbx1, EGFR, enl/hrx, erg/TLS, erbB, erbB-2, ets-1, ews/fli-1, fms, fos, fps, gli, gsp, Her2/neu, hox11, hst, IL-3, int-2, jun, kit, KS3, K-sam, Lbc, lck, lmo1, lmo2, L-myc, lyl-1, lyt-10/C alpha1, mas, mdm-2, mll, mos, mtg8/aml1, myb, MYH11/CBFB, neu, N-myc, ost, pax-5, pbx1/E2A, pim-1, PRAD-1, raf, RAR/PML, rash, rasK, rasN, rel/nrg, ret, rhom1, rhom2, ros, ski, sis, set/can, src, tall, tal2, tan-1, Tiam1, TSC2, and trk.

In certain embodiments, in accordance with the methods described herein the one, two, three, four, five, six, seven, eight, nine, ten or more orthologs of the oncogene is overexpressed in the *Drosophila* larva avatar as compared to the expression of the one, two, three, four, five, six, seven, eight, nine, ten or more orthologs of the oncogenes in a non-modified *Drosophila.*

In some embodiments, in accordance with the methods described herein the activity of more than one ortholog *(e.g.,* 2, 3, 4, 5, 6, 7, 8, 9, 10 or more orthologs) of the oncogenes is increased by genetically engineering the *Drosophila* to express cDNAs encoding the orthologs, which expression of the cDNAs are regulated by exposure to the external factor. In certain embodiments, in accordance with the methods described herein the activity of more than one ortholog *(e.g.,* 2, 3, 4, 5, 6, 7, 8, 9, 10 or more orthologs) of more than one oncogene *(e.g.,* 2, 3, 4, 5, 6, 7, 8, 9, 10 or more oncogenes) is increased by genetically engineering the *Drosophila* to express cDNAs encoding the orthologs, which expression of the cDNAs are regulated by exposure to the external factor.

In some embodiments, in accordance with the methods described herein the activity of the one, two, three, four, five, six, seven, eight, nine, ten or more orthologs of the tumor suppressors is decreased by at least 10%, 20%, 30%, 40%, 50%, 60%, 70%, 80%, 90%, 100%, 125%, 150%, 175%, 200%, 250%, or 300% or more as compared to the activity of the one, two, three, four, five, six, seven, eight, nine, ten or more orthologs of the tumor suppressors in a non-modified *Drosophila.*

In specific embodiments, in accordance with the methods described herein the one, two, three, four, five, six, seven, eight, nine, ten or more tumor suppressors are selected from the group consisting of APC, BRCA1, BRCA2, CDKN2A, DCC, DPC4 (SMAD4), MADR2/JV18 (SMAD2), MEN1, MTS1, NF1, NF2, p53, PTEN, Rb, VHL, WRN, and WT1.

In some embodiments, in accordance with the methods described herein the one, two, three, four, five, six, seven, eight, nine, ten or more orthologs of the tumor suppressors is underexpressed in the *Drosophila* larva avatar as compared to the expression of the one or more orthologs of the oncogenes in a non-modified *Drosophila.* In certain embodiments, in accordance with the methods described herein expression of the one, two, three, four, five, six, seven, eight, nine, ten or more orthologs of the tumor suppressors is inhibited in the *Drosophila* larva avatar.

In some embodiments, in accordance with the methods described herein more than one ortholog *(e.g.,* 2, 3, 4, 5, 6, 7, 8, 9, 10 or more orthologs) of the tumor suppressors is inhibited by genetically engineering the *Drosophila* larva to express interfering RNAs which expression of the interfering RNAs are regulated by exposure to the external factor. In certain embodiments, in accordance with the methods described herein more than one ortholog (*e.g., 2,* 3, 4, 5, 6, 7, 8, 9, 10 or more orthologs) of more than one tumor suppressor (*e.g*., 2, 3, 4, 5, 6, 7, 8, 9, 10 or more tumor suppressors) is inhibited by genetically engineering the *Drosophila* larva to express interfering RNAs which expression of the interfering RNAs are regulated by exposure to the external factor.

In certain embodiments, in accordance with the methods described herein the subject has failed standard of care therapy. In some embodiments, in accordance with the methods described herein there is no standard of care therapy for the cancer. In certain embodiments, in accordance with the methods described herein the subject is a treatment naive subject.

In certain embodiments, in accordance with the methods described herein the drug or combination of drugs allows the *Drosophila* larva avatar to survive to adulthood. In some embodiments, in accordance with the methods described herein treatment of the *Drosophila* larva avatar with the drug or combination of drug increases survival of the avatar to adulthood.

In certain embodiments, in accordance with the methods described herein the drug is or the combination of drugs comprises an anti-cancer therapy. In specific embodiments, in accordance with the methods described herein the anti-cancer therapy is an alkylating agent(s), an anthracycline(s), a cytoskeletal disruptor(s), a histone deacetylase inhibitor(s), an inhibitor(s) of topoisomerase I, an inhibitor(s) of topoisomerase II, a kinase inhibitor(s), a nucleotide analog(s), a precursor analog(s), a peptide antibiotic(s), a platinum-based agent(s), a retinoid(s), a vinca alkaloid(s), a derivative of any of the foregoing, or a combination of two or more of the foregoing. In certain embodiments, in accordance with the methods described herein the combination of drugs does not comprise one or more of the combinations listed in Table 2. In other embodiments, in accordance with the methods described herein the combination of drugs comprises one or more of the combinations listed in Table 2, wherein the subject is not suffering from the corresponding cancer listed in Table 2.

In some embodiments, in accordance with the methods described herein the drug is or the combination of drugs comprises vandetanib, sunitinib, sorafenib, AD57 (the DFG-out Type II compound), AD58, AD80, caprelsa, or the Ret-targeting inhibitor DL06. In other embodiments, in accordance with the methods described herein the drug is not or the combination of drugs does not comprise vandetanib, sunitinib, sorafenib, AD57 (the DFG-out Type II compound), AD58, AD80, caprelsa, or the Ret-targeting inhibitor DL06.

In another aspect, provided herein is a method for screening/selecting a therapeutically effective drug or combination drugs for treating a subject diagnosed with cancer, wherein cancer cells from the subject exhibit increased activity of one, two, three, four, five, six, seven, eight, nine, ten or more oncogenes and/or reduced activity of one, two, three, four, five, six, seven, eight, nine, ten or more tumor suppressors, comprising screening a library of drugs for those drugs or combination of drugs that when fed to a culture of a *Drosophila* larva avatar, allow the *Drosophila* larva avatar to survive to pupation, such that upon induction through an external factor there is an increase in the activity of an ortholog(s) of the subject's oncogene(s) and/or inhibition an ortholog(s) of the subject's tumor suppressor(s) in a larval tissue that is necessary for survival to pupation, which increase in activity and/or inhibition prevents an untreated *Drosophila* larva avatar from surviving to pupation. In a specific embodiment, the larval tissue that is necessary for survival is a hindgut or an imaginal disc. In another specific embodiment, the external factor is temperature. In another specific embodiment, the subject is human.

In certain embodiments, in accordance with the methods described herein the cancer is a solid tumor. In a specific embodiment, in accordance with the methods described herein the cancer is bladder cancer, bone cancer, breast cancer, cervical cancer, colorectal cancer, esophageal cancer, gallbladder cancer, head and neck cancer, kidney cancer, lung cancer, ocular cancer, oral cancer, ovarian cancer, pancreatic cancer, pelvic cancer, penile cancer, prostate cancer, skin cancer, throat cancer, thyroid cancer, such as medullary thyroid cancer, or uterine cancer.

In some embodiments, the activity of the one, two, three, four, five, six, seven, eight, nine, ten or more orthologs of the oncogenes is increased by at least 10%, 20%, 30%, 40%, 50%, 60%, 70%, 80%, 90%, 100%, 125%, 150%, 175%, 200%, 250%, or 300% or more as compared to the activity of the one or more orthologs of the oncogenes in a non-modified *Drosophila.*

In specific embodiments, in accordance with the methods described herein the one, two, three, four, five, six, seven, eight, nine, ten or more oncogenes are selected from the group consisting of abl, Af4/hrx, akt-2, alk, alk/npm, aml1, aml1/mtg8, axl, bcl-2, bcl-3, bcl-6, bcr/abl, c-myc, dbl, dek/can E2A/pbx1, EGFR, enl/hrx, erg/TLS, erbB, erbB-2, ets-1, ews/fli-1, fms, fos, fps, gli, gsp, Her2/neu, hox11, hst, IL-3, int-2, jun, kit, KS3, K-sam, Lbc, lck, lmo1, lmo2, L-myc, lyl-1, lyt-10/C alpha1, mas, mdm-2, mll, mos, mtg8/aml1, myb, MYH11/CBFB, neu, N-myc, ost, pax-5, pbx1/E2A, pim-1, PRAD-1, raf, RAR/PML, rash, rasK, rasN, rel/nrg, ret, rhom1, rhom2, ros, ski, sis, set/can, src, tall, tal2, tan-1, Tiam1, TSC2, and trk.

In certain embodiments, in accordance with the methods described herein the one, two, three, four, five, six, seven, eight, nine, ten or more orthologs of the oncogene is overexpressed in the *Drosophila* larva avatar as compared to the expression of the one, two, three, four, five, six, seven, eight, nine, ten or more orthologs of the oncogenes in a non-modified *Drosophila.*

In some embodiments, in accordance with the methods described herein the activity of more than one ortholog *(e.g.,* 2, 3, 4, 5, 6, 7, 8, 9, 10 or more orthologs) of the oncogenes is increased by genetically engineering the *Drosophila* to express cDNAs encoding the orthologs, which expression of the cDNAs are regulated by exposure to the external factor. In certain embodiments, in accordance with the methods described herein the activity of more than one ortholog *(e.g.,* 2, 3, 4, 5, 6, 7, 8, 9, 10 or more orthologs) of more than one oncogene *(e.g.,* 2, 3, 4, 5, 6, 7, 8, 9, 10 or more oncogenes) is increased by genetically engineering the *Drosophila* to express cDNAs encoding the orthologs, which expression of the cDNAs are regulated by exposure to the external factor.

In some embodiments, in accordance with the methods described herein the activity of the one, two, three, four, five, six, seven, eight, nine, ten or more orthologs of the tumor suppressors is decreased by at least 10%, 20%, 30%, 40%, 50%, 60%, 70%, 80%, 90%, 100%, 125%, 150%, 175%, 200%, 250%, or 300% or more as compared to the activity of the one, two, three, four, five, six, seven, eight, nine, ten or more orthologs of the tumor suppressors in a non-modified *Drosophila.*

In specific embodiments, in accordance with the methods described herein the one, two, three, four, five, six, seven, eight, nine, ten or more tumor suppressors are selected from the group consisting of APC, BRCA1, BRCA2, CDKN2A, DCC, DPC4 (SMAD4), MADR2/JV18 (SMAD2), MEN1, MTS1, NF1, NF2, p53, PTEN, Rb, VHL, WRN, and WT1.

In some embodiments, in accordance with the methods described herein the one, two, three, four, five, six, seven, eight, nine, ten or more orthologs of the tumor suppressors is underexpressed in the *Drosophila* larva avatar as compared to the expression of the one or more orthologs of the oncogenes in a non-modified *Drosophila.* In certain embodiments, in accordance with the methods described herein expression of the one, two, three, four, five, six, seven, eight, nine, ten or more orthologs of the tumor suppressors is inhibited in the *Drosophila* larva avatar.

In some embodiments, in accordance with the methods described herein more than one ortholog *(e.g.,* 2, 3, 4, 5, 6, 7, 8, 9, 10 or more orthologs) of the tumor suppressors is inhibited by genetically engineering the *Drosophila* larva to express interfering RNAs which expression of the interfering RNAs are regulated by exposure to the external factor. In certain embodiments, in accordance with the methods described herein more than one ortholog (*e.g., 2,* 3, 4, 5, 6, 7, 8, 9, 10 or more orthologs) of more than one tumor suppressor (*e.g*., 2, 3, 4, 5, 6, 7, 8, 9, 10 or more tumor suppressors) is inhibited by genetically engineering the *Drosophila* larva to express interfering RNAs which expression of the interfering RNAs are regulated by exposure to the external factor.

In certain embodiments, in accordance with the methods described herein the subject has failed standard of care therapy. In some embodiments, in accordance with the methods described herein there is no standard of care therapy for the cancer. In certain embodiments, in accordance with the methods described herein the subject is a treatment naive subject.

In certain embodiments, in accordance with the methods described herein the drug or combination of drugs allows the *Drosophila* larva avatar to survive to adulthood. In some embodiments, in accordance with the methods described herein treatment of the *Drosophila* larva avatar with the drug or combination of drug increases survival of the avatar to adulthood.

In certain embodiments, in accordance with the methods described herein the library of drugs comprises an alkylating agent(s), an anthracycline(s), a cytoskeletal disruptor(s), a histone deacetylase inhibitor(s), an inhibitor(s) of topoisomerase I, an inhibitor(s) of topoisomerase II, a kinase inhibitor(s), a nucleotide analog(s), a precursor analog(s), a peptide antibiotic(s), a platinum-based agent(s), a retinoid(s), a vinca alkaloid(s), a derivative of any of the foregoing, or a combination of two or more of the foregoing. In certain embodiments, in accordance with the methods described herein the library of drugs does not comprise one or more of the combinations listed in Table 2. In other embodiments, in accordance with the methods described herein the library of drugs comprises one or more of the combinations listed in Table 2, wherein the subject is not suffering from the corresponding cancer listed in Table 2.

In some embodiments, in accordance with the methods described herein the library of drugs comprises vandetanib, sunitinib, sorafenib, AD57 (the DFG-out Type II compound), AD58, AD80, caprelsa, or the Ret-targeting inhibitor DL06. In other embodiments, in accordance with the methods described herein the library of drugs does not comprise vandetanib, sunitinib, sorafenib, AD57 (the DFG-out Type II compound), AD58, AD80, caprelsa, or the Ret-targeting inhibitor DL06.

### 3.1. DEFINITIONS

As used herein, the following terms shall have the indicated meanings.

The term "avatar" refers to an organism *(e.g.,* an organism of the *Drosophila* genus or subgenus, such as the species *Drosophila melanogaster*) whose genomic, proteomic and/or phenomic profile is modified as compared to non-modified organism. The genomic, proteomic and/or phenomic profile of the organism *(e.g., Drosophila)* may be modified such that the activity of one or more orthologs of disease drivers (*e.g*., tumor drivers) are altered in the organism as compared to the non-modified organism (*e.g., Drosophila).* The genomic, proteomic and/or phenomic profile of the organism may be modified such that the activity of one or more orthologs of disease drivers (*e.g*., tumor drivers) is altered to correspond to the profile of a subject suffering from cancer; sometimes referred to herein as a "personalized avatar." Alternatively, the genomic, proteomic and/or phenomic profile of a population of organisms *(e.g., Drosophila)* may be modified such that the activity of one or more orthologs of disease drivers (*e.g*., tumor drivers) is altered to correspond to the profile of a population of subjects *(e.g.,* 5, 10, 20, 25, 30, 40, 50, 75, 100, 125, 150, 175, 200, 225, 250, 275, 300, 325, 350, 375, 400 or more subjects) suffering from cancer; sometimes referred to herein as an "avatar army." In some embodiments, an avatar army refers to a collection of personalized avatars. Alternatively, the genomic, proteomic and/or phenomic profile of the organism (*e.g*., *Drosophila)* or population of organisms *(e.g., Drosophila)* may be modified such that the activity of one or more orthologs of disease drivers (*e.g*., tumor drivers) is altered to correspond to the profile of a population of subjects suffering from cancer or patient information from one or more databases; sometimes referred to herein as a "generic avatar." A generic avatar may refer to an avatar whose genomic, proteomic and/or phenomic profile is based on (a) one or more disease drivers from the genomic, proteomic and/or phenomic profile of a population of subjects suffering from a disease; (b) the genomic, proteomic and/or phenomic profile from one or more databases; or (c) genomic, proteomic and/or phenomic information from one or more databases. In some embodiments, a generic avatar refers to an avatar whose genomic, proteomic, and/or phenomic profile comprises altered expression or activity of tumor drivers that are commonly altered in a population of subjects suffering from cancer. Generic avatars may be useful starting points for the generation of personalized avatars and/or avatar armies through a combination of genetic engineering and breeding/crossing. In one disclosure, the avatar is from the order Diptera. In the invention, the avatar is a member of the *Drosophila* genus or subgenus. In another specific embodiment, the avatar is a member of the species *Drosophila melanogaster.*

As used herein, the terms "control sample" and "control samples" refers to a sample obtained or derived from a subject. A control sample may be a sample obtained or derived from a subject who is suffering from cancer. Alternatively, a control sample may be a sample obtained or derived from a healthy subject or a subject who is not suffering from cancer. In a specific embodiment, a reference sample and a control sample are samples from the same subject. In another embodiment, a reference sample and a control sample are samples from different subjects. In a specific embodiment, a control sample is the same type of sample as the reference sample. For example, a control sample is from the same organ, tissue and/or body fluid of the subject as the reference sample. Alternatively, a control sample is a different type of sample as the reference sample. For example, a control sample may be a blood sample, whereas the reference sample is a sample from the tumor. In another example, a control sample is from a non-diseased tissue or organ from a subject and a reference sample is from a diseased tissue or organ from the same subject.

The terms "disease driver" and "disease drivers" refer to any gene product(s) whose altered activity in a subject contributes to progression of disease. The terms "tumor driver" and "tumor drivers" refer to any gene product(s) whose altered activity in a subject contributes to the progression of cancer; for example, increased activity of one or more oncogene products and/or reduced activity of one or more tumor suppressor gene products that contribute to tumor progression.

The term "genome" refers to an organism's complete set of DNA, including all of its genes. Each genome contains all of the information needed to build and maintain that organism.

The term "in combination" in the context of the administration of (a) therapy(ies) to a subject, refers to the use of more than one therapy. The two or more therapies may be administered in one or more dosage forms. The use of the term "in combination" does not restrict the order in which therapies are administered to a subject. A first therapy can be administered prior to, concomitantly with, or subsequent to the administration of a second therapy to a subject.

The term"non-modified" in the context of an organism, such as *Drosophila,* refers to an organism without known mutation. In a specific embodiment, the non-modified organism is a naturally occurring organism that you would find in nature.

The terms "organ necessary for maturation to an adult fly" or "tissue necessary for maturation to an adult fly" may be used interchangeably and refer to an organ or tissue that prevents an organism from maturing to adulthood if the organ or tissue was entirely removed from the organism.

The terms "organ necessary for pupation" or "tissue necessary for pupation" are used interchangeably and refer to an organ or tissue that prevents the pupal development of an organism if the organ or tissue was entirely removed from the organism. An "organ necessary for pupation" or "tissue necessary for pupation" may refer to an organ or tissue that prevents the larval-pupal transformation of an organism if the organ or tissue was entirely removed from the organism. An "organ necessary for pupation" or "tissue necessary for pupation" may refer to an organ or tissue that prevents the pupal-adult transformation of an organism if the organ or tissue was entirely removed from the organism.

The term "phenome" refers to a set of phenotypes expressed by a cell, tissue, organism, or species. A phenomic profile may refer to a set of phenotypes expressed by (a) a cell or tissue in a sample from a subject; (b) a subject; (c) a cell or tissue in a sample from a population of subjects; and/or (d) a population of subjects. A phenomic profile may refer to a subset of phenotypes expressed by (a) a cell or tissue in a sample from a subject; (b) a subject; (c) a cell or tissue in a sample from a population of subjects; and/or (d) a population of subjects. A phenomic profile may be based on phenomic information from one or more databases, one or more subjects, one or more samples from one or more subjects, or any combination thereof.

The term "proteome" refers to the entire set of proteins expressed by a genome, cell, tissue or organism at a certain time. More specifically, it is the set of expressed proteins in a given type of cell or organism, at a given time, under defined conditions. A proteomic profile may refer to a set of proteins expressed by (a) a cell or tissue in a sample from a subject; (b) a subject; (c) a cell or tissue in a sample from a population of subjects; and/or (d) a population of subjects. A proteomic profile may refer to a subset of proteins expressed by (a) a cell or tissue in a sample from a subject; (b) a subject; (c) a cell or tissue in a sample from a population of subjects; and/or (d) a population of subjects. A proteomic profile may be based on proteomic information from one or more databases, one or more subjects, one or more samples from one or more subjects, or any combination thereof.

The terms "reference sample" and "reference samples" refer to a sample obtained or derived from a subject suffering from cancer. A reference sample may be a sample comprising biological material from a subject suffering from cancer. A reference sample may comprise cells, tissues, organs and/or fluids from a subject suffering from cancer. A reference sample may comprise blood, plasma, serum, urine, saliva, stool, or other biological fluid. In a specific embodiment, a reference sample comprises a cancer cell or at least a portion of a tumor from a subject suffering from cancer. A reference sample may comprise nucleic acids and/or proteins from a subject suffering from cancer.

The term "sample" in the context of a reference sample or control sample may refer to a sample comprising biological material obtained or derived from a subject. Biological material may refer to nucleic acids *(e.g.,* DNA and/or RNA), proteins, cells, tissues, organs, and/or fluids. A sample may comprise nucleic acids, proteins, cells, tissues, organs, fluids, or any combination thereof. A sample may comprise nucleic acids, proteins and/or cells derived from a subject. A sample may comprise any cell in the subject. In some embodiments, a sample comprises one or more cells from the skin, eye, mouth, tongue, cheek, ear, nose, throat, pharynx, gallbladder, breast, prostate, bone, blood, ovary, uterus, heart, lung, kidney, liver, pancreas, intestine, stomach, or a combination thereof. A sample may comprise one or more cells from the musculoskeletal system, digestive system, respiratory system, urinary system, reproductive system, endocrine system, circulatory system, nervous system, and/or integumentary system. A sample may comprise blood, plasma, serum, urine, saliva, stool, or other biological fluid.

The terms "subject" or "patient" are used interchangeably. As used herein, the terms "subject" and "subjects" refer to an animal. In some embodiments, the subject is a mammal including a non-primate (*e.g*., a camel, donkey, zebra, cow, horse, horse, cat, dog, rat, and mouse) and a primate *(e.g.,* a monkey, chimpanzee, and a human). In some embodiments, the subject is a non-human mammal. In certain embodiments, the subject is a pet *(e.g.,* dog or cat) or farm animal *(e.g.,* a horse, pig or cow). In specific embodiments, the subject is a human.

The terms "therapies" and "therapy" can refer to any protocol(s), method(s), and/or agent(s) that can be used in the treatment of a disease (*e.g*., cancer). In certain embodiments, the terms "therapies" and "therapy" refer to chemotherapy, immune modulatory drugs, and/or other therapies useful in the treatment of a cancer. In a specific embodiment, a therapy includes adjuvant therapy. For example, using a therapy in conjunction with a drug therapy, biological therapy, hormonal therapy, surgery, and/or supportive therapy.

The term "transgene" may refer to a cDNA of an ortholog of a disease driver *(e.g.,* tumor driver), a cDNA of an activator of an ortholog of a disease driver, a cDNA of an inhibitor of an ortholog of a disease driver, a small interfering RNA (siRNA) that targets an ortholog of the disease driver (*e.g*., tumor driver), a short hairpin RNA (shRNA) that targets an ortholog of the disease driver (*e.g*., tumor driver), or any combination thereof. In some embodiments, the cDNA of an inhibitor of an ortholog of a disease driver is an interfering RNA (sometimes referred to herein as an "RNAi").

The terms "treat" and "treating" in the context of the administration of a therapy refer to a treatment/therapy from which a subject receives a beneficial effect, such as the reduction, decrease, attenuation, diminishment, stabilization, remission, suppression, inhibition or arrest of the development or progression of cancer, or a symptom thereof. In some disclosures, the treatment/therapy that a subject receives does not cure the disease (*e.g*., cancer), but prevents the progression or worsening of the disease. In certain disclosures, the treatment/therapy that a subject receives does not prevent the onset/development of disease (*e.g*., cancer), but may prevent the onset of disease (*e.g*., cancer) symptoms.

The terms "vital organ", "organ necessary for survival", "vital tissue", or "tissue necessary for survival" are used interchangeably and refer to an organ or tissue that an organism cannot live without at one or more stages of its development if the organ or tissue was entirely removed from the organism.

The term "wild-type" refers to an organism that is naturally occurring and that you would find in nature.

### 4. DESCRIPTION OF THE FIGURES

FIG. 1 shows a schematic of a cloning strategy for generating a construct that is used to transform an organism to produce a subject-specific avatar. In particular, this figure is a schematic showing how *Drosophila* orthologs of patient oncogenes and tumor suppressors are placed into standard *Drosophila* transformation vector. When stably transformed into animals, the inserts can be targeted to specific tissues through tissue-specific drivers.
FIG. 2 shows a screening platform for the avatar. In particular, this figure is a schematic showing the overall screening approach; flies are placed into tubes or plates and fed drug mixed with food. Phenotypes such as viability are then assessed.
FIG. 3 shows a scheme for developing a treatment database for rapid recommendations. In particular, this figure is an overview indicating how fly avatars are generated and tested to identify personalized therapies for patients.
FIG. 4A, FIG. 4B, FIG. 4C, and FIG. 4D: Fly Colorectal Cancer Phenotypes: byn>ras^{G12V} p53^{RNAi} pten^{RNAi} apc^{RNAi} cells marked with GFP. Cells exhibit multilayering (FIG. 4A) and migration (FIG. 4B) to distant sites (FIG. 4C) to form distant loci (^{∗}), *e.g.*, associated with trachea in the abdomen ( FIG. 4D).
FIG.5 depicts an interaction map that demonstrates emergent phenotypes in *Drosophila* with various cancer gene combinations targetd by transgene.
FIG. 6 shows drugs and compounds tested orally on genetically ras and ras-pten-apc-p53 *Drosophila* larvae. Feeding experiments indicating drugs with (√) and without (^{∗}) significant efficacy.
FIG. 7 shows that bortezomib/BEZ235 was more effective on genotypically *KRas-PIK3CA-APC* mouse allografts versus single drugs. Bar = 1 animal.
FIG. 8 shows that 1 µM trametinib (Tra) rescued *blt>Ras1*, *PTEN* larval lethality (p<0.0001) at 25°C. Fluvastatin (Flu) synergized with trametinib (Tra) at select concentrations, shown in µM food concentration.

### 5. DETAILED DESCRIPTION OF THE INVENTION

Improved methods for treating a human subject diagnosed with cancer using combination drug regimens tailored to the genome/proteome/phenome of the subject's tumor/cancer are described. Particularly attractive disclosures involve treating a human subject diagnosed with a solid tumor cancer who has failed standard of care therapy or for which there is no substantially effective standard of care therapy, using a therapeutically effective combination drug regimen tailored to the genome/proteome/phenome of the subject's tumor/cancer. The invention relates to screening methods that can be used to quickly identify and select therapeutically effective regimens for the subject, or to confirm the therapeutic efficacy of the selected regimen tailored to the patient. The regimens used in accordance with the invention are those most efficacious to treat the particular genomic/proteomic/phenomic profile of the subject's tumor/cancer.

Cancer is genetically complex - involving the accumulation of multiple mutations over time that obscures the culprits responsible for disease, as well as the identification of the targets within a patient's profile that are most beneficial for intervention. Attributing causal relationships to any one mutated target among a panoply of mutations in the subject introduces a bias that may render the selected treatment less than an ideal match for the patient.

Moreover, treatments for cancers involving multiple mutations are not effectively designed by merely combining "single hit" regimens. In experiments with a fly model for colorectal cancer involving the activation of Ras1 in hindgut tumors, we found that sixteen different kinase inhibitors were effective when used as single agents against the single hit. However, when three additional genes were targeted within the fly model to inhibit tumor suppressors-disrupting p53, pten, and apc1/2—single drugs were no longer effective when used as single agents. Combinations of two or more drugs were required for efficacy against the multiple targeted tumor. *See* Sections 7 and 8, *infra.*

The invention is based, in part, on the use of a *Drosophila* "avatar" of the patient's genomic/proteomic/phenomic profile to test the therapeutic regimens selected or screened. The avatar can be used to screen different regimens to identify those most effective, or to confirm the efficacy of a selected regimen for the patient. In a related embodiment, "avatar armies" (*e.g*., *Drosophila* avatars) reflecting the genomic/proteomic/phenomic profile of a population of patients diagnosed with a disease or disorder can be used to screen and select therapeutic regimens for treatment and/or to screen for new lead compounds and identify new therapeutics for the disease or disorder.

While the disclosure is not limited to the use of the fly avatar to select the therapeutic regimen (other animal model avatars could be used for this purpose), the fly avatar model system as described herein offers the advantage of flexibility and speed - the genetic tools available for rapidly generating transgenic flies can be used to up- or down- regulate the activity of multiple orthologs of human gene products in the fly avatar to reflect the patient's profile. Moreover, the assay used to screen or test therapeutic regimens is rapid and does not require expensive equipment for read-outs. Thus saving precious time in selecting a regimen tailored to the patient - a factor extremely important for treating a cancer patient at late stages of disease and morbidity.

In the methods described herein, tumor/cancer cells from the human subject are analyzed to characterize the patient's mutations, particularly those predicted to increase activity of one or more oncogene products and/or reduced activity of one or more tumor suppressor gene products. This information is used to design and construct a *Drosophila* avatar that recapitulates the patient's phenome. Similar information obtained from patients within a disease population can be used to design and construct the *Drosophila* "avatar armies."

A *Drosophila* avatar is engineered by genetically modifying a fly to correspondingly increase the activity of ortholog(s) of the human subject's oncogene product(s), and inhibit the activity of ortholog(s) of the human subject's tumor suppressor gene product(s) in a tissue/organ vital/necessary for survival (for example, the hindgut of the larva). The activity of the engineered orthologs should be designed to be under inducible control so that, *e.g.,* upon induction, the untreated *Drosophila* larva avatar does not survive to pupation or mature to an adult fly. In some embodiments, the activity of the engineered orthologs should be designed to be under inducible control so that, *e.g.,* upon induction, the untreated *Drosophila* larva avatar does not survive to pupariation or mature to an adult fly. This allows for the preferred activity to be controlled at will to facilitate screening.

The drug regimens to be screened or confirmed for therapeutic efficacy are added to the food supplied to the culture of *Drosophila* avatars. Embryos are placed on the food; they begin consuming the food as larvae, at which point the activity of the transgenic orthologs are or have been induced. Therapeutic efficacy of the regimen is indicated by survival of the larva. The assay does not require tumor visualization, expensive equipment or detection of markers not compatible with high through-put screening for a read-out. However, such assays are not precluded for follow up studies.

The therapeutic regimen arrived at or confirmed by the fly avatar assay can be communicated to the oncologist and ultimately to the patient for treatment. Where the therapeutic regimen involves combinations of known drugs where the toxicity and therapeutic indices are known, no further testing may be necessary. This could be especially important for patients with advanced disease where time is of the essence. Nevertheless, standard laboratory animal models (*e.g*., mice) could be used for further safety and efficacy testing of the regimen. Optionally, the regimen can be tested using cells from the patient to confirm efficacy prior to treatment.

In constructing the fly avatar assays, the following guiding principles should be followed:
(a) The exact mutation of the patient's tumor is not required to be engineered into the fly avatar for the invention to work. All that is required is to up-regulate the activity of orthologs of the patient's genes that demonstrate increased activity, and down-regulate the activity of orthologs of the patient's genes that exhibit decreased activity.
(b) Due to the lethality of the engineered phenotype, the expression of the orthologs should be placed under inducible control so that the lethal activity can be induced at will, *e.g.,* when the larva cultures are fed the drug regimens. (*N.B.,* during fly development, embryos hatch to progress through three larval stages followed by pupation and metamorphosis to adult flies).
(c) While the activity of the orthologs is required to be altered in a larval organ vital to survival, the altered activity need not be confined/targeted solely to that organ - activity of the orthologs can also be altered in other tissues. However, it is critical for the assay that the altered expression/activity occur in an organ vital to survival of the larvae - in this way, one can rapidly test drugs, combinations of drugs and regimens for efficacy by incorporating the drugs into the larval food, using survival of the larvae as the readout.

Methods, compositions and kits are described for generating one or more avatars or avatar armies - preferably using *Drosophila.* The avatars or avatar armies comprise organisms *(e.g., Drosophila,* such as *Drosophila melanogster*) whose genomic, proteomic and/or phenomic profile have been modified such that the activity of an ortholog of disease driver(s) are altered to correspond to the profile of a subject or population of subjects suffering from a disease (*e.g*., cancer). Methods are described for identifying disease drivers and orthologs of disease drivers, as well as constructs or expression vectors for modifying the activity of an ortholog of a disease driver in the avatar.

The avatars or avatar armies may be used to screen, identify or confirm the efficacy of therapeutic regimens for treating a disease in a subject in need thereof. The avatars or avatar armies may be used in one or more screening assays, which involve exposing the avatars or avatar armies to one or more candidate drug regimens and identifying or confirming efficacy of the candidate drug regimens for treating the disease.

Treating a subject suffering from cancer may comprise administering to the subject a therapeutic regimen that was identified, confirmed, or that demonstrates efficacy in the avatar screening assay. One or more such therapeutic regimens for treating a subject suffering from cancer, as well as methods for adjusting a therapeutic regimen to be administered to the subject suffering from cancer can be communicated to the oncologist and ultimately to the patient. Where the therapeutic regimen involves combinations of known drugs where the toxicity and therapeutic indices are known, no further testing may be necessary. This could be especially important for patients with advanced disease where time is of the essence. Nevertheless, standard laboratory animal models (*e.g*., mice) could be used for further safety and efficacy testing of the regimen. Optionally, the regimen can be tested using cells from the patient to confirm efficacy prior to treatment.

### 5.1. GENOME/PROTEOME/PHENOME ANALYSIS OF THE SUBJECT'S TUMOR/CANCER AND IDENTIFICATION OF DISEASE DRIVERS

Methods well known to those skilled in the art can be used for a genomic, proteomic, and/or phenomic analysis of a biological sample(s) obtained from a patient suffering from, cancer. Deep sequencing techniques are advantageously used to capture the full repertoire, and to ensure that the identification of mutations in tumor subpopulations is captured with confidence.

### 5.1.1. Genomic and Functional Genomic Analyses of Patient Samples

Biological material (*e.g*., tissue(s) and/or body fluid(s)) removed from a patient suffering from cancer ideally includes a sample of the patient's cancer cells and/or a portion of the patient's tumor, and may also include a sample of non-cancerous, unaffected cells. This biological sample may be obtained from the patient using techniques known to the skilled artisan and can be manipulated using techniques known in the art to, *e.g.,* extract nucleic acids *(e.g.,* DNA or RNA), protein, or cells. In addition, the history (*e.g*., medical history) of the patient from which the biological sample is removed may be obtained.

In a particular aspect, the tissue(s) and/or body fluid(s) *(e.g.,* blood) removed should include a sample of the patient's tumor/cancer cells as well as, in certain embodiments, a sample of non-cancerous/unaffected cells. Sequence and functional genomic analyses of the diseased tissue (*e.g*., tumor or cancer cell) is compared to such analyses of the patient's unaffected cells, and/or other non-disease controls (which may include samples from unaffected patients, healthy controls), and/or database information. Analysis of the reference sample can be performed on site or can be obtained from the subject's history (*e.g*., medical history). Techniques known to one of skill in the art can be used to obtain the tissue(s) sample and/or body fluid(s) from a patient. In addition, techniques known to one of skill in the art can be used to manipulate the tissue(s) and/or body fluid(s) sample from the patient to, *e.g.,* extract DNA, RNA, protein and cells.

*Genomic analysis:* Sequence analysis of a patient sample is conducted utilizing techniques known to one of skill in the art. For example sequence analysis of a patient sample may include, but is not limited to, DNA sequencing, RNA sequencing, first-generation sequencing, next-generation sequencing (NGS), third generation sequencing (TGS), massively parallel sequencing, whole genome sequencing, whole exome sequencing, single cell sequencing, and global run-on sequencing (GRO-seq). Examples of sequencing reactions include, but are not limited to, whole genome sequencing, whole exome sequence, transcriptome sequencing, single-molecule real time (SMRT) sequencing, paired end sequencing, and dideoxy sequencing. Any of the sequencing methods described in the following references may be used to analyze the sequence of a patient sample: Mardis et al., 2008, Genomics Hum. Genet. 9:387-402, Sun et al., 2015, Cancer Lett 365:1-10, Patel et al., 2013, Cancer Lett 340:152-60, Gao et al., 2015, Viruses 7:4502-28, Rozhkov, 2015, Methods Mol Biol, 1328:217-30, Sequencing Methods Review: A review of publications featuring Illumina® Technology, 2014, Illumina®, and Mabert et al., 2014, Int J Radiat Biol 90:659-77,

In addition, single nucleotide polymorphism (SNP) analysis may be performed on the patient sample using techniques known to one of skill in the art, such as those described in De Wit et al., 2015, Mol Ecol 24:2310-23, Mabert et al., *supra,* and Kumar et al., 2014, Cell Biochem Biophys 68:233-9,

*Functional Genomic*/*Expression analysis:* Expression analysis can be helpful to model the signaling networks that are active in a tumor cell, and provides additional information to determine whether a pathway has been activated. For example, transcriptome analysis can be used to quantify RNA expression in sample, for example, using RNA-Seq. Transcriptome analysis may be performed using techniques known to one of skill in the art, such as those described in Hoeijmakers et al., 2013, Methods Mol Bio 923:221-39; Mutz et al., Curr Opin Biotechnol 24:22-30; Sand et al., 2015, Int J Mol Sci 16:16176-215; and Veneziano et al., 2015, Front Bioeng Biotechnol 3:77,

Proteome analysis may be used to analyze protein expression in a sample. Techniques known to one of skill in the art may be used to perform proteome analysis. For example, mass spectrometry including, but not limited to, tandem mass spectrometry, highresolution mass spectrometry, and high-throughput mass spectrometry, and fluorescence imaging-based methods may advantageously be used to perform proteome analysis. Proteome analysis may comprise two-dimensional electrophoresis or protein microarray. Proteome analysis may be performed as described in Mustafa et al., 2015, World J Hepatol 7:1312-24, Deutsch et al., 2015, J Am Med Inform Assoc 22:495-506, Feist et al., 2015, Int J Mol Sci 16:3537-63, Alvarez-Chaver et al., 2014, World J Gastroneterol 20:3804-24, and Ohno et al., 2014, Molecules 19:1392-47,

Global analysis of protein activities in a patient sample may be performed as described in Zhu et al., 2001, Science 293:2101-5,

### 5.1.2. Bioinformatics and Computational Assessment for Identification of Candidate Disease Drivers

Bioinformatics (computational biology) can be used to analyze the genomic and functional genomic data obtained from the patient samples compared to controls in order to identify candidate disease drivers. The computational assessment may include generating a genomic, proteomic, and/or phenomic profile of a control (*e.g*., non-diseased or healthy) or by comparison to genomic, proteomic, and/or phenomic information obtained from a control sample. The genomic, proteomic, and/or phenomic information may be obtained from the control sample by any of the methods or steps described, *supra.* Alternatively, the genomic, proteomic, and/or phenomic profile may be generated from one or more genomic, proteomic, phenomic, and/or disease databases. Examples of databases include, but are not limited to, Personal Genome Project, 1000 Genomes Project, The Human Protein Atlas, PhenCode, PhenomicDB, Planform and Limbform. The genomic, proteomic, and/or phenomic profile may be based on genomic, proteomic, and/or phenomic information obtained from one or more databases. The genomic, proteomic, and/or phenomic profile may be based on genomic, proteomic, and/or phenomic information obtained from one or more subjects from one or more databases. For example, a genomic profile of the control sample may be generated from the genomic information obtained from conducting whole genome sequencing of the nucleic acid molecules in the control sample. The computational assessment may comprise ranking or selecting one or more genes, proteins and/or phenotypes from a list of genes, proteins and/or phenotypes based on genomic, proteomic, and/or phenomic information from a control sample, one or more databases and/or one or more subjects from one or more databases. The subjects from the one or more databases may be a subject(s) who is not suffering from cancer. The subjects from one or more databases may be healthy.

In some instances, the genomic, proteomic, and/or phenomic profile of a cancer may be generated from one or more genomic, proteomic, phenomic, and/or cancer databases. Examples of databases include, but are not limited to, Catalog of Somatic Mutation in Cancer (COSMIC), IntOGen, Diagnostic Mutation Database, DNA-mutation Inventory to Refine and Enhance Cancer Treatment (DIRECT), National Human Genome Research Institute, Breast Cancer Information Core (BIC), and Clinical Genomic Database (CGD).

The computational assessment may further comprise selecting a subset of the genomic, proteomic, and/or phenomic information from one or more databases. For example, proteomic information from a database may comprise information related to the activity of all proteins (*e.g*., entire proteome), whereas the proteomic profile of a cancer may comprise proteomic information pertaining to a specific organ (*e.g*., kidney). The computational assessment may comprise selecting a subset of the genomic, proteomic, and/or phenomic information from the genomic, proteomic and/or phenomic profile from one or more databases. For example, a proteomic profile from a database may comprise the activity profile for all protein kinases (*e.g*., kinome profile), whereas a proteomic profile of a disease may comprise an activity profile for a subset of protein kinases (*e.g*., serine/threonine kinases).

The computational assessments disclosed herein may comprise selecting proteins that are most likely to have an altered activity in a subject suffering from cancer as compared to a subject who is not suffering from cancer. The computational assessment may comprise selecting phenotypes that are most likely to be altered in a subject suffering from cancer as compared to a subject who is not suffering from cancer. In some embodiments, if there is conflicting genomic, proteomic, and/or phenomic information relating to a gene, protein, or phenotype, the computational assessment may further comprise ranking the genes, proteins and/or phenotypes by their likelihood of being associated with cancer. Methods for determining the likelihood of a gene, protein and/or phenotype being associated with a disease may comprise using one or more computational tools to prioritize genes, proteins, and/or phenotypes.

### 5.1.3. Identification of Disease Drivers

The candidate disease drivers are analyzed to identify the disease drivers. In a specific embodiment, each DNA mutation (*i.e.,* variant) identified in a patient sample is assessed to determine whether the variant in the patient's tumor alters protein function. Meta-analysis software including MutationAssessor can be used to query different computer programs that provide a "score" for each variant. In a specific embodiment, each variant is examined against the literature and against other patients' mutation profiles to determine whether it occurs in an above-random frequency, within functional domains, etc. In another specific embodiment, the functional genomic data, such as the RNA and proteome expression analyses permits identification of signaling pathways that are over-active *(e.g.,* oncogenes), or inhibited *(e.g.,* tumor suppressors) in the tumor cell. *See* Sections 6, 7 and 8, *infra,* for an example of the application of this analysis.

*Bioinformatics "Scoring" and Ranking of Disease Driver Mutation Candidates.* Any of the computational assessments disclosed herein may comprise using one or more computational tools to prioritize, rank or select genes, proteins, and/or phenotypes from a genomic, proteomic, and/or phenomic profile or from genomic, proteomic and/or phenomic information. The computational tools may be used to generate a genomic, proteomic, and/or phenomic profile of a cancer, a reference sample, and/or control sample. The computational tools may be used to generate a consensus genomic, proteomic, and/or phenomic profile of a disease, a reference sample, and/or control sample. Examples of computational tools that can be used in a computational assessment include, but are not limited to, aGeneApart, BioGraph, Biomine, Bitola, Candid, CGI, DIR, DomainRBF, Endeavour, G2D, GeneDistiller, GeneFriends, GeneProspector, GeneRank, GeneRanker, GeneSeeker, GeneWanderer, Gnie, GenTrepid, GLAD4U, GPSy, GUILD, MedSim, MetaRanker, MimMiner, PGMapper, PhenoPred, Pinta, PolySearch, PosMed, PRINCE, ProDiGe, ProphNet, S2G, SNPs3D, TargetMine. Alternatively, or additionally, a computational assessment may comprise use of one or more computational tools as described in Moreau and Tranchevent, 2012, Nat Rev Genet 13:523-36, Oti et al., 2011, Methods Mol Biol 760:189-206, Masoudi-Nejad et al., 2012, Mol Genet Genomics 287:679-98, Zhu et al., 2014, Adv Exp Med Biol 799:69-84, Zhang et al., 2014, Mol Biosyst 10:1400-8, Bornigen et al., 2012, Bioinformatics 28(23):3081-8), Wang and Marcotte, 2010, J Proteomics 73:2277-89,

*Functional Analysis of Candidate Disease Drivers.* Examples of mutations that could be detected in the disease driver (*e.g*., tumor driver) include, but are not limited to, a missense mutation, nonsense mutation, insertion, deletion, duplication, frameshift mutation, inversion, translocation, or repeat expansion. The disease driver (*e.g*.,. tumor driver) may contain a structural variation. A mutation may occur in a coding region, promoter, termination sequence, and/or enhancer region of a disease driver (*e.g*., tumor driver). A mutation may occur in an exon, intron, or untranslated region of a disease driver (*e.g*., tumor driver). The methods may further comprise conducting an analysis of the one or more mutations identified in the disease driver (*e.g*., tumor driver) to determine whether the mutation is likely to disrupt the function of a protein encoded by the disease driver (*e.g*., tumor driver).

Determining whether the mutation is likely to disrupt the function of the protein may comprise determining whether the mutation has been previously identified in a publication or database as disrupting or likely to disrupt the function of the protein. For example, the computational assessment may comprise determining whether the mutation has been identified in a scientific article as disrupting or likely to disrupt the function of the protein. In another example, the computational assessment may comprise determining whether the mutation has been identified in a database as disrupting or likely to disrupt the function of the protein.

Alternatively, or additionally, determining whether the mutation is likely to disrupt the function of the protein may comprise conducting a structural analysis of likely active protein domains (*e.g*., putative active protein domains). The method may further comprise determining whether the mutation occurs in the putative active protein domain. If the mutation occurs in the putative active protein domain, then the mutation is likely to disrupt the function of the protein. If the mutation does not occur in the putative active protein domain, then the mutation is unlikely to disrupt the function of the protein. One or more databases may be used to determine whether the mutation occurs in a putative active protein domain. Examples of databases include, but are not limited to, InterPro protein, NCBI Conserved Domain Search, ExPASy PROSITE, Simple Modular Architecture Research Tool (SMART), Class Architecture Topology Homology (CATH), Dali Domain Dictionary, and ProDom. Additional databases are described in Liu et al., 2004, Nucleic Acids Res 32:3522-30,

The computational assessments may further comprise identifying disease drivers *(e.g.,* tumor drivers) that are most likely to cause the cancer in a subject or in a population of subjects. The computational assessment may comprise ranking the disease drivers (*e.g*., tumor drivers) by their likelihood of causing the disease. One or more databases may be used to identify and/or rank disease drivers that are most likely to cause the disease. Examples of databases include, but are not limited to, Catalog of Somatic Mutation in Cancer (COSMIC), IntOGen, Diagnostic Mutation Database, DNA-mutation Inventory to Refine and Enhance Cancer Treatment (DIRECT), National Human Genome Research Institute, Breast Cancer Information Core (BIC), and Clinical Genomic Database (CGD). Identifying and/or ranking the disease drivers (*e.g*., tumor drivers) may comprise conducting an analysis of previously published data, previous subject data indicating the variant is shared between subjects, and genetic modifier tests to determine whether reducing a candidate gene's activity enhances the *Drosophila* model's phenotype with a subset of variants modeled.

*Orthologs of disease drivers.* The computational assessment may be used to identify an ortholog of a disease driver *(e.g.,* tumor driver) in an organism *(e.g., Drosophila,* such as *Drosophila melanogaster)* other than the subject. The organism may be an invertebrate organism. Examples of invertebrate organisms include, but are not limited to, worms, fish, and flies. The organism may be of a different genus from the genus of the subject. The organism may be of a different species from the species of the subject. The organism may be of the order *Diptera.* The genus of organism may be *Drosophila.* The subgenus of organism may be *Drosophila.* The species of the organism may be *Drosophila melanogaster.* The organism may be a *Drosophila melanogaster, Caenorhabditis elegans* or *Danio rerio.*

Identifying the ortholog may comprise identifying an ortholog in the organism (*e.g*., fly, such as *Drosophila)* that can be manipulated to recapitulate the activities found in a population of subjects suffering from cancer. Identifying the ortholog may comprise identifying an ortholog in the organism *(e.g.,* fly, such as *Drosophila)* that can be manipulated to recapitulate the activities found in a reference sample (*e.g*., tumor or cancer cell), in a population of subjects suffering from cancer. A gene or protein in the organism may be identified as an ortholog of a disease driver if the gene or protein is functionally similar to the disease driver. In some embodiments, the functional similarity of a gene or protein in the organism to the disease driver is determined by gene ontology (GO), IntelliGO, and EnzDP. The functional similarity of the gene or protein in the organism to the disease driver may be determined by any method known to one of skill in the art, such as any of the methods described in Teng et al., 2013, Bioinformatics 29:1424-32, Benabderrahmane et al., 2010, BMC Bioinformatics 11:588, and Nguyen et al., 2015, J Bioinform Comput Biol 13:1543003,

One or more databases may be used to identify an ortholog. Examples of databases that can be used to identify orthologs include, but are not limited to, Flybase, Homologene, TreeFam, OrthoMCI, MetaPhOrs, P-POD (Princeton Protein Orthology Database), KEGG Orthology, EggNog, POGS (plant-specific), OrthoDB, Roundup, ProtozoaDB, and Gene-Oriented Orthology Database. Additional databases may include any of the databases described in Pandey et al., 2011, Pharmacol Rev 63:411-36 and Kotowski et al., 2015, Parasit Vectors 8:494,

Examples of orthologs of disease drivers can include, but are not limited to cell cycle regulators, such as, cdc2, cdacapo, E2F transcription factor, and retinoblastoma-family protein; DNA damage checkpoint proteins, such as meiotic 41 and telomere fusion; members of the epidermal growth factor receptor/Ras/MapK pathway, such as Cbl, EGF receptor, pointed, pole hole (common alternative name: raf), Ras oncogene at 85D, Rolled, sprouty, and Vav ortholog; FGF and FGF receptor, such as breathless and branchless; members of the Hedgehog pathway, such as cubitus interruptus and patched; members of the Jak/Stat pathway, such as hopscotch and Signal-transducer and activator of transcription protein at 92E; members of the JNK pathway, such as basket, CYLD, kayak/Fos-related antigen, and Jun-related antigen; junctional proteins and their interactors, such as discs large 1, fat, FER ortholog (H. sapiens) (Fps oncogene analog), grindelwald, lethal (2) giant larvae, Merlin, Sanpodo, rapsynoid (also known as partner of inscutable), scribbled and shotgun; members of the Notch pathway, such as Notch and numb, ovarian tumor genes, such as bag of marbles, benign gonial cell neoplasm, fused, mei-P26, ovarian tumor, Sans fille and sex lethal, members of the PI3K/PTEN/AKT and Tor pathways, such as Akt1, gigas (Tsc2), phosphotidylinositol 3 kinase 92E, Pten, Rheb, target of rapamycin and Tsc1; members of the Warts/Hippo pathway, such as dachs, capping protein alpha & capping protein beta, expanded, fat, hippo, kibra, lethal (3) malignant brain tumor, Merlin, mob as tumor suppressor, myopic, salvador, warts, and yorkie; members of the Wingless pathway, such as APC-like, adenomatous polyposis coli tumor suppressor homolog 2, armadillo, Medea, Mothers against dpp, and wingless; and members of the Myc-Max-Mad/Mnt network, such as diminutive/Myc and Mnt. An ortholog of a disease driver may be selected from the group consisting of Abl oncogene, AMP-activated protein kinase alpha subunit (SNF1A), anastral spindle 2, Aos1, archipelago, Axud1, brain tumor, Beadex, C-terminal Src kinase, death executioner Bcl-2 homologue, Btk family kinase at 29A, casein kinase II, Csk, eiger, eukaryotic initiation factor 4E, extradenticle, insulin-like peptide 8, klumpfuss, lkb1, Menin 1, microtubule star, miranda, Myb oncogene-like, posterior sexcombs and suppressor two of zeste, prospero, runt, p53, snoN, Src oncogene at 42A, Src oncogene at 64B, Snf5-related 1, trithorax, tout-velu, Ubiquitin activating enzyme 1, von Hippel-Lindau, vps25, members of the TGFβ/BMP pathway, members of nuclear receptor pathways, and members of G protein-coupled pathways.

Where the disease driver is an oncogene, the oncogene(s) may include but is not limited to abl, Af4/hrx, akt-2, alk, alk/npm, aml1, aml1/mtg8, axl, bcl-2, bcl-3, bcl-6, bcr/abl, c-myc, dbl, dek/can E2A/pbx1, EGFR, enl/hrx, erg/TLS, erbB, erbB-2, ets-1, ews/fli-1, fms, fos, fps, gli, gsp, Her2/neu, hox11, hst, IL-3, int-2, jun, kit, KS3, K-sam, Lbc, lck, lmo1, lmo2, L-myc, lyl-1, lyt-10/C alpha1, mas, mdm-2, mll, mos, mtg8/aml1, myb, MYH11/CBFB, neu, N-myc, ost, pax-5, pbx1/E2A, pim-1, PRAD-1, raf, RAR/PML, rash, rasK, rasN, rel/nrg, ret, rhom1, rhom2, ros, ski, sis, set/can, src, tall, tal2, tan-1, Tiam1, TSC2, and/or trk.

Where the disease driver is a tumor suppressor, the tumor suppressor(s) may include but is not limited to APC, BRCA1, BRCA2, CDKN2A, DCC, DPC4 (SMAD4), MADR2/JV18 (SMAD2), MEN1, MTS1, NF1, NF2, p53, PTEN, Rb, VHL, WRN, and/or WT1.

### 5.2. TRANSGENIC "AVATARS" THAT RECAPITULATE DISEASE PROFILES

Once the set of variants is established, individual fly models that target up to about 15 to 20 (or more) patient driver mutations can be engineered. It is not critical to reproduce the patient's exact mutation(s) into the fly ortholog of the disease driver(s). The orthologs are engineered so that activity of the disease drivers is dialed up or down to correspond to the patient's phenome.

If a patient's genomic/functional genomic analysis identifies 10 or fewer "hits" (*i.e.,* overactivity or underactivity of gene products/pathways in the diseased tissue as compared to controls), a fly avatar can be constructed that includes all of the fly variant orthologs as described below. However, if a patient has more than ten altered protein activities, a "base" cancer avatar may be generated using a subset of the variants that are identified as functional drivers of tumor progression and/or disease progression. The remaining candidate genes can then be used in a dominant genetic modifier screen to identify those genes that are functional drivers of tumor progression. For example, the level of expression of one or more of the remaining genes in the base cancer avatar can be modified by techniques known to one of skill in the art *(e.g.,* by drugs and/or expression of a cDNA encoding a gene and/or a cDNA encoding a dominant negative) to determine if those one or more remaining genes are functional drivers of tumor progression.

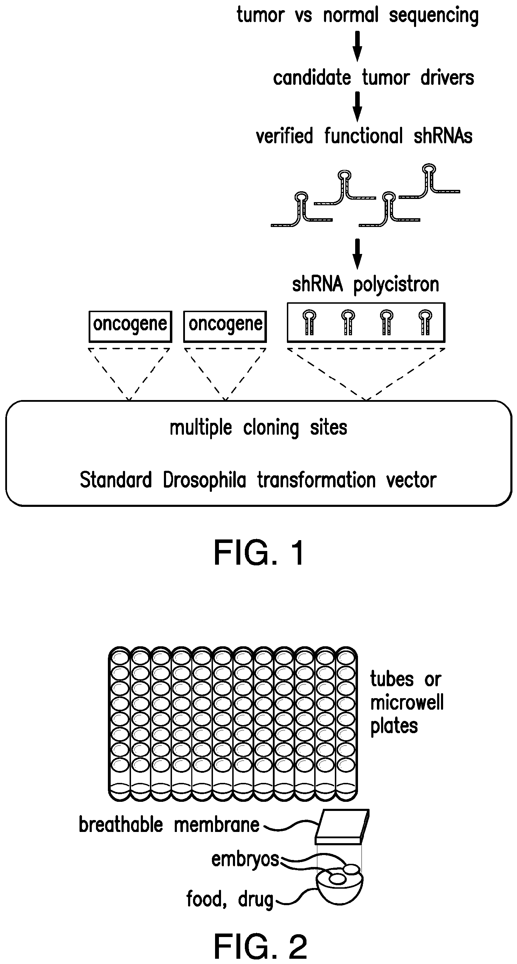

A fly *(e.g., Drosophila)* can be engineered so that the genetic variants in the patient's tumor are recapitulated in the fly embryos and activated in an organ vital to survival of larvae; *e.g*., hindgut. The disease driver activity need not be confined/targeted to the vital organ, but is necessary to be active in that organ, and it is preferred that activity be designed to be controlled at will to facilitate screening. *See, e.g.,* Figure 1 which outlines the process of identifying the disease drivers and the generation of shRNAs for silencing genes.

In a specific embodiment, the tumor drivers of a patient's cancer that are identified include one or more oncogenes and/or one or more tumor suppressors. This information can then be used to build an avatar *(e.g.,* a fly avatar, such as a *Drosophila* avatar). For example, a fly *(e.g., Drosophila)* may be genetically modified such that upon induction through an external factor (*e.g*., temperature or exposure to light at a specific wavelength) there is an increase in the activity of an ortholog(s) of the subject's oncogene(s) and/or inhibition an ortholog(s) of the subject's tumor suppressor(s) in the tissue/organ of a fly *(e.g., Drosophila)* that is necessary for survival to pupation. In a specific embodiment, the activity of the ortholog of the oncogene is increased by at least 10%, 20%, 30%, 40%, 50%, 60%, 70%, 80%, 90%, 100%, 125%, 150%, 175%, 200%, 250%, or 300% or more in an avatar (*e.g*., fly avatar, such as a *Drosophila* avatar) as compared to the activity of the ortholog of the oncogenes in the non-modified organism (*e.g*., fly, such as *Drosophila).* In another specific embodiment, the activity of the ortholog of the tumor suppressor is decreased by at least 10%, 20%, 30%, 40%, 50%, 60%, 70%, 80%, 90%, 100%, 125%, 150%, 175%, 200%, 250%, or 300% or more in an avatar (*e.g*., fly avatar, such as a *Drosophila* avatar) as compared to the activity of the ortholog of the oncogenes in the non-modified organism (*e.g*., fly, such as *Drosophila).*

The avatars or avatar armies disclosed herein may comprise an organism (*e.g*., *Drosophila,* such as *Drosophila melanogster*) that has been modified to overexpress an ortholog of a disease driver (*e.g.*, tumor driver, such as an oncogene). Alternatively, or additionally, the avatars or avatar armies disclosed herein may comprise an organism *(e.g., Drosophila,* such as *Drosophila melanogster*) that has been modified to underexpress an ortholog of a disease driver (*e.g*., tumor driver, such as a tumor suppressor). In some embodiments, the avatars or avatar armies disclosed herein comprise an organism *(e.g., Drosophila,* such as *Drosophila melanogster*) that has been modified to overexpress an ortholog of a disease driver (e.g., tumor driver, such as an oncogene) and to underexpress an ortholog of a disease driver (e.g., tumor driver, such as a tumor suppressor). In some embodiments, altering the expression or activity of the ortholog of a disease driver in the organism results in a lethal phenotype or barely dead phenotype. In some embodiments, altering the expression or activity of two or more orthologs of disease drivers in the organism increases the severity of the lethal or barely dead phenotype. In some embodiments, the severity of the lethal or barely dead phenotype is titrated by varying the number and types of orthologs of diseases drivers whose expression or activity are altered in the organism.

In some embodiments, the avatar or avatar armies disclosed herein comprises *a ptc > dRet^{MEN2B}* organism *(e.g., Drosophila).* In some embodiments, the avatar or avatar armies disclosed herein does not comprise a *ptc > dRet^{MEN2B}* organism *(e.g., Drosophila).* In some embodiments, the avatar or avatar armies disclosed herein comprises a UAS-Ret2B organism *(e.g., Drosophila).* In some embodiments, the avatar or avatar armies disclosed herein does not comprise a UAS-Ret2B organism *(e.g., Drosophila).* In some embodiments, the avatar or avatar armies disclosed herein comprises a UAS-RasV12 organism *(e.g., Drosophila).* In some embodiments, the avatar or avatar armies disclosed herein does not comprise a UAS-RasV12 organism *(e.g., Drosophila).*

### 5.2.1. Genetic Engineering of Avatars

Provided herein are transgenes that can modulate the expression of orthologs of disease drivers. The transgene may comprise a cDNA of the ortholog of a disease driver *(e.g.,* tumor driver), a cDNA of an activator of the ortholog of a disease driver, a cDNA of an inhibitor of an ortholog of a disease driver, an interfering RNA *(e.g.,* small interfering RNA (siRNA) or short hairpin RNA (shRNA)) that targets the ortholog of the disease driver (*e.g*., tumor driver), or a combination thereof. The interfering RNA molecule may comprise one or more shRNAs that that target one or more orthologs of one or more disease drivers (*e.g*., tumor drivers). In a specific embodiment, a transgene comprises a cDNA of an ortholog of an oncogene. The transgene may comprise 1, 2, 3, 4, 5, 6, 7, 8, 9, 10 or more cDNAs of an ortholog of 1, 2, 3, 4, 5, 6, 7, 8, 9, 10 or more oncogenes. In another specific embodiment, a transgene comprises an interfering RNA *(e.g.,* small interfering RNA (siRNA) or short hairpin RNA (shRNA)) that targets the ortholog of a tumor suppressor. The transgene may comprise 1, 2, 3, 4, 5, 6, 7, 8, 9, 10 or more interfering RNAs of an ortholog of 1, 2, 3, 4, 5, 6, 7, 8, 9, 10 or more tumor suppressors. In another specific embodiment, a transgene comprises at least cDNA of an ortholog of an oncogene and at least interfering RNA that targets the ortholog of a tumor suppressor.

To model reduced activity of multiple tumor suppressors a series of interfering RNAs, such as small hairpin RNAs (shRNAs) or small interfering RNAs (siRNAs), each designed to reduce activity of a single tumor suppressor, can be fused together by standard cloning methods. The resulting oligonucleotide can then be fused 3' to a promoter (*e.g*., an inducible promoter element or upstream activating system (*UAS*) promoter). The result is a single genetic element that can silence as many as ten or more genes. In certain embodiments, the genetic element is cloned into a multiple cloning site of a plasmid. In specific embodiments, the genetic element is cloned into a remaining multiple cloning site within a plasmid carrying an over-expressed oncogene(s) as described herein. The result is a single transformation plasmid that targets multiple oncogenes and tumor suppressors designed to model a single tumor. In a specific embodiment, the single transformation plasmid comprises 1, 2, 3, 4, 5, 6, 7, 8, 9, 10, 11, 12, 13, 14 or 15 interfering RNAs, such shRNAs. In another specific embodiment, the single transformation plasmid comprises 1, 2, 3, 4, 5, 6, 7, 8, 9, 10, 11, 12, 13, 14 or 15 overexpressed orthologs. In another specific embodiment, the single transformation plasmid comprises 1, 2, 3, 4, 5, 6, 7, 8, 9, 10, 11, 12, 13, 14 or 15 interfering RNAs (*e.g*., shRNAs) and 1, 2, 3, 4, 5, 6, 7, 8, 9, 10, 11, 12, 13, 14 or 15 overexpressed orthologs.

To elevate activity of fly oncogene orthologs *(e.g., Drosophila* oncogene orthologs), a transgene(s) representing an ortholog(s) of each of the patient's oncogenes can be fused to a promoter *(e.g.,* an inducible promoter or *UAS* promoter) and cloned into a multiple cloning site contained within a transformation plasmid. The transgene(s) can be the unaltered sequence or can contain activating mutations that model activation of the human ortholog within the patient's tumor. In a specific embodiment, the single transformation plasmid comprises 1, 2, 3, 4, 5, 6, 7, 8, 9, or 10 overexpressed orthologs.

In a specific embodiment, the transgene(s) representing an ortholog(s) of a patient's ortholog is an unaltered sequence. In another embodiment, the trangene(s) representing an ortholog(s) of a patient's oncogene(s) contains an activating mutation that models the activation of the human ortholog. The ortholog(s) of the one or more disease drivers may be modified by introducing a mutation in the ortholog that alters protein function to recapitulate the patient's phenotype. The ortholog of the one or more disease drivers may be modified such that it contains the same mutation as the disease driver, although this is not required - as long as the activity of the disease driver is up- or down-regulated in a way that recapitulates the phenome of the patient.

The mutation in the ortholog(s) of the disease driver(s) may be introduced in a coding region, promoter, termination sequence, and/or enhancer region of a disease driver (*e.g*,. tumor driver). The mutation may occur in an exon, intron, or untranslated region of a disease driver (*e.g,.* tumor driver). The one or more mutations in the disease driver(s) may be a deletion, an insertion, a substitution, and/or a frameshift mutation, to name a few. Alternatively, where inhibition of activity of the disease driver is desired, the ortholog need not be mutated - rather RNAi silencers (such as the shRNAs described above) can be employed.

In a preferred embodiment, the expression of a transgene(s) is controlled and regulated at will. Inducible control of the transgene within the transgenic fly model *(e.g.,* transgenic *Drosophila* model) can be used to ensure that the engineered ortholog(s) is not expressed until the avatar founder lines are established and the larvae are ready to be fed the drug regimen(s). In a specific embodiment, an inducible promoter that is temperature sensitive is used to regulate transgene expression so that an increase in culture temperature can be used to turn on the transgene, which in turn modulates the expression of a disease driver *(e.g.,* a tumor suppressor or oncogene). For example, when expression of the transgene is regulated in a temperature sensitive manner, the transgene(s) is expressed when the avatar is cultured at a certain temperature *(e.g.,* 20° C to 29° C) but not at another temperature *(e.g.,* 18° C). *See, e.g.,* Sections 6, 7 and 8, *infra,* for examples of temperature sensitive systems and this Section 5.2.1, *infra,* for information regarding expression systems. In one example, a transgene is linked to a promoter (*e.g., UAS*) regulated by a transcriptional activator *(e.g.,* GAL4) and the activator is linked to a temperature sensitive promoter so only when expression of the transcriptional activator is induced at a certain temperature is the transgene expressed. In another example, a transgene is linked to a promoter regulated by a transcriptional repressor and the repressor is linked to a temperature sensitive promoter so only when expression of the repressor is inhibited at a certain temperature is the transgene expressed. In yet another example, a transgene is linked directly to a temperature sensitive promoter. The induction of expression of a cDNA of an ortholog of a oncogene increases the expression of the ortholog, whereas the induction of an interfering RNA reduces the expression of a tumor suppressor. Expression of the transgene should result in a change in the genomic profile, proteomic profile, and/or phenomic profile of the organism as compared to non-modified organism. *See, e.g.,* this Section 5.2.1, *infra,* and Sections 6, 7, and 8, *infra,* for additional information regarding at will expression of the transgene and inducible promoters.

In a preferred embodiment, expression of a transgene should be engineered to occur in an organ vital to the survival of the avatar. The expression of a transgene in an organ vital to survival of the avatar enables high throughput, rapid read out - rescue from lethality upon induction of the transgene. Examples of vital organs of the fly *(e.g., Drosophila)* include, but are not limited to, the hindgut, midgut imaginal disc, nervous system, fat body, nephrocyte, heart, trachea, thorax, abdomen, and malphigian tubules. Expression of the transgene may also occur outside an organ vital to the survival of the organism and in another cell type, tissue or organ of the organism. For example, expression of the transgene may occur in the hindgut, wing, ovaries, genitalia, or eye of the organism. *See, e.g.,* Sections 6, 7 and 8, *infra,* for techniques resulting in expression of a transgene in a vital organ of the fly *(e.g., Drosophila).*

In a specific embodiment, a transgene(s) is regulated (directly or indirectly) by an inducible promoter such that when expression of the transgene(s) in an organ vital to survival *(e.g.,* the hindgut) is induced it results in 80%, 85%, 86%, 87%, 88%, 89%, 90%, 91%, 92%, 93%, 94%, 95%, 96%, 97%, 98%, 99% or 100% lethality of the pupa or the larvae stage prior to pupariation (e.g, stage 4 of the larvae) and 98%, 99% or 100% lethality of adult flies. In another embodiment, a transgene(s) is regulated (directly or indirectly) by an inducible promoter such that when expression of the transgene(s) in an organ vital to survival (*e.g*., the hindgut) is induced it results in 80% to 90%, 85% to 95%, 85% to 99%, 80% to 100%, or 90% to 100% lethality of the pupa or the larvae stage prior to pupariation (e.g, stage 4 of the larvae) and 98%, 99% or 100% lethality of adult flies.

*Constructs and Expression Systems* Also, provided herein are constructs *(e.g.,* DNA plasmids) comprising a transgene(s). In one embodiment, a construct for altering the expression level or activity of an ortholog of a disease driver *(e.g.,* tumor driver) in an organism *(e.g.,* fly) comprises one or more interfering RNAs (RNAi) (*e.g*., small hairpin RNAs (shRNAs) or small interfering RNAs (siRNAs)). In certain embodiments, the construct comprises at least two RNAi (*e.g*., shRNAs or siRNAs). In certain embodiments, the construct comprises 2, 3, 4, 5, 6, 7, 8, 9, or 10 or more interfering RNAs (*e.g*., shRNAs or siRNAs). In some embodiments, the construct further comprises the cDNA of 1, 2, 3, 4, 5, 6, 7, 8, 9, or 10 or more orthologs of disease drivers *(e.g.,* tumor drivers). In certain embodiments, the construct further comprises more inhibitors of orthologs of disease drivers. The construct may further comprise one or more promoter elements. The one or more promoter elements may be an inducible promoter element. The inducible promoter element may be an upstream activator sequence (*UAS*) promoter element. The fused RNAi (*e.g*., shRNAs or siRNAs) may be fused to the promoter element. The one or more cDNAs may be fused to the promoter element. The construct may comprise two or more promoter elements. The two or more promoter elements may be different. The two or more promoter elements may be the same. The construct may further comprise one or more recombinase recognition sites. The two or more recombinase recognition sites may flank the one or more RNAi (*e.g*., shRNAs or siRNAs), one or more cDNAs, or both. The recombinase recognition site may be an attP site. The construct may comprise two or more attP sites.

In another embodiment, a construct for altering the expression level or activity of an ortholog of a disease driver *(e.g.,* tumor driver) in an organism *(e.g.,* fly) comprises cDNA of an ortholog of a disease driver (*e.g*., tumor drivers, such as oncogenes). In some embodiments, the construct further comprises the cDNA of 1, 2, 3, 4, 5, 6, 7, 8, 9, or 10 or more RNAi *(e.g.,* shRNAs or siRNAs) that target orthologs of disease drivers (*e.g*., tumor drivers, such as tumor suppressors). In certain embodiments, the construct further comprises more inhibitors of orthologs of disease drivers. The construct may further comprise one or more promoter elements. The one or more promoter elements may be an inducible promoter element. The inducible promoter element may be an upstream activator sequence (*UAS*) promoter element. The one or more cDNAs may be fused to the promoter element. The construct may comprise two or more promoter elements. The two or more promoter elements may be different. The two or more promoter elements may be the same. The construct may further comprise one or more recombinase recognition sites. The two or more recombinase recognition sites may flank the one or more RNAi (*e.g*., shRNAs or siRNAs), one or more cDNAs, or both. The recombinase recognition site may be an attP site. The construct may comprise two or more attP sites.

In some embodiments, a construct for altering the expression level or activity of an ortholog of a disease driver *(e.g.,* tumor driver) in an organism *(e.g.,* fly) comprises at least one RNAi molecule *(e.g.,* shRNA or siRNA) that targets an ortholog of a disease driver *(e.g.,* tumor drivers, such as tumor suppressor) and at least one cDNA of an ortholog of a disease driver *(e.g.,* tumor drivers, such as oncogenes). The construct may comprise at least one shRNA that targets an ortholog of a disease driver (*e.g*., tumor drivers, such as tumor suppressor), at least one promoter and at least one cDNA of an ortholog of a disease driver (*e.g*., tumor drivers, such as oncogenes).

In certain embodiments, a construct for altering the expression level or activity of an ortholog of a disease driver *(e.g.,* tumor driver) in an organism *(e.g.,* fly) comprises at least one RNAi molecule (*e.g*., shRNA or siRNA) that targets an ortholog of a disease driver and at least one cDNA of an activator of ortholog of a disease driver. The construct may comprise at least one shRNA that targets an ortholog of a disease driver, at least one promoter and at least one cDNA of an activator of an ortholog of a disease driver.

In some embodiments, a construct for altering the expression level or activity of an ortholog of a disease driver *(e.g.,* tumor driver) in an organism *(e.g.,* fly) comprises at least one RNAi molecule (*e.g*., shRNA or siRNA) that targets an ortholog of a disease driver and at least one cDNA of an inhibitor of ortholog of a disease driver. The construct may comprise at least one shRNA that targets an ortholog of a disease driver, at least one promoter and at least one cDNA of an activator of an ortholog of a disease driver.

In some embodiments, the construct for altering the expression level or activity of an ortholog of a disease driver *(e.g.,* tumor driver) in an organism *(e.g.,* fly) is a DNA plasmid and a transgene is inserted into a region between two inverted repeat elements on the DNA plasmid. The inverted repeat element may be a P element. Alternatively, the transgene may be inserted into a region between two recombination sites *(e.g.,* attP site) into a construct for altering the expression level or activity of an ortholog of a tumor driver in an organism (*e.g*., fly).

In some embodiments, a construct for altering the expression level or activity of an ortholog of a tumor driver in an organism (*e.g*., fly) comprises a flipase (Flp)-out cassette. A Flp-out cassette may comprise two flipase recognition target sequences (FRTs). The construct may further comprise a transgene located between the two FRTs. The transgene may be flanked by FRT sites, which result in silencing of the transgene. Expression of the transgene can occur by excision of the FRT sites by flipase (Flp). The construct may comprise a Flp-out cassette placed between a promoter and a transgene.

In certain embodiments, a construct for altering the expression level or activity of an ortholog of a tumor driver in an organism (*e.g*., fly) comprises a ΦC31-integrase mediated cassette and a transgene. The ΦC31-integrase mediated cassette comprises two attP sites. The transgene may be located between the two attP sites. The ΦC31-integrase mediated cassette is described in Bateman et al., 2006, Genetics 173:769-77,

Exemplary expression systems that can be used for introducing a transgene into an organism include, but are not limited to, GAL4-UAS, LexA-*lex*Aop, and/or QF-QUAS systems. The methods for using or generating GAL4-UAS, LexA-*lex*Aop, and/or QF-QUAS systems to alter gene *(e.g.,* ortholog) expression are described in del Valle Rodriguez et al., *supra,* Chan et al., 2015, PLoS One, 10(9):e0138181, Potter et al., 2010, Cell, 141(3):536-48, and Luan et al., 2006, Neuron, 52(3):425-36

The expression system that can be used for introducing a transgene into an organism may be a binary expression system. The binary expression system may comprise a transcriptional activator and a transgene under the control of a promoter that is under the control of the transcriptional activator. The transcriptional activator may be expressed in a specific pattern *(e.g.* in a specific cell type or organ). Alternatively, the transcriptional activator may be randomly expressed. Examples of binary expression systems include, but are not limited to, GAL4-UAS, LexA-*lex*Aop, and QF-QUAS systems.

The expression system that can be used for introducing a transgene into an organism may comprise a ternary expression system. The ternary expression system may comprise a transcriptional activator, a transgene under the control of a promoter, and a repressor of the transcriptional activator. The promoter that controls the transgene may be under the control of the transcriptional activator. Examples of ternary expression systems include, but are not limited to, GAL80, split Gal4 and split LexA.

The expression system that can be used for introducing a transgene into an organism may comprise a modular expression system. The modular expression system may comprise a convertible genetic platform. Generally, a modular expression system uses multiple recombinase systems to swap a transcriptional activator sequence with another sequence. For example, the integrase swappable *in vivo* targeting element (InSITE) system uses three different site-specific recombinases (*e.g*., Flp-FRT, Cre recombinase-Lox, and ΦC31 integrase-attP/attB) to swap *GAL4* with another sequence. Additional details on the InSITE system is described in Gohl et al., 2011, Nat Methods 8:231-7,

The expressions systems may be used for cell- or tissue-specific gene expression or RNA interference. The expression systems may comprise one or more promoters that control the expression of the transgene. The one or more promoters may restrict the expression of the transgene to one or more cell types or tissues. The one or more promoters may restrict the expression of the transgene to specific temperatures or time periods. The expression system may comprise one or more inducible promoters. The expression systems may comprise one or more cell- or tissue-specific promoters. Examples of cell- or tissue-specific promoters include, but are not limited to, GMR promotor, Byn promoter, Btl promoter, Eyeless promoter, 765 promoter, Sd promoter, Ptc promoter, Omb promoter, B29 promoter, CD14 promoter, CD43 promoter, CD45 promoter, CD68 promoter, desmin promoter, elastase-1 promoter, endoglin promoter, fibronectin promoter, Flt-1 promoter, GFAP promoter, GPIIb promoter, ICAM-2 promoter, mouse INF-β promoter, Mb promoter, nphsI promoter, OG-2 promoter, SP-B prmoter, SYN1 promoter, WASP promoter, SV40/bAlb promoter, SV40/hAlb promoter, SV40/CD43 promoter, SV40/CD45 promoter, and NSE/RU5' promoter. The expression systems may comprise a heat shock promoter, GAL4/UAS system, GAL4/GAL80/UAS system, EGUF system, FLP/FRTregulated system, QUAS system, and/or lexA system.

The construct or expression system may be temporally expressed in the organism *(e.g.,* fly). Temporal expression of the construct or expression system may comprise expression of the construct or expression system during a specific stage of development. For example, the construct or expression system may be expressed during the adult stage of development of the organism (*e.g*., fly). Alternatively, the construct or expression system may be expressed during the pupal or larval stage of development of the organism (*e.g*., fly). In another example, the construct or expression system may be expressed during the embryonic stage of development of the organism. Tissue-specific and/or temporal expression of the construct or expression vector may be engineered using expression systems as described in Roman et al., 2001, Proc Natl Acad Sci USA, 98:12602-7; McGuire et al., 2004, Trends Genet, 20:384-91,

Preferably, expression of the transgene(s) is controlled at will. For example, expression of the transgene is preferably under the control of an inducible promoter. The inducible promoter may confine expression of the transgene to a specific cell type, tissue or organ, to a specific time, to a specific temperature and/or to a specific light condition. Expression of the transgene can then be induced at will in any cell type, tissue, or organ, at a specific time (*e.g*., stage of development), at any time, for example in response to a change in temperature or light induced by the operator.

Additional examples of constructs and expression systems for altering the expression level or activity of an ortholog of a disease driver (*e.g*., tumor driver) in an organism (e.g., fly) are described in del Valle Rodriguez et al., 2011, Nat. Methods 9:47-55, Roman et al., supra, McGuire et al., supra, Luan et al., 2006, Neuron 52:425-36,

*Cloning Techniques* & *Introduction of Expression System*/*Construct In Organism.*

Cloning techniques known to one of skill in the art may be used to clone a cDNA or RNAi into a construct, such as a plasmid. Methods for cloning a cDNA and/or RNAi molecule into a plasmid may comprise inserting the cDNA and/or RNAi molecule into the multiple cloning site (MCS) of a plasmid. Additional methods for cloning a cDNA and/or RNAi molecule into a plasmid are described in Harbers, 2008, Genomics, 91(3):232-42 and Green and Sambrook, 2012, Molecular Cloning: A Laboratory Manual (Fourth Edition),

Cloning the cDNA and/or RNAi molecule into the plasmid may comprise flanking the cDNA and/or RNAi molecule with inverted repeat elements or recombinase recognition sites. The inverted repeat elements or recombinase recognition sites may be fused to the cDNA and/or RNAi molecule prior to insertion into the plasmid. Alternatively, the plasmid may contain the inverted repeat elements or recombinase recognition sites and the cDNA and/or RNAi molecule may be inserted between the inverted repeat elements or recombinase recognition sites.

Techniques known to one of skill in the art can be used to introduce a construct or expression system into an organism (*e.g*., a fly). Methods for introducing a construct or expression system into an organism include, but are not limited to transfection, injection (*e.g*., microinjection), and viral infection. Additional methods for introducing a construct or expression system into an organism are described in Xue et al., 2014, G3 4:925-9, Pastor-Pareja et al., 2013, Annu Rev Genet 47:51-74; del Valle Rodriguez, *supra,* Katsuyama et al., 2013, Nucleic Acids Res 41e163,

The construct or expression system can be introduced into the organism (*e.g.*, fly) by injection. For example, the construct and/or expression system can be injected into the posterior germ cells of fly embryos.

In a specific embodiment, a construct or expression system is targeted to a specific location *(e.g.,* a standard position) within the organism *(e.g.,* fly, such as Drosophila) via directed integration (*e.g., attb*/*attp* directed integration on a chromosome *(e.g.,* a second chromosome). The integration of the construct/expression system into a standard position on a chromosome allows comparison of separate transgenic lines. Expression levels of transgenes and combinations can be confirmed using techniques known to one skilled in the art, e.g., by qPCR and/or fluorescence microscopy (*e.g., UAS-GFP).*

*Selection of Avatars and Production of Avatars.* Avatars are selected based on the activity of one or more tumor drivers or orthologs of the tumor drivers in the organism. Generating an avatar may comprise selecting one or more organisms based on their genomic, proteomic, and/or phenomic profile. An organism may be selected based on the similarity of its genomic, proteomic, and/or phenomic profile to the genomic, proteomic, and/or phenomic profile of the reference sample. An organism may be selected based on the similarity of its genomic, proteomic, and/or phenomic profile to the genomic, proteomic, and/or phenomic profile of a subject. An organism may be selected based on the similarity of its genomic, proteomic, and/or phenomic profile to the genomic, proteomic, and/or phenomic profile of a population of subjects. An organism may be selected based on the similarity of its genomic, proteomic, and/or phenomic profile to the genomic, proteomic, and/or phenomic profile that is characteristic of a disease.

Genetic engineering techniques known to one of skill in the art can be used to produce the transgenes and constructs described herein as well as fly avatar described herein. *See, e.g.,* Gratz et al., "Genome Engineering of Drosophila with the CRISPR RNA-Guided Cas9 Nuclease", Genetics, 2013, 194(4):1029-35, Venken et al., "Genome engineering: Drosophila melanogaster and beyond", Wiley Interdiscip Rev Dev Biol, 2015, Venken and Bellen, "Emerging technologies for gene manipulation in Drosophila melanogaster", Nat Rev Genet, 2005, 6(3):167-78, Venken and Bellen, "Transgenesis upgrades for Drosophila melanogaster", Development, 2007, 134(20):3571-84, Hales et al., "Genetics on the Fly: A Primer on the Drosophila Model System", Genetics, 2015, 201(3):815-42, Huang et al., "Directed, efficient, and versatile modifications of the Drosophila genome by genomic engineering", Proc Natl Acad Sci USA, 2009, 106(20):8284-9, Huang et al., "Targeted engineering of the Drosophila genome", Fly (Austin), 2009, 3(4):274-7, Lee et al., RNA-guided genome editing in Drosophila with the purified Cas9 protein", G3 (Bethesda), 2014, 4(7):1291-5, Liu et al., "Methods for TALENmediated genomic manipulations in Drosophila", Methods Mol Biol, 2016, 1338:179-90, and Dahmann, Drosophila: Methods and Protocols, 1st ed., 2008,

In some embodiments, generating an avatar may comprise conducting multiple crosses with multiple founder lines to produce an avatar whose genomic, proteomic and/or phenomic profile resembles the genomic, proteomic and/or phenomic profile of a disease, wherein the resemblance of the genomic, proteomic and/or phenomic profiles is based on the similarity of the expression or activity of one or more orthologs of disease drivers in the genomic, proteomic and/or phenomic profile of the avatar to the expression or activity of the disease drivers in the genomic, proteomic and/or phenomic profile of the disease. A founder line may comprise an organism comprising a transgene that alters the expression or activity of an ortholog of at least one disease driver. A founder line may comprise an organism comprising a transgene that alters the expression or activity of one or more orthologs of one or more disease drivers. A founder line may comprise an organism comprising a set of transgenes that alters the expression or activity of one or more orthologs of one or more disease drivers. In some embodiments, an avatar is generated from crossing two or more founder lines. *See, e.g.,* Sections 6, 7, and 8, *infra,* regarding crossing lines.

Generating an avatar may comprise: (a) transforming a first organism with a first construct or expression system to produce a first founder line; (b) transforming a second organism with a second construct or expression system to produce a second founder line; and (c) crossing the first organism with the second organism to produce a progeny line. The first organism and/or second organism may be transformed with a construct or expression system described herein. The avatar may be an organism of the progeny line produced by a cross between the first founder line and second founder line.

The avatar generated may be a personalized avatar or a generic avatar. *See, e.g.,* Sections 5.3, 6, 7, and 8, *infra,* for a discussion of personalized avatars and generic avatars.

*Generating an Avatar Army.* A method of generating an avatar army may comprise generating a clonal population of avatars. The method may comprise generating two or more avatars whose genomic, proteomic, and/or phenomic profile is based on a first subject. The one or more additional avatars may have a genomic profile, proteomic profile, and/or phenomic profile that is based on the genomic profile, proteomic profile, and/or phenomic profile of the first subject.

A method of generating an avatar army may comprise generating a polyclonal population of avatars. The method may comprise (a) generating a first avatar whose genomic, proteomic, and/or phenomic profile is based on a genomic, proteomic, and/or phenomic profile of a first subject; and (b) generating a second avatar whose genomic, proteomic, and/or phenomic profile is based on genomic, proteomic, and/or phenomic profile of a second subject.

The first avatar and second avatar may have a genomic profile, proteomic profile, and/or phenomic profile that is based on the genomic profile, proteomic profile, and/or phenomic profile of a first subject. The one or more additional avatars may have a genomic profile, proteomic profile, and/or phenomic profile that is based on the genomic profile, proteomic profile, and/or phenomic profile of the first subject. Alternatively, the one or more additional avatars may have a genomic profile, proteomic profile, and/or phenomic profile that is based on the genomic profile, proteomic profile, and/or phenomic profile of the second subject.

In one embodiment, a method of generating an avatar army comprises generating two or more avatars whose genomic, proteomic, and/or phenomic profile is based on a genomic, proteomic, and/or phenomic profile that is characteristic of cancer. The first avatar and second avatar may have a genomic profile, proteomic profile, and/or phenomic profile that is based on the genomic profile, proteomic profile, and/or phenomic profile of a first subject of a particular cancer. The one or more additional avatars may have a genomic profile, proteomic profile, and/or phenomic profile that is based on the genomic profile, proteomic profile, and/or phenomic profile of one or more additional subjects with the same type of cancer.

In another embodiment, a method of generating an avatar army may comprise generating two or more avatars whose genomic, proteomic, and/or phenomic profile is based on a genomic, proteomic, and/or phenomic profile of a population of subjects. The first avatar may have a genomic profile, proteomic profile, and/or phenomic profile that is based on the genomic profile, proteomic profile, and/or phenomic profile of a first subject. The second avatar may have a genomic profile, proteomic profile, and/or phenomic profile that is based on the genomic profile, proteomic profile, and/or phenomic profile of a second subject. The one or more additional avatars may have a genomic profile, proteomic profile, and/or phenomic profile that is based on the genomic profile, proteomic profile, and/or phenomic profile of one or more additional subjects.

The method of generating an avatar army may further comprise generating one or more additional avatars. The method may further comprise generating 3, 4, 5, 6, 7, 8, 9, 10, 11, 12, 13, 14, 15, 20, 30, 40, 50, 60, 70, 80, 90, 100, 200, 300, 400, 500, 600, 700, 800, 900 or 1000 or more avatars, or 5 to 10, 25 to 50, 50 to 100, 100 to 200, 200 to 500, or 500 to 1000 avatars.

Generating an avatar of the avatar army may comprise any of the steps or methods described in this Section 5.2.1. Generating an avatar of the avatar army may comprise the use of any of the constructs or expressions systems described in this Section 5.2.1.

### 5.2.2. Kits Used to Generate Avatars

Provided herein kits comprising one or more transgenes described herein. The transgene may comprise a cDNA of the ortholog of a disease driver (*e.g*., tumor driver), a cDNA of an activator of the ortholog of a disease driver, a cDNA of an inhibitor of an ortholog of a disease driver, an interfering RNA *(e.g.,* small interfering RNA (siRNA) or short hairpin RNA (shRNA)) that targets the ortholog of the disease driver (*e.g*., tumor driver), or a combination thereof. In certain disclosures, the transgene comprises 1, 2, 3, 4, 5, 6, 7, 8, 9, 10 or more interfering RNAs (*e.g*., small interfering RNAs (siRNAs) or short hairpin RNAs (shRNAs)) that target 1, 2, 3, 4, 5, 6, 7, 8, 9, 10 or more orthologs of tumor suppressors. In some disclosures, the transgene comprises 1, 2, 3, 4, 5, 6, 7, 8, 9, 10 or more cDNAs of the orthologs of oncogenes. In certain disclosures, the transgene comprises 1, 2, 3, 4, 5, 6, 7, 8, 9, 10 or more interfering RNAs *(e.g.,* small interfering RNAs (siRNAs) or short hairpin RNAs (shRNAs)) that target 1, 2, 3, 4, 5, 6, 7, 8, 9, 10 or more orthologs of tumor suppressors, and 1, 2, 3, 4, 5, 6, 7, 8, 9, 10 or more cDNAs of the orthologs of oncogenes. The kits may also include plasmids or other constructs that can be used to express the transgene(s). The kits comprising transgenes may be stored at the appropriate temperature and may include instructions for use of the transgenes. For example, the instructions may include instructions for engineering plasmids or other constructs to contain the transgene(s) and techniques for engineering fly avatars *(e.g., Drosophila* avatars) to express the transgene(s).

Provided herein are kits comprising constructs or expression systems for altering the expression level or activity of an ortholog of a tumor driver in an organism (*e.g*., fly). In a specific disclosures, provided herein are kits comprising a construct or expression system described herein. *See, e.g.,* Section 5.2.1, *supra,* and Sections 6, 7, and 8, *infra,* for constructs and expression systems that may be included in a kit. In a specific disclosure, the kits comprise a construct comprising a transgene regulated by a promoter (e.g., an inducible promoter and/or tissue, cell-specific promoter). For example, the transgene may be regulated by the *UAS* promoter. In certain disclosures, the kits may also include a construct comprising a transcriptional activator (*e.g*., GAL4) regulated by a temperature sensitive promoter. In one example, a kit comprises two constructs, one construct comprising a transgene linked to a promoter (*e.g., UAS*) regulated by a transcriptional activator *(e.g.,* GAL4) and a second construct comprising the transcriptional activator linked to a temperature sensitive promoter. In another example, a kit comprises two constructs, one construct comprising a transgene linked to a promoter regulated by a transcriptional repressor and a second construct comprising the repressor linked to a temperature sensitive promoter. The kits comprising one or more constructs or expression systems may be stored at the appropriate temperature and may include instructions for use of the constructs or expression systems. For example, the instructions may include information regarding how to transform cell (*e.g*., embryonic cells) with the constructs or expression system, how to induce the expression of a transgene(s) and/or how to conduct a screen for drugs effective to treat a particular type of cancer.

Also provided herein are kits comprising embryonic cells transformed with one or more of the constructs or expression systems described herein. *See, e.g.,* Section 5.2.1, *supra,* and Sections 6, 7, and 8, *infra,* for constructs and expression systems that may be included in a kit. In a specific disclosure, the embryonic cells are frozen and may be stored appropriately until use. Techniques known to one of skill in the art may be used to freeze the embryonic cells, store the frozen cells and thaw them for use.

Further provided herein are kits comprising embryos transformed with one or more of the constructs or expression systems described herein. *See, e.g.,* Section 5.2.1, *supra,* and Sections 6, 7, and 8, *infra,* for constructs and expression systems that may be included in a kit. In a specific disclosure, the embryos are frozen and may be stored appropriately until use. Techniques known to one of skill in the art may be used to freeze the embryos, store the frozen embryos and thaw them for use. For example, the instructions may include information regarding how to induce the expression of a transgene(s) and/or how to conduct a screen for drugs effective to treat a particular type of cancer.

### 5.3. TREATMENT OF FLY AVATARS WITH DRUG REGIMENS TO SELECT OR CONFIRM EFFICACY

The avatars *(e.g.,* fly avatars, such as *Drosophila)* are useful in high-throughput screens to identify drugs that are beneficial for the treatment of cancer. As discussed herein (*see, e.g.,* Section 5.2.1, *supra,* and Sections 6, 7, and 8, *infra*)*,* the avatars *(e.g.,* fly avatars, such as *Drosophila)* are engineered to express a transgene(s) in a vital organ of the avatar when induced and the induction of the transgene(s) is lethal to development of the avatar. In one example, the screenable phenotype would be rescue of lethality caused by expression of the shRNAs and the oncogene orthologs; lethality is calibrated to a 'barely dead' phenotype by testing a variety of temperatures with the intent of altering GAL4 activity to the lowest required to direct complete organismal lethality. As used herein, the term "barely dead phenotype" means at least 80%, 85%, 86%, 87%, 88%, 89%, 90%, 91%, 92%, 93%, 94%, 95%, 96%, 97%, 98%, 99%, or 100% lethality at a certain stage of development of the organism. In the case of fly avatars, the barely dead phenotype" means at least 80%, 85%, 86%, 87%, 88%, 89%, 90%, 91%, 92%, 93%, 94%, 95%, 96%, 97%, 98%, 99%, or 100% lethality at pupariation and at least 98%, 99%, or 100% lethality at adulthood of the fly avatar. In some embodiments, in the case of fly avatars, the barely dead phenotype" means at least 80%, 85%, 86%, 87%, 88%, 89%, 90%, 91%, 92%, 93%, 94%, 95%, 96%, 97%, 98%, 99%, or 100% lethality at pupation and at least 98%, 99%, or 100% lethality at adulthood of the fly avatar.

Accordingly, in a specific embodiment, the screenable phenotype of lethality when the transgene is expressed is calibrated prior to conducting a screen to identify drugs beneficial for the treatment of cancer. This may involve selecting the appropriate temperature of the culture for inducing expression of the transgene(s) such that at least 80%, 85%, 86%, 87%, 88%, 89%, 90%, 91%, 92%, 93%, 94%, 95%, 96%, 97%, 98% or 99% lethality results at a certain stage of development of the avatar. In a specific embodiment, calibration involves selecting the appropriate temperature of the culture for inducing expression of the transgene(s) by the fly avatar *(e.g., Drosophila* avatar) such that at least 80%, 85%, 86%, 87%, 88%, 89%, 90%, 91%, 92%, 93%, 94%, 95%, 96%, 97%, 98% or 99% lethality at pupariation results and at least 98%, 99%, or 100% lethality at adulthood results. In certain specific embodiments, calibration involves selecting the appropriate temperature of the culture for inducing expression of the transgene(s) by the fly avatar *(e.g., Drosophila* avatar) such that at least 80%, 85%, 86%, 87%, 88%, 89%, 90%, 91%, 92%, 93%, 94%, 95%, 96%, 97%, 98% or 99% lethality at pupation results and at least 98%, 99%, or 100% lethality at adulthood results.

Once the avatar is calibrated to the screenable phenotype of lethality, the avatar may be used to screen for drugs that increase the viability of the avatar (*e.g*., fly avatar, such as *Drosophila)* to a certain stage of development, *e.g.,* pupariation and/or adulthood for the fly avatar. In certain embodiments, the certain stage of development is pupation. The screening assay may involve (a) inducing expression of the construct or expression system in an avatar(s) in the presence or absence of one or more candidate drugs or a combination of candidate drugs; and (b) determining the phenotype of the avatar(s) in the presence of the one or more candidate drugs. The avatar *(e.g.,* fly avatar, such as *Drosophila)* may be exposed to the candidate drug(s) or combination of candidate drugs by the addition of the candidate drug(s) or combination of candidate drugs to the food or water that is given to the avatar. Alternatively, the avatar (*e.g*., fly avatar, such as *Drosophila)* may be exposed to the candidate drug or combination of candidate drugs by injection, such as intravenous injection or peritoneal injection. In a specific embodiment, the amount of drug each avatar is exposed to is not lethal to the non-modified counterpart of the avatar. For example, the amount of drug a fly avatar *(e.g., Drosophila* avatar) is exposed to is not lethal to a non-modified fly *(e.g., Drosophila).* In specific embodiments, multiple doses of the same drug or a combination of drugs may be tested in an avatar described herein. Certain doses of the drug or a combination of drugs may be more effective in an avatar described herein than others. The screening assay may also include exposing an avatar(s) with a control composition, such a negative control (*e.g*., phosphate buffered saline). For example, the screening assay may comprise placing media comprising food and a control composition (*e.g*., phosphate buffered saline) into a container or well of the plate with an avatar(s).

The avatar *(e.g.,* fly avatar, such as Drosophila avatar) may be exposed to the candidate drugs in combinations. An avatar (*e.g*., fly avatar, such as Drosophila avatar) may be exposed to two or more candidate drugs at the same time, or sequentially. For example, the avatar *(e.g.,* fly avatar, such as Drosophila avatar) may be exposed to a first candidate drug and a second candidate drug at a first time point and exposed to a third candidate drug at a second time point. The avatar (*e.g*., fly avatar, such as Drosophila avatar) may be exposed to two or more candidate drugs at two or more overlapping time periods. For example, the avatar (*e.g*., fly avatar, such as Drosophila avatar) may be exposed to a first candidate drug for a first time period and exposed to a second candidate drug for a second period, wherein the first and second time periods at least partially overlap. The avatar (*e.g*., fly avatar, such as Drosophila avatar) may be exposed to two or more candidate drugs at two or more non-overlapping time periods. For example, the avatar *(e.g.,* fly avatar, such as Drosophila avatar) may be exposed to a first candidate drug for a first time period and exposed to a second candidate drug for a second period, wherein the first and second time periods do not overlap. As discussed herein, the exposure of the avatar(s) *(e.g.,* fly avatar, such as *Drosophila* avatar) to the combination of candidate drugs may be done by placing the drug in food.

In a specific embodiment, the screening assays for drugs for treating cancer in fly avatars *(e.g., Drosophila* avatars) is performed in individual tubes or wells of plate *(e.g.,* a 96 well plate). The intent is to place food, a candidate drug or combination of candidate drugs, and avatars into each tube or well. Food *(e.g.,* fly, such as Drosophila, media) is placed into each tube or well; this may be done by hand or by automated sorting, *e.g., via* a liquid handler. Each candidate drug(s) may be added into duplicate tubes or wells at a chosen concentration that is not lethal to non-modified organism *(e.g.,* fly, such as *Drosophila).* For example, the final food concentration of each candidate drug may be 25 µM, 30 µM, 35 µM, 40 µM, 45 µM, 50 µM, 55 µM, 60 µM, 65 µM, 70 µM, 75 µM, 80 µM, 85 µM, 90 µM, 95 µM, 100 µM, 110 µM, 125 µM, 150 µM, 175 µM, or 200 µM. A candidate drug(s) is mixed into the food and may be allowed to further diffuse for a period of time *(e.g.,* 6-12 hours, 8-12 hours, 12-18 hours, 12-24 hours, 16-24 hours, 18 to 24 hours, 24 to 36 hours, or 12 to 36 hours). A designated number of transgenic embryos *(e.g.,* fly embryos, such as *Drosophila* embryos) are placed into each tube or well on top of the solidified food/drug mixture. For example, each tube or well may contain 3, 4, 5, 6, 7, 8, 9, 10, 11, 12, 13, 14, 15 or more embryos. Each tube or plate may then be covered with a breathable substance and animals develop at the optimized temperature.

A candidate drug may be determined to be effective if the presence of the candidate drug at least partly rescues the lethality caused by expression of the construct or expression system. For example, a candidate drug may be determined to be effective if it rescues at least 0.5%, 1%, 5%, 10%, 15%, 20%, 25% 30%, 35%, 40%, 45%, 50%, 55%, 60%, 65%, 70%, 75%, 80%, 85%, 90%, 95% or more of fly avatars to adulthood. A candidate drug may be determined to be effective if the presence of the candidate drug reduces the degree of lethality caused by expression of the construct or expression system. For example, a candidate drug may be determined to be effective if it rescues at least 0.5%, 1%, 5%, 10%, 15%, 20%, 25% 30%, 35%, 40%, 45%, 50%, 55%, 60%, 65%, 70%, 75%, 80%, 85%, 90%, 95% or more of fly avatars to pupariation. In another example, a candidate drug may be determined to be effective if it rescues at least 0.5%, 1%, 5%, 10%, 15%, 20%, 25% 30%, 35%, 40%, 45%, 50%, 55%, 60%, 65%, 70%, 75%, 80%, 85%, 90%, 95% or more of fly avatars to pupation. A candidate drug may be determined to be effective if the presence of the candidate drug reduces the severity of a phenotype caused by expression of the construct or expression system.

The candidate drug may be determined to be effective if the candidate drug causes the proteomic and/or phenomic profile of the avatar to more closely resemble the proteomic and/or phenomic profile of a healthy subject as compared to the proteomic and/or phenomic profile of the avatar in the absence of the candidate drug.

A combination of candidate drugs may be determined to be effective if the presence of the combination of candidate drugs rescues the lethality caused by expression of the construct or expression system. For example, the combination of candidate drugs may be determined to be effective if it rescues at least 0.5%, 1%, 5%, 10%, 15%, 20%, 25% 30%, 35%, 40%, 45%, 50%, 55%, 60%, 65%, 70%, 75%, 80%, 85%, 90%, 95% or more of fly avatars to adulthood. A combination of candidate drugs may be determined to be effective if the presence of the combination of candidate drugs reduces the degree of lethality caused by expression of the construct or expression system. For example, a combination of candidate drugs may be determined to be effective if it rescues at least 0.5%, 1%, 5%, 10%, 15%, 20%, 25% 30%, 35%, 40%, 45%, 50%, 55%, 60%, 65%, 70%, 75%, 80%, 85%, 90%, 95% or more of fly avatars to pupariation. In another example, a combination of candidate drugs may be determined to be effective if it rescues at least 0.5%, 1%, 5%, 10%, 15%, 20%, 25% 30%, 35%, 40%, 45%, 50%, 55%, 60%, 65%, 70%, 75%, 80%, 85%, 90%, 95% or more of fly avatars to pupation. A combination of candidate drugs may be determined to be effective if the combination of candidate drugs causes a greater reduction in the degree of lethality in the avatar as compared to the reduction in the degree of lethality in the avatar caused by the separate candidate drugs.

Alternatively or in addition, the combination of candidate drugs may be determined to be effective if the combination of candidate drugs causes the proteomic and/or phenomic profile of the avatar to more closely resemble the proteomic and/or phenomic profile of a healthy subject as compared to the effect of the separate candidate drugs on the proteomic and/or phenomic profile of the avatar. For instance, a combination of candidate drugs comprising drug A, drug B, and drug C may be determined to be effective if the combination of candidate drugs causes the proteomic and/or phenomic profile of the avatar *(e.g.,* fly avatar, such as Drosophila avatar) to more closely resemble the proteomic and/or phenomic profile of a non-modified organism (*e.g*., fly) as compared to the separate effect of drug A, drug B, and drug C on the proteomic and/or phenomic profile of the avatar *(e.g.,* fly avatar, such as *Drosophila* avatar). The combination of candidate drugs may be determined to be effective if the synergistic effect of the combination of candidate drugs on the proteomic and/or phenomic profile of the avatar (*e.g*., fly avatar, such as Drosophila avatar) is greater than the effect of the separate candidate drugs on the proteomic and/or phenomic profile of the avatar *(e.g.,* fly avatar, such as Drosophila avatar).

In a specific embodiment, a method for screening/selecting a therapeutically effective drug or combination drugs for treating a subject diagnosed with cancer, wherein cancer cells from the subject exhibit increased activity of one or more oncogenes and/or reduced activity of one or more tumor suppressors, comprises: screening a library of drugs for those drugs or combination of drugs that when fed to a culture of a *Drosophila* larva avatar, allow the *Drosophila* larva avatar to survive to pupation, such that upon induction through an external factor there is an increase in the activity of an ortholog(s) of the subject's oncogene(s) and/or inhibition an ortholog(s) of the subject's tumor suppressor(s) in a larval tissue that is necessary for survival to pupation, which increase in activity and/or inhibition prevents an untreated *Drosophila* larva avatar from surviving to pupation. In certain embodiments, the combination of drugs allows 0.5%, 1%, 5%, 10%, 15%, 20%, 25% 30%, 35%, 40%, 45%, 50%, 55%, 60%, 65%, 70%, 75%, 80%, 85%, 90%, 95% or more of *Drosophila* larva avatar to survive to pupation. In certain embodiments, the combination of drugs allows at least 0.5%, 1%, 5%, 10%, 15%, 20%, 25% 30%, 35%, 40%, 45%, 50%, 55%, 60%, 65%, 70%, 75%, 80%, 85%, 90%, 95% or more of *Drosophila* larva avatar to survive to pupation. In certain embodiments, the combination of drugs allows between 0.5% and 5%, between 5% and 15%, between 15% and 25%, between 25% and 35%, between 35% and 50%, between 50% and 70%, between 70% and 90%, or between 80% and 98% of *Drosophila* larva avatar to survive to pupation.

In a specific embodiment, a method for screening/selecting a therapeutically effective drug or combination drugs for treating a subject diagnosed with cancer, wherein cancer cells from the subject exhibit increased activity of one or more oncogenes and/or reduced activity of one or more tumor suppressors, comprises: screening a library of drugs for those drugs or combination of drugs that when fed to a culture of a *Drosophila* larva avatar, allow the *Drosophila* larva avatar to survive to pupariation, such that upon induction through an external factor there is an increase in the activity of an ortholog(s) of the subject's oncogene(s) and/or inhibition an ortholog(s) of the subject's tumor suppressor(s) in a larval tissue that is necessary for survival to pupariation, which increase in activity and/or inhibition prevents an untreated *Drosophila* larva avatar from surviving to pupariation. In certain embodiments, the combination of drugs allows 0.5%, 1%, 5%, 10%, 15%, 20%, 25% 30%, 35%, 40%, 45%, 50%, 55%, 60%, 65%, 70%, 75%, 80%, 85%, 90%, 95% or more of *Drosophila* larva avatar to survive to pupariation. In certain embodiments, the combination of drugs allows at least 0.5%, 1%, 5%, 10%, 15%, 20%, 25% 30%, 35%, 40%, 45%, 50%, 55%, 60%, 65%, 70%, 75%, 80%, 85%, 90%, 95% or more of *Drosophila* larva avatar to survive to pupariation. In certain embodiments, the combination of drugs allows between 0.5% and 5%, between 5% and 15%, between 15% and 25%, between 25% and 35%, between 35% and 50%, between 50% and 70%, between 70% and 90%, or between 80% and 95% of *Drosophila* larva avatar to survive to pupariation.

The avatars (*e.g*., fly avatar) used in a screening assay described herein may be a personalized avatar, a generic avatar, or an avatar(s) that is part of an avatar army. *See, e.g.,* Section 5.3.1, *infra,* for discussion of such avatars. The personalized avatar can be used to screen for candidate drugs that may be effective to treat a particular subject; to provide a recommendation for treating a particular subject; or to treat a particular subject. Generic avatars and/or avatar armies can be used in screening assays to identify one or more candidate drugs that may be effective for the treatment of a particular type cancer in a population of subjects; to provide a recommendation for treating a particular type cancer in a population of subjects; or to treat a population of subjects suffering from a particular type cancer.

Any of the avatars can be used to screen for new chemical entities for the treatment of cancer, however the generic avatars and avatar armies may be particularly useful for identifying new therapeutics for larger populations of patients that share variations in disease drivers.

In a specific embodiment, a fly avatar *(e.g.,* a *Drosophila* avatar) is used in a screening assay described herein. In a particular, embodiment, the embryo or larva of the avatar is exposed to a drug or a combination of drugs in a screening assay described herein. The larva of flies *(e.g.,* a *Drosophila)* are known to eat a lot of food which facilitates a screening assay described.

### 5.3.1. Disease Avatars: Personalized, Generic and Armies

*Personalized Avatars.* A personalized avatar recapitulates a patient's genome, proteome and/or phenome and can be used to select a therapeutic regimen most tailored for the patient; to identify one or more drug regimens that may be effective for the treatment of the patient, or to provide a recommendation for treatment. For example, the disease drivers of a particular cancer subject may be identified to comprise a mutation in an epidermal growth factor receptor (EGFR) gene and a mutation in a tumor protein p53 (p53) gene. An avatar *(e.g.,* a fly avatar, such as a *Drosophila* avatar) may be engineered as described herein (*see, e.g.,* Section *5.2, supra,* and Sections 6, 7, and 8, *infra*) to contain a cDNA representing the ortholog of EGFR and an interfering RNA of the ortholog of p53. Once the avatar is induced to express the cDNA and interfering RNA, overexpression of the EGFR ortholog and reduction in the expression of the p53 ortholog result.

*Generic Avatars.* Generic avatars can be used as a starting point to engineer a personalized avatar, or for the generation of avatar armies. A generic avatar may comprise a cDNA(s) representing of an ortholog(s) of a disease driver(s) (*e.g.,* a tumor driver(s), such as an oncogene(s)) and/or an interfering RNA(s) that targets an ortholog of a disease driver(s) (e.g., a tumor driver, such as a tumor suppressor). In some embodiments, the disease driver(s) (e.g., tumor driver(s), such as an oncogenes(s) or tumor suprressor(s)) commonly have altered expression or activity in a population of subjects suffering from a disease (e.g., cancer). For example, the disease driver(s) of colorectal cancer may be characterized as mutations in certain oncogenes and/or tumor suppressors, such as adenomatous polyposis coli (APC), epidermal growth factor (EGFR), tumor protein p53 (p53), SMAD family member 2 (SMAD2), SMAD3, SMAD4, phosphatidylinositol-4,5-bisphosphate 3-kinase, catalytic subunit alpha (PIK3CA), and phosphatase and tensin homolog (PTEN). A generic avatar may comprise a cDNA representing one or more of the following oncogenes and/or an interfering RNA targeting one or more of the following tumor suppressors: adenomatous polyposis coli (APC), epidermal growth factor (EGFR), tumor protein p53 (p53), SMAD family member 2 (SMAD2), SMAD3, SMAD4, phosphatidylinositol-4,5-bisphosphate 3-kinase, catalytic subunit alpha (PIK3CA), and/or phosphatase and tensin homolog (PTEN). One or more generic avatars can be crossed with each other to produce a personalized avatar or avatar army. *See, e.g.,* Section 5.2.1, *supra,* and Sections 6, 7, and 8, *infra,* for a discussion regarding generic avatars.

*Avatar Armies.* An avatar army may comprise 1, 2, 3, 4, 5, 6, 7, 8, 9, 10, 15, 20, 25, 30, 35, 40, 45, 50, 60, 70, 80, 90, 100, 150, 200, 250, 300, 350, 400, 450, 500, 550, 600, 650, 700, 750, 800, 850, 900, 950, or 1000 or more personalized avatars. An avatar army may comprise avatars that recapitulate the phenome from multiple individual patients. An avatar army may also comprise avatars that reflect the phenome of patients reported in public databases. An avatar army may comprise avatars representing the phenomes of patients for a particular type of cancer. *See, e.g.,* Sections 6, 7, and 8, *infra,* regarding a colorectal avatar army. In a specific embodiment, an avatar from an avatar army is selected that most closely recapitulates a patient's phenome.

### 5.3.2. Candidate Drugs For Use in Screens and Treatment Regimens

Candidate drugs for use in a screening assay described herein include known drugs (e.g., known cancer drugs) and/or new chemical entities. The screening assay described herein can be used to identify known drugs (e.g., known cancer drugs) for the treatment of a particular type of cancer and/or combinations of known drugs (e.g., known cancer drugs) for the treatment of cancer. The screening assay described herein can be used to identify new chemical entities for the treatment of cancer and/or combinations of new chemical entities, or combinations of new chemical entities and known drugs (e.g., known cancer drugs) for the treatment of cancer. In addition, the drugs identified or confirmed in a screening assay described herein and/or used in the treatment of cancer can include known drugs (e.g., known cancer drugs) and/or new chemical entities.

Cancer drugs, include but are not limited to:
***Alkylating Agents:*** Bifunctional Alkylators including but not limited to Cyclophosphamide, Mechlorethamine, Chlorambucil and Melphalan; and Monofunctional Alkylators including but not limited to Nitrosoureas and Temozolomide.
***Anthracyclines:*** including but not limited to Daunorubicin, Doxorubicin, Epirubicin, Idarubicin, Mitoxantrone, Varubicin.
***Cytoskeletal Disruptors:*** Taxanes, including but not limited to Paclitaxol and Docetaxol; as well as Epothilones
***Histone Deacetylase Inhibitors*:** including but not limited to Vorinostat and Romidepsin.
***Inhibitors of Topoisomerase I*:** including but not limited to Irenotecan and Topotecan.
***Inhibitors of Topoisomerase II*:** including but not limited to Etoposide, Teniposide and Tafluposide.
***Kinase Inhibitors*:** including but not limited to Bortezomib, Erlotinib, Gefitinib, Imatinib, Vemurafenib, and Vismodegib.
***Nucleotide Analogs and precursor analogs*:** including but not limited to Azacitidine, Azathioprine, Capecitabine, Cytarabine, Doxifluridine, Fluorouracil, Gemcitabine, Hydroxyurea, Mercaptopurine, Methotrexate, and Thioguanine (aka Tioguanine).
***Peptide Antibiotics*:** including but not limited to Bleomycin, and Actinomycin.
***Platinum-based Agents*:** including but not limited to Carboplatin, Cisplatin, and Oxaliplatin.
***Retinoids*:** including but not limited to Tretinoin, Alitretinin, and Bexarotene.
***Vinca Alkaloids and derivatives:*** including but not limited to Vinblastine, Vincristine, Vindesine, and Vinorelbine.
*Chemical Libraries*: Chemical libraries containing new and/or known drugs can be used in a screening assay described herein. In addition, a drug or a combination of drugs from a chemical library can be identified or confirmed to treat cancer and/or used to treat cancer. Chemical libraries containing the scaffolds for known drugs that can be used in accordance with the invention include, but are not limited, to the SelleckChem library of FDA-approved drugs, DTP approved oncology drug sets, and Enzo's SCREEN-WELL^{®} FDA approved drug library V2. In one embodiment, one or more of the chemical libraries listed in Table 1, *infra,* can used in accordance with the invention.

**Table 1: Chemical Libraries**

| Library **Name** | **Provider** |
|---|---|
| Anti-Cancer Compound Library | MedChem Express |
| Clinical Compound Library | MedChem Express |
| Kinase Inhibitor Library | MedChem Express |
| Protein Tyrosine Kinase Compound Library | MedChem Express |
| PI3K/Akt/mTOR Compound Library | MedChem Express |
| MAPK Compound Library | MedChem Express |
| Akt-Targeted Library | ChemDiv |
| PI3K-Targeted Library | ChemDiv |
| KRAS-Targeted Library | ChemDiv |
| EXPRESS-Pick Collection | ChemBridge |
| DIVERSet^{™}-EXP | ChemBridge |
| CORE Library | ChemBridge |
| DIVERSet^{™}-CL | ChemBridge |
| Bioactive Compound Library | SelleckChem |
| Inhibitor Library | SelleckChem |
| Kinase Inhibitor Library | SelleckChem |
| Anti-Cancer Compound Library | SelleckChem |
| Tyrosine Kinase Inhibitor Library | SelleckChem |
| Cambridge Cancer Compound Library | SelleckChem |
| Ion Channel Ligand Library | SelleckChem |
| Pfizer Licensed Compound Library | SelleckChem |
| Target Selective Inhibitor Library | SelleckChem |
| PI3K/Akt Inhibitor Library | SelleckChem |
| MAPK Inhibitor Library | SelleckChem |

| Apoptosis Compound Library | SelleckChem |
|---|---|
| NCI Diversity Set IV | DTP (NCI) |
| Mechanistic Set III | DTP (NCI) |
| Natural Products Set III | DTP (NCI) |
| LOPAC^{®1280} | Sigma Aldrich |
| US Drug Collection | MicroSource Discovery System |
| International Drug Collection | MicroSource Discovery System |
| Pharmakon 1600 | MicroSource Discovery System |
| Anti-Cancer Compound Library | Stratech |
| Tocriscreen Total or Tocriscreen Mini | Tocris |
| Tocriscreen Mini | Tocris |

In certain embodiments, the chemical library comprises a drug described herein. *See*, *e.g.,* Sections 6, 7, and 8, *infra,* for examples of drugs. In certain embodiments, the chemical library does not comprise one or more of the combinations listed in Table 2.

In some embodiments, the chemical library comprises vandetanib, sunitinib, sorafenib, AD57 (the DFG-out Type II compound), AD58, AD80, caprelsa, or the Ret-targeting inhibitor DL06. In other embodiments, the chemical library does not comprise the drug vandetanib, sunitinib, sorafenib, AD57 (the DFG-out Type II compound), AD58, AD80, caprelsa, or the Ret-targeting inhibitor DL06.

### 5.3.3. Identifying a Therapeutic Regimen

Therapeutic regimens are identified by analyzing results from the screening assays to determine which candidate drugs or combination of drugs rescued the lethality phenotype or reduced the degree of the lethality phenotype in the avatar or avatar army. Identifying one or more therapeutic regimens may comprise identifying the candidate drugs that had a synergistic effect in rescuing the lethality phenotype or reducing the degree of the lethality phenotype in the avatar for use in a therapeutic regimen for treating a subject.

The candidate drugs or combination of drugs may be selected based on their ability to rescue the lethality phenotype or reduce the degree of the lethality phenotype in the avatar or avatar army. Identifying one or more therapeutic regimens may comprise selecting the candidate drugs or combination of drugs that were capable of rescuing the lethality phenotype or reducing the degree of the lethality phenotype in the avatar or avatar army. Identifying one or more therapeutic regimens may comprise selecting the candidate drugs that had a synergistic effect in rescuing the lethality phenotype or reducing the degree of the lethality phenotype in the avatar.

Reducing the degree of the lethality phenotype in the avatar may comprise extending the life span of the avatar in culture. Reducing the degree of the lethality phenotype in the avatar army may comprise extending the average life span of the avatar army. Reducing the degree of the lethality phenotype in the avatar army may comprise increasing the survival rate of the avatar army. Reducing the degree of the lethality phenotype in the avatar army may comprise increasing the number of avatars in the avatar army that develop into pupa and/or adulthood. For example, reducing the degree of the lethality phenotype in the avatar army may comprise increasing the number of avatars in the avatar army that develop into pupation. For example, reducing the degree of lethality might comprise increasing the number of avatars that survive to pupation to 0.5%, 1%, 5%, 10%, 15%, 20%, 25%, 30%, 35%, 40%, 45%, 50%, 55%, 60%, 65%, 70%, 75%, 80%, 85%, 90%, 95%, or 98%. In another example, reducing the degree of lethality might comprise increasing the number of avatars that survive to pupation to at least 0.5%, 1%, 5%, 10%, 15%, 20%, 25%, 30%, 35%, 40%, 45%, 50%, 55%, 60%, 65%, 70%, 75%, 80%, 85%, 90%, 95%, or 98%. In another example, reducing the degree of lethality might comprise increasing the number of avatars that survive to pupation to between 0.5% and 5%, between 5% and 15%, between 15% and 25%, between 25% and 35%, between 35% and 50%, between 50% and 70%, between 70% and 90%, or between 80% and 98%. In another example, reducing the degree of the lethality phenotype in the avatar army may comprise increasing the number of avatars in the avatar army that develop into pupariation. For example, reducing the degree of lethality might comprise increasing the number of avatars that survive to pupariation to 0.5%, 1%, 5%, 10%, 15%, 20%, 25%, 30%, 35%, 40%, 45%, 50%, 55%, 60%, 65%, 70%, 75%, 80%, 85%, 90%, 95%, or 98%. In another example, reducing the degree of lethality might comprise increasing the number of avatars that survive to pupariation to at least 0.5%, 1%, 5%, 10%, 15%, 20%, 25%, 30%, 35%, 40%, 45%, 50%, 55%, 60%, 65%, 70%, 75%, 80%, 85%, 90%, 95%, or 98%. In another example, reducing the degree of lethality might comprise increasing the number of avatars that survive to pupariation to between 0.5% and 5%, between 5% and 15%, between 15% and 25%, between 25% and 35%, between 35% and 50%, between 50% and 70%, between 70% and 90%, or between 80% and 98%. In yet another example, reducing the degree of lethality might comprise increasing the number of avatars that survive to adulthood to 0.5%, 1%, 5%, 10%, 15%, 20%, 25%, 30%, 35%, 40%, 45%, 50%, 55%, 60%, 65%, 70%, 75%, 80%, 85%, 90%, 95%, or 98%. In another example, reducing the degree of lethality might comprise increasing the number of avatars that survive to adulthood to at least 0.5%, 1%, 5%, 10%, 15%, 20%, 25%, 30%, 35%, 40%, 45%, 50%, 55%, 60%, 65%, 70%, 75%, 80%, 85%, 90%, 95%, or 98%. In another example, reducing the degree of lethality might comprise increasing the number of avatars that survive to adulthood to between 0.5% and 5%, between 5% and 15%, between 15% and 25%, between 25% and 35%, between 35% and 50%, between 50% and 70%, between 70% and 90%, or between 80% and 98%.

Identifying one or more therapeutic regimens may include analyzing the one or more results from one or more screening assays to determine which candidate drugs or combination of drugs caused the proteomic and/or phenomic profile of the avatar or avatar army to more closely resemble the proteomic and/or phenomic profile of a healthy subject or a subject who is responding to therapy. Exposure of the avatar or avatar army to the candidate drug or combination of drugs may alter the expression level or activity of one or more genes in the avatar. Identifying one or more therapeutic regimens may comprise identifying the candidate drugs or combination of drugs that reduced the expression level or activity of a disease driver *(e.g.,* tumor driver, such as an oncogenes) in the avatar. Identifying one or more therapeutic regimens may comprise identifying the candidate drugs or combination of drugs that increased the expression level or activity of a disease driver (*e.g.*, tumor driver, such as a tumor suppressor) in the avatar. Identifying one or more therapeutic regimens may comprise identifying the candidate drugs or combination of drugs that reduced the expression level or activity of a disease driver (*e.g.*, tumor driver, such as an oncogenes) in the avatar and increased the expression level or activity of a disease driver (*e.g.*, tumor driver, such as a tumor suppressor) in the avatar.

Identifying one or more therapeutic regimens may comprise generating a report comprising a list of therapeutic regimens that can be used to treat a subject suffering from cancer (*e.g.,* a particular type of cancer) or population of subjects suffering from cancer (*e.g.,* a particular type of cancer). Identifying one or more therapeutic regimens may comprise transmitting a report comprising a list of therapeutic regimens that can be used to treat a subject suffering from cancer (*e.g.,* a particular type of cancer) or population of subjects suffering from cancer (*e.g.,* a particular type of cancer). Identifying one or more therapeutic regimens may comprise obtaining a report comprising a list of therapeutic regimens that can be used to treat a subject suffering from cancer (*e.g.,* a particular type of cancer) or population of subjects suffering from cancer (*e.g.,* a particular type of cancer). Identifying one or more therapeutic regimens may comprise receiving a report comprising a list of therapeutic regimens that can be used to treat a subject suffering from cancer (*e.g.,* a particular type of cancer) or population of subjects suffering from cancer (*e.g.,* a particular type of cancer). Generating a report may comprise generating a list of therapeutic regimens. Generating a report may comprise ranking a list of therapeutic regimens. The therapeutic regimens may be listed in order of efficacy, with the most efficacious regimens at the top of the list and the less efficacious regimens at the bottom of the list. The efficacy of the therapeutic regimen may be based on the rescue of the lethality phenotype or the degree of reduction of the lethality phenotype in the avatar, possible side effects associated with a candidate drug or combination of drugs in the therapeutic regimen, pharmacokinetic properties of the candidate drug or combination of drugs, toxicity of the candidate drug or combination of drugs, dosage and/or dosing frequency of the candidate drug or combination of drugs, and route of administration of the candidate drug or combination of drugs. For instance, candidate drugs A and B may both rescue the lethality phenotype in the avatar, but the side effects associated with candidate drug A may be more severe than the side effects associated with candidate drug B. Thus, candidate drug B may be listed before candidate drug A in the report. The ranking of the therapeutic regimens may also be based subject-specific information. For instance, the ranking of the therapeutic regimens may also be based on the subject's medical history, such as prior therapies, current medications, comorbidities, current health, predisposition to diseases/conditions, and surgeries, familial history, age, gender, and occupation. Generating a report may comprise a ranking method as described in Buti et al., 2013, J Clin Periodontol 40:372-86 and Sun et al., 2015, Nat Commun 6:8481, each of which is incorporated by reference in its entirety.

### 5.4. TREATMENT OF THE SUBJECT

*Report with results*. In one aspect, the results from an avatar screening assay are provided to a subject with cancer and/or his/her healthcare provider in the form of a report which includes those drugs and/or combination of drugs tested in the avatar screening assay and the results from those tests. *See, e.g.,* Section 5.3.3, *supra,* for the types of information that may be included in such a report.

*Report with treatment recommendation*. In another aspect, the results from an avatar screening assay is provided to a subject with cancer and/or his/her healthcare provider in the form of a report which includes those drugs and/or combination of drugs tested in the avatar screening assay, the results from those tests, and a recommendation for a therapeutic regimen to treat the subject. *See, e.g.,* Section 5.3.3, *supra,* for the types of information that may be included in such a report.

*Treatment of Cancer Patient*. In another disclosure, provided herein are methods for treating cancer in a patient utilizing one or more drugs. *See, e.g.,* Sections 5.3.2, *supra,* and 7 and 8, *infra,* for examples of such drugs. Provided herein is a method for treating cancer in a patient comprising administering to the patient an effective amount of a drug or a combination of drugs, wherein the drug or combination of drugs in an avatar screening assay described herein increases the survival of the avatar. The patient treated in accordance with the methods described herein may have been diagnosed with cancer. Techniques for diagnosing cancer are known to one of skill in the art, MRI, X-ray, CT Scan etc.

Also provided herein is a method for treating cancer in a patient comprising administering to the patient an effective amount of a drug or a combination of drugs, wherein the drug or combination of drugs in a fly avatar (*e.g.*, Drosophila avatar) screening assay described herein increases the survival of the fly avatar. In a specific disclosure, the increase in survival of the fly avatar is at least a 20%, 25%, 30%, 35%, 40%, 45%, 50%, 55%, 60%, 65%, 70%, 75%, 80%, 85%, 90%, 95% or 98% increase in survival to pupation. In a specific disclosure, the increase in survival of the fly avatar is at least a 20%, 25%, 30%, 35%, 40%, 45%, 50%, 55%, 60%, 65%, 70%, 75%, 80%, 85%, 90%, 95% or 98% increase in survival to pupariation. In another specific disclosure, the increase in survival of the fly avatar is at least a 20%, 25%, 30%, 35%, 40%, 45%, 50%, 55%, 60%, 65%, 70%, 75%, 80%, 85%, 90%, 95% or 98% increase in survival to adulthood. In another disclosure, provided herein is a method for treating cancer in a patient comprising administering to the patient an effective amount of a drug or a combination of drugs, wherein the drug or combination of drugs in a fly avatar (*e.g.*, Drosophila avatar) screening assay described herein reduces the degree of the lethality phenotype in the fly avatar or fly avatar army. In a specific disclosure, the drug or a combination of drugs that reduces the degree of the lethality phenotype of the avatar as described herein by at least about 1.5-fold, 2-fold, 2.5-fold, 3-fold, 3.5-fold, 4-fold, 4.5-fold, 5-fold, 6-fold, 7-fold, 8-fold, 9-fold, or 10-fold or more can be used to treat a cancer patient. In another disclosure, provided herein is a method for treating cancer in a patient comprising administering to the patient an effective amount of a combination of drugs, wherein the combination of drugs in a fly avatar (*e.g., Drosophila* avatar) screening assay described herein has a synergistic effect in rescuing the lethality phenotype or reducing the degree of the lethality phenotype in the avatar or avatar army is used to treat a cancer patient.

In a specific disclosure, a method for treating a subject diagnosed with cancer, comprises: administering a therapeutic regimen to a subject diagnosed with cancer, wherein the therapeutic regimen comprises a drug or a combination of drugs, wherein
(a) cancer cells from the subject exhibit increased activity of one or more oncogenes and/or reduced activity of one or more tumor suppressors, and
(b) the drug or combination of drugs, when fed to a culture of a *Drosophila* larva avatar, allows the *Drosophila* larva avatar to survive to pupation, wherein the *Drosophila* larva avatar is genetically modified such that upon induction through an external factor there is an increase in the activity of an ortholog(s) of the subject's oncogene(s) and/or inhibition an ortholog(s) of the subject's tumor suppressor(s) in a larval tissue that is necessary for survival to pupation, which increase in activity and/or inhibition prevents an untreated *Drosophila* larva avatar from surviving to pupation.

In a specific disclosure, a method for treating a subject diagnosed with cancer, comprises: administering a therapeutic regimen to a subject diagnosed with cancer, wherein the therapeutic regimen comprises a drug or a combination of drugs, wherein
(a) cancer cells from the subject exhibit increased activity of one or more oncogenes and/or reduced activity of one or more tumor suppressors, and
(b) the drug or combination of drugs, when fed to a culture of a *Drosophila* larva avatar, allows the *Drosophila* larva avatar to survive to pupariation, wherein the *Drosophila* larva avatar is genetically modified such that upon induction through an external factor there is an increase in the activity of an ortholog(s) of the subject's oncogene(s) and/or inhibition an ortholog(s) of the subject's tumor suppressor(s) in a larval tissue that is necessary for survival to pupariation, which increase in activity and/or inhibition prevents an untreated *Drosophila* larva avatar from surviving to pupariation.

In certain embodiments, the drug or a combination of drugs used to treat cancer in a patient is a drug described herein. *See, e.g.,* Section 5.3.2, *supra,* and Sections 6, 7, and 8, *infra,* for examples of drugs. In certain embodiments, one or more of the combinations listed in Table 2 is not used to treat the particular cancer listed or, in certain embodiments, any type of cancer.

In some embodiments, the drug used to treat cancer is vandetanib, sunitinib, sorafenib, AD57 (the DFG-out Type II compound), AD58, AD80, caprelsa, or the Ret-targeting inhibitor DL06. In other embodiments, the drug vandetanib, sunitinib, sorafenib, AD57 (the DFG-out Type II compound), AD58, AD80, caprelsa, or the Ret-targeting inhibitor DL06 is not used to treat cancer.

**Table 2**

| **Drug Combination Name** | **Drugs in the Drug Combination** | **Indication** |
|---|---|---|
| ABVD | A = Doxorubicin Hydrochloride (Adriamycin); | Hodgkin lymphoma |
| | B = Bleomycin; | |
| | V = Vinblastine Sulfate; and | |
| | D = Dacarbazine | |
| ABVE | A = Doxorubicin Hydrochloride (Adriamycin); | Hodgkin lymphoma |
| | B = Bleomycin; | |
| | V = Vinblastine Sulfate; and | |
| | E = Etoposide | |
| ABVE-PC | A = Doxorubicin Hydrochloride (Adriamycin); | Hodgkin lymphoma |
| | B = Bleomycin; | |
| | V = Vinblastine Sulfate; | |
| | E = Etoposide; | |
| | P = Prednisone; and | |
| | C = Cyclophosphamide | |
| AC | A = Doxorubicin Hydrochloride (Adriamycin); and | Breast cancer |
| | C = Cyclophosphamide | |
| AC-T | A = Doxorubicin Hydrochloride (Adriamycin); | Breast cancer |
| | C = Cyclophosphamide; and | |
| | T = Paclitaxel (Taxol) | |
| ADE | A = Cytarabine (Ara-C); | Acute myeloid leukemia |
| | D = Doxorubicin Hydrochloride; and | |
| | E = Etoposide | |
| BEACOPP | B = Bleomycin; | Hodgkin lymphoma |
| | E = Etoposide; | |
| | A = Doxorubicin Hydrochloride (Adriamycin); | |
| | C = Cyclophosphamide; | |
| | O = Vincristine Sulfate (Oncovin); | |
| | P = Procarbazine Hydrochloride; and | |
| | P = Prednisone | |
| BEP | B = Bleomycin; | Ovarian germ cell tumors; Testicular germ cell tumors |
| | E = Etoposide; and | |
| | P = Cisplatin (Platinol) | |
| CAF | C = Cyclophosphamide; | Breast cancer |
| | A = Doxorubicin Hydrochloride (Adriamycin); and | |
| | F = Fluorouracil | |
| CAPOX | CAP = Capecitabine; and | Colorectal cancer |
| | OX = Oxaliplatin | |
| CARBOPLATIN-TAXOL | Carboplatin; and Paclitaxel (Taxol) | Non-small cell lung cancer; Ovarian cancer |
| CHLORAMBUCIL-PREDNISONE | Chlorambucil; and Prednisone | Chronic lymphocytic leukemia |
| CHOP | C = Cyclophosphamide; | Non-Hodgkin |
| | H = Doxorubicin Hydrochloride (Hydroxydaunomycin); | Lymphoma |
| | O = Vincristine Sulfate (Oncovin); and | |
| | P = Prednisone | |
| CMF | C = Cyclophosphamide; | Breast cancer |
| | M = Methotrexate; and | |
| | F = Fluorouracil | |
| COPDAC | C = Cyclophosphamide; | Hodgkin |
| | O = Vincristine Sulfate (Oncovin); | lymphoma |
| | P = Prednisone; and | |
| | DAC = Dacarbazine | |
| COPP | C = Cyclophosphamide; | Hodgkin |
| | O = Vincristine Sulfate (Oncovin); | lymphoma; |
| | P = Procarbazine Hydrochloride; and | Non-Hodgkin |
| | P = Prednisone | Lymphoma |
| COPP-ABV | C = Cyclophosphamide; | Hodgkin |
| | O = Vincristine Sulfate (Oncovin); | lymphoma |
| | P = Procarbazine Hydrochloride; | |
| | P = Prednisone; | |
| | A = Doxorubicin Hydrochloride (Adriamycin); | |
| | B = Bleomycin; and | |
| | V = Vinblastine Sulfate | |
| CVP | C = Cyclophosphamide; | Non-Hodgkin |
| | V = Vinblastine Sulfate; and | Lymphoma; |
| | P = Prednisone | Chronic lymphocytic leukemia |
| EPOCH | E = Etoposide; | Non-Hodgkin |
| | P = Prednisone; | Lymphoma |
| | O = Vincristine Sulfate (Oncovin); | |
| | C = Cyclophosphamide; and | |
| | H = Doxorubicin Hydrochloride (Hydroxydaunomycin) | |
| FEC | F = Fluorouracil; | Breast cancer |
| | E = Epirubicin Hydrochloride; and | |
| | C = Cyclophosphamide | |
| FOLFIRI | FOL = Leucovorin Calcium (Folinic Acid); | Colorectal cancer |
| | F = Fluorouracil; and | |
| | IRI = Irinotecan Hydrochloride | |
| FOLFIRI-BEVACIZUMAB | FOL = Leucovorin Calcium (Folinic Acid); | Colorectal cancer |
| | F = Fluorouracil; | |
| | IRI = Irinotecan Hydrochloride; and Bevacizumab | |
| FOLFIRI-CETUXIMAB | FOL = Leucovorin Calcium (Folinic Acid); | Colorectal cancer |
| | F = Fluorouracil; | |
| | IRI = Irinotecan Hydrochloride; and Cetuximab | |
| FOLFIRINOX | FOL = Leucovorin Calcium (Folinic Acid); | Pancreatic cancer |
| | F = Fluorouracil; | |
| | IRIN = Irinotecan Hydrochloride; and | |
| | OX = Oxaliplatin | |
| FOLFOX | FOL = Leucovorin Calcium (Folinic Acid); | Colorectal cancer |
| | F = Fluorouracil; and | |
| | OX = Oxaliplatin | |
| FU-LV | FU = Fluorouracil; and | Colorectal cancer; Esophageal cancer; Gastric cancer |
| | LV = Leucovorin Calcium | |
| GEMCITABINE-CISPLATIN | Gemcitabine Hydrochloride; and Cisplatin | Biliary tract cancer; Bladder cancer; Cervical cancer; Malignant mesothelioma; Non-small cell lung cancer; Ovarian cancer; Pancreatic cancer |
| GEMCITABINE-OXALIPLATIN | Gemcitabine Hydrochloride; and Oxaliplatin | Pancreatic cancer |
| hyper-CV AD | C = Cyclophosphamide; V = Vincristine Sulfate; | Acute lymphoblastic leukemia; Non-Hodgkin lymphoma |
| | A = Doxorubicin Hydrochloride (Adriamycin); and | |
| | D = Dexamethasone | |
| ICE | I = Ifosfamide | Hodgkin lymphoma ; Non-Hodgkin lymphoma |
| | C = Carboplatin; and | |
| | E = Etoposide | |
| MOPP | M = Mechlorethamine Hydrochloride; | Hodgkin lymphoma |
| | O = Vincristine Sulfate (Oncovin); | |
| | P = Procarbazine Hydrochloride; and | |
| | P = Prednisone | |
| OEPA | O = Vincristine Sulfate (Oncovin); | Hodgkin lymphoma |
| | E = Etoposide ; | |
| | P = Prednisone; and | |
| | A = Doxorubicin Hydrochloride (Adriamycin) | |
| OFF | O = Oxaliplatin; | Pancreatic cancer |
| | F = Fluorouracil; and | |
| | F = Leucovorin Calcium (Folinic Acid) | |
| OPPA | O = Vincristine Sulfate (Oncovin); | Hodgkin lymphoma |
| | P = Procarbazine Hydrochloride; | |
| | P = Prednisone; and | |
| | A = Doxorubicin Hydrochloride (Adriamycin) | |
| PAD | P = Bortezomib (PS-341); | Multiple myeloma |
| | A = Doxorubicin Hydrochloride (Adriamycin); and | |
| | D = Dexamethasone | |
| PCV | P = Procarbazine Hydrochloride; | Brain tumors |
| | C = Lomustine (CCNU); and | |
| | V = Vincristine Sulfate | |
| R-CHOP | R = Rituximab; | Non-Hodgkin Lymphoma |
| | C = Cyclophosphamide; | |
| | H = Doxorubicin Hydrochloride (Hydroxydaunomycin); | |
| | O = Vincristine Sulfate (Oncovin); and | |
| | P = Prednisone | |
| R-CVP | R = Rituximab; | Non-Hodgkin Lymphoma |
| | C = Cyclophosphamide; | |
| | V = Vinblastine Sulfate; and | |
| | P = Prednisone | |
| R-EPOCH | R = Rituximab; | B-cell non-Hodgkin Lymphoma |
| | E = Etoposide; | |
| | P = Prednisone; | |
| | O = Vincristine Sulfate (Oncovin); | |
| | C = Cyclophosphamide; and | |
| | H = Doxorubicin Hydrochloride (Hydroxydaunomycin) | |
| STANFORD V | Mechlorethamine Hydrochloride; | Hodgkin Lymphoma |
| | Vinblastine Sulfate; | |
| | Vincristine Sulfate; | |
| | Bleomycin; | |
| | Etoposide; and Prednisone | |
| TAC | T = Docetaxel (Taxotere); | Breast cancer |
| | A = Doxorubicin Hydrochloride (Adriamycin); and | |
| | C = Cyclophosphamide | |
| TPF | T = Docetaxel (Taxotere); | Squamous cell carcinoma of the head and neck; Gastric (stomach) cancer |
| | P = Cisplatin (Platinol): and | |
| | F = Fluorouracil | |
| VAMP | V = Vincristine Sulfate; | Hodgkin lymphoma |
| | A = Doxorubicin Hydrochloride (Adriamycin); | |
| | M = Methotrexate; and | |
| | P = Prednisone | |
| VeIP | Ve = Vinblastine Sulfate (Velban); | Ovarian germ cell cancer; Testicular cancer |
| | I = Ifosfamide; and | |
| | P = Cisplatin (Platinol) | |
| VIP | V = Etoposide (VePesid); | Testicular cancer |
| | I = Ifosfamide; and | |
| | P = Cisplatin (Platinol) | |
| XELIRI | XEL = Capecitabine (Xeloda); and | Colorectal cancer; Esophageal cancer; Gastric (stomach) cancer |
| | IRI = Irinotecan Hydrochloride | |
| XELOX | XEL = Capecitabine (Xeloda); and | Colorectal cancer |
| | OX = Oxaliplatin | |

A drug or a combination of drugs may be administered to a cancer patient by any route known to one of skill in the art. For example, the drug or combination of drugs may be administered locally or systemically to a cancer patient. The drug or combination of drugs may be administered to a cancer patient parenterally, orally, topically, transdermally, intranasally, intraperitoneally, intratumorally, intradermally, or intracerebrally. In some disclosures, the drug or a combination of drugs is administered subcuteanously, intravenously, or intramuscularly. If the drug or a combination of drugs is already FDA approved or approved by another regulatory authority, the drug or combination of drugs may be administered by a route previously approved by the FDA or another regulatory authority.

If a combination of drugs is administered to a cancer patient, the drugs may be administered concurrently or sequentially. For example, one drug may be administered prior to (e.g., 5 minutes, 15 minutes, 30 minutes, 45 minutes, 1 hour, 2 hours ,3 hours, 4 hours, 6 hours, 12 hours, 18 hours, 24 hours, 2 days, 5 days, 7 days, 14 days or 21 days prior to) the administration of another drug. The combination of drugs may be administered via the same route of administration or a different route of administration to the cancer patient.

The amount of a drug or a combination of drugs to use to treat a cancer patient may be extrapolated by one of skill in the art from the avatar. Alternatively or in addition, the amount of a drug or a combination of drugs to administer to a cancer patient may be determined based on cell culture studies using patient cancer cells exposed to the drug or combination of drugs and/or animal model studies (*e.g.*, patient xenograft studies) using the drug or combination of drugs. If the drug or a combination of drugs has been FDA approved or approved by another regulatory authority, the dose and/or frequency of administration previously approved can be used to determine the dosing regimen for the drug or combination of drugs. In some disclosures, a low dose of a drug or a combination of drugs is initially administered to a cancer patient and the dose is increased over time. In addition or alternatively, the frequency of the drug or the combination of drugs administered to the patient may be altered (*e.g.*, increased or decreased). In certain disclosures, the dose and/or frequency of administration of a drug or a combination of drugs to a cancer patient is adjusted over time depending on the condition of the patient (*e.g.,* the growth of a tumor, whether the cancer has metastasized, whether the patient is experiencing any side effects, such as adverse events, etc.).

In some disclosures, the dose and/or frequency of administration of a drug or a combination of drugs to a cancer patient is adjusted based on the expression or activity of a disease driver in a sample from the subject after administration of the drug or combination or drugs to the subject. For example, the dosing frequency of a drug or a combination of drugs may be increased if the expression or activity of a disease driver (*e.g.*, oncogene) is increased in a sample from the patient after administration of the drug or combination of drugs to the cancer patient as compared to the expression or activity of the disease driver (*e.g.*, oncogene) prior to administration of the drug or combination of drugs to the cancer patient. The dosing frequency of a drug or a combination of drugs may be increased if the expression or activity of a disease driver (*e.g.*, tumor suppressor) is decreased in the sample from the patient after administration of the drug or combination of drugs to the cancer patient to the patient as compared to the expression or activity of the disease driver (*e.g.*, tumor suppressor) prior to administration of the drug or combination of drugs to the cancer patient.

The dosing frequency of a drug or a combination of drugs may be decreased or maintained if the expression or activity of a disease driver (*e.g.*, oncogene) is decreased in the sample from the cancer patient after administration of the drug or combination of drugs to the patient as compared to the activity of the disease driver (*e.g.*, oncogene) prior to administration of the drug or combination of drugs to the patient. The dosing frequency of a drug or a combination of drugs may be decreased or maintained if the expression or activity of a disease driver (*e.g.*, tumor suppressor) is increased in the sample from the cancer patient after administration of the drug or combination of drugs to the patient as compared to the activity of the disease driver (*e.g.*, tumor suppressor) prior to administration of the drug or combination of drugs to the patient.

In certain disclosures, the efficacy of a drug or a combination of drugs for the treatment of a cancer patient is assessed by measuring one or more biomarkers of a particular cancer. Biomarkers for different types of cancers are known in the art and can be used to assess the efficacy of a drug or a combination of drugs to treat a particular type of cancer in a patient. Techniques known to one of skill in the art can be used to measure the biomarkers at the RNA and/or protein level.

*Additional Assays.* In certain embodiments, the drug or combination of drugs identified or confirmed in an avatar screening assay described herein are tested in one or more other assays prior to providing a list of the results from the screening assay to a healthcare provider and/or the subject; a recommendation for treatment of the subject with a particular drug or combination of drugs; or treating the subject with a particular drug or combination of drugs. The other assays may include (i) assessing the efficacy of the drug or combination of drugs identified in the avatar screening assay described herein on cancer cells from the subject or a cell line derived from the subject's cancer; (ii) the safety of the drug or combination of drugs identified in the avatar screening assay described herein on non-cancerous cells; and (iii) testing the safety and/or efficacy of the drug or combination of drugs in an animal model of the subject's cancer *(e.g.,* a xenograft). *See, e.g.,* Section 9, *infra,* for examples of such assays. If the drug or combination of drugs identified or confirmed in the avatar screening assay described herein is already FDA approved, there may be no need to conduct any other assays aside from the avatar assay to assess the safety of the drug or combination of drugs and/or to assess the therapeutic indices of the drug or combination of drugs. If a personalized avatar cannot be constructed (e.g., because of time constraints), then a generic avatar used in the avatar screening assay and the efficacy of the drug or combination of drugs identified or confirmed in that assay may be tested in a cell culture of the patient's cells or a patient xenograft.

### 5.4.1. Cancers that can be Treated

In a specific embodiment, a patient described herein has cancer. In particular embodiment, the patient has been diagnosed with a cancer. *See* this section below for examples of types of cancers that a patient may have/be diagnosed with having. In certain embodiments, the patient has failed standard of care therapy. In some embodiments, the patient has a cancer for which there is no standard of care. In certain embodiments, the cancer patient is a naive to treatment (*i.e.,* the patient has not been treated for his/her cancer). In some embodiments, the cancer patient has previously undergone cancer therapy but was unresponsive to the therapy or the therapy was too toxic for the patient. In a specific embodiment, the cancer patient is a human.

Examples of cancers include, but are not limited to, carcinoma, sarcoma, leukemia, lymphoma, myeloma, and brain and spinal cord cancer. The cancer may be a bladder cancer, bone cancer, breast cancer, cervical cancer, esophageal cancer, gallbladder cancer, head and neck cancer, kidney cancer, lung cancer, ocular cancer, oral cancer, ovarian cancer, pancreatic cancer, pelvic cancer, penile cancer, prostate cancer, skin cancer, throat cancer, thyroid cancer, and uterine cancer.

In some embodiments, the cancer is a carcinoma. In some embodiments, the carcinoma is a squamous cell carcinoma, adenocarcinoma, transitional cell carcinoma, or basal cell carcinoma. In some embodiments, the carcinoma is a carcinoma of the breast, colon, liver, lung, pancreas, prostate, or stomach. In some embodiments, the cancer is a solid tumor.

In some embodiments, the cancer is a sarcoma. Examples of sarcomas include, but are not limited to, bone sarcomas and soft-tissue sarcomas. In some embodiments, the bone sarcoma is osteosarcoma, Ewing's sarcoma or chondrosarcoma. In some embodiments, the soft-tissue sarcoma is liposarcoma, fibrosarcoma, dermatofibrosarcoma protuberans, malignant fibrous histiocytoma, synovial cell sarcoma, epithelioid sarcoma, rhabdomyosarcoma, leiomyosarcoma, uterine sarcoma, gastrointestinal sarcoma, myxoma, mesenchymomas, vascular sarcoma, malignant neurilemoma, alveolar soft-parts sarcoma, or Kaposi's sarcoma. In some embodiments, the malignant fibrous histiocytoma is storiform pleomorphic, myxoid malignant fibrous histiocytoma, malignant giant cell tumor of soft parts, or inflammatory malignant fibrous histiocytoma. In some embodiments, the rhabdomyosarcoma is embryonal rhabdomyosarcoma, alveolar rhabdomyosarcoma, botryoid rhabdomyosarcoma, or pleomorphic rhabdomyosarcoma. In some embodiments, the vascular sarcoma is hemangioendothelioma, angiosarcoma, or hemangiopericytoma.

In some embodiments, the cancer is a leukemia. In some embodiments, the leukemia is an acute leukemia. In some embodiments, the acute leukemia is acute myeloid leukemia or acute lymphoblastic leukemia. In some embodiments, the leukemia is a chronic leukemia. In some embodiments, the chronic leukemia is chronic myeloid leukemia, chronic lymphocytic leukemia, or hairy cell leukemia.

In some embodiments, the cancer is a lymphoma or myeloma. In some embodiments, the lymphoma is Hodgkin lymphoma or non-Hodgkin lymphoma.

In some embodiments, the cancer is a brain and spinal cord cancer. In some embodiments, the brain and spinal cord cancer is astrocytoma, glioma, meningioma, peituatry tumor, craniopharyngioma, germ cell tumor, pineal region tumor, meduloblastoma, or primary central nervous system lymphoma. In some embodiments, the glioma is ependymoma, oligodengroglioma, or mixed glioma.

In some embodiments, the cancer is a breast cancer. In some embodiments, the breast cancer is ductal carcinoma in situ, invasive (or infiltrating) ductal carcinoma, invasive (or infiltrating) lobular carcinoma, inflammatory breast cancer, Paget disease, or phyllodes tumor. In some embodiments, the infiltrating ductal carcinoma is adenoid cystic carcinoma, low-grade adenosquamous carcinoma, medullary carcinoma, metaplastic carcinoma, micropapillary carcinoma, mixed carcinoma, mucinous carcinoma, papillary carcinoma, tubular carcinoma. In some embodiments, the breast cancer is invasive breast cancer, ductal carcinoma, lobular carcinoma, inflammatory breast cancer, male breast cancer, metastatic brast cancer, papillary carcinoma, or triple-negative breast cancer.

In some embodiments, the cancer is a colorectal cancer. In some embodiments, the colorectal cancer is a colorectal adenocarcinoma, gastrointestinal stromal tumor, colorectal squamous cell carcinoma, gastrointestinal carcinoid tumor, primary colorectal lymphoma, colorectal melanoma and colorectal leiomyosarcoma.

In some embodiments, the cancer is a liver cancer. In some embodiments, the liver cancer is hepatocellular carcinoma (HCC), fibrolamellar hepatocellular carcinoma, cholangiocarcinoma, angiosarcoma, or secondary liver cancer.

In some embodiments, the cancer is a lung cancer. In some embodiments, the lung cancer is a small cell lung cancer or non-small cell lung cancer. In some embodiments, the lung cancer is a metastatic lung cancer. In some embodiments, the non-small cell lung cancer is squamous cell lung cancer, adenocarcinoma lung cancer, or large-cell undifferentiated carcinoma lung cancer.

In some embodiments, the cancer is a pancreatic cancer. In some embodiments, the pancreatic cancer is an exocrine tumor or endocrine tumor.

In some embodiments, the cancer is a prostate cancer. In some embodiments, the prostate cancer is an adenocarcinoma or sarcoma.

In some embodiments, the cancer is a stomach cancer. In some embodiments, the stomach cancer is an adenocarcinoma, lymphoma, gastrointestinal stromal tumor, or carcinoid tumor.

In some embodiments, the cancer is an oral cancer. In some embodiments, the oral cancer is a squamous cell carcinoma, verrucous carcinoma, minor salivary gland carcinoma, lymphoma, benign oral cavity and oropharyngeal tumor, leukoplakia, and erythroplakia. In some embodiments, the oral cancer is a jaw cancer, lip cancer, mouth cancer, or tongue cancer.

In some embodiments, the cancer is a bladder cancer. In some embodiments, the bladder cancer is a transitional cell (urothelial) carcinoma, squamous cell carcinoma, adenocarcinoma, small-cell carcinoma, or sarcoma. In some embodiments, the transitional cell carcinoma is a papillary carcinoma or flat carcinoma.

In some embodiments, the cancer is an ovarian cancer. In some embodiments, the ovarian cancer is an epithelial ovarian cancer, germ cell ovarian cancer, or sex cord-stromal ovarian cancer.

In some embodiments, the cancer is a uterine cancer. In some embodiments, the uterine cancer is an endometrial cancer or uterine sarcoma.

In some embodiments, the cancer is a throat cancer. In some embodiments, the throat cancer is a squamous cell carcinoma or adenocarcinoma. The throat cancer may be laryngeal cancer or pharyngeal cancer. The pharyngeal cancer may be nasopharynx cancer, oropharync cancer, or hypopharynx cancer.

In some embodiments, the cancer is a thyroid cancer. In some embodiments, the thyroid cancer is a papillary carcinoma, follicular carcinoma, Hurthle cell carcinoma, medullary thyroid carcinoma, and anaplastic carcinoma.

In some embodiments, the cancer is a skin cancer. In some embodiments, the skin cancer is melanoma, basal cell carcinoma, or squamous cell carcinoma.

In some embodiments, the cancer is thyroid cancer, medullary thyroid cancer, or multiple endocrine neoplasia type 2. In some embodiments, the cancer is not thyroid cancer, medullary thyroid cancer, or multiple endocrine neoplasia type 2.

### 5.4.2. Treatment of Cancer

In certain disclosures, a method of treating cancer as described herein results in one, two, three or more of the following effects: complete response, partial response, increase in overall survival, increase in disease free survival, increase in objective response rate, increase in time to progression, increase in progression-free survival, increase in time-to-treatment failure, and improvement or elimination of one or more symptoms of cancer. In a specific disclosure, a method of treating cancer as described herein results in an increase in overall survival. In another specific disclosure, a method of treating cancer as described herein results in an increase in progression-free survival. In another specific disclosure, a method of treating cancer as described herein results in an increase in overall survival and an increase in progression-free survival. *See, e.g.,* Section 5.4.1, *supra,* for cancers to be treated.

"Complete response" refers to an absence of clinically detectable disease with normalization of any previously abnormal radiographic studies, bone marrow, and cerebrospinal fluid (CSF) or abnormal monoclonal protein measurements.

"Partial response" refers to at least about a 10%, 20%, 30%, 40%, 50%, 60%, 70%, 80%, or 90% decrease in all measurable tumor burden (*i.e.,* the number of malignant cells present in the subject, or the measured bulk of tumor masses or the quantity of abnormal monoclonal protein) in the absence of new lesions.

"Overall survival" is defined as the time from randomization until death from any cause, and is measured in the intent-to-treat population. Overall survival should be evaluated in randomized controlled studies. Demonstration of a statistically significant improvement in overall survival can be considered to be clinically significant if the toxicity profile is acceptable, and has often supported new drug approval.

Several endpoints are based on tumor assessments. These endpoints include disease free survival (DFS), objective response rate (ORR), time to progression (TTP), progression-free survival (PFS), and time-to-treatment failure (TTF). The collection and analysis of data on these time-dependent endpoints are based on indirect assessments, calculations, and estimates (e.g., tumor measurements).

Generally, "disease free survival" (DFS) is defined as the time from randomization until recurrence of tumor or death from any cause. Although overall survival is a conventional endpoint for most adjuvant settings, DFS can be an important endpoint in situations where survival may be prolonged, making a survival endpoint impractical. DFS can be a surrogate for clinical benefit or it can provide direct evidence of clinical benefit. This determination is based on the magnitude of the effect, its risk-benefit relationship, and the disease setting. The definition of DFS can be complicated, particularly when deaths are noted without prior tumor progression documentation. These events can be scored either as disease recurrences or as censored events. Although all methods for statistical analysis of deaths have some limitations, considering all deaths (deaths from all causes) as recurrences can minimize bias. DFS can be overestimated using this definition, especially in patients who die after a long period without observation. Bias can be introduced if the frequency of long-term follow-up visits is dissimilar between the study arms or if dropouts are not random because of toxicity.

"Objective response rate" (ORR) is defined as the proportion of patients with tumor size reduction of a predefined amount and for a minimum time period. Response duration usually is measured from the time of initial response until documented tumor progression. Generally, the FDA has defined ORR as the sum of partial responses plus complete responses. When defined in this manner, ORR is a direct measure of drug antitumor activity, which can be evaluated in a single-arm study. If available, standardized criteria should be used to ascertain response. A variety of response criteria have been considered appropriate (*e.g.*, RECIST criteria) (Therasse et al., (2000) J. Natl. Cancer Inst, 92: 205-16). The significance of ORR is assessed by its magnitude and duration, and the percentage of complete responses (no detectable evidence of tumor).

"Time to progression" (TTP) and "progression-free survival" (PFS) have served as primary endpoints for drug approval. TTP is defined as the time from randomization until objective tumor progression; TTP does not include deaths. PFS is defined as the time from randomization until objective tumor progression or death. Compared with TTP, PFS is the preferred regulatory endpoint. PFS includes deaths and thus can be a better correlate to overall survival. PFS assumes patient deaths are randomly related to tumor progression. However, in situations where the majority of deaths are unrelated to cancer, TTP can be an acceptable endpoint.

As an endpoint to support drug approval, PFS can reflect tumor growth and be assessed before the determination of a survival benefit. Its determination is not confounded by subsequent therapy. For a given sample size, the magnitude of effect on PFS can be larger than the effect on overall survival. However, the formal validation of PFS as a surrogate for survival for the many different malignancies that exist can be difficult. Data are sometimes insufficient to allow a robust evaluation of the correlation between effects on survival and PFS. Cancer trials are often small, and proven survival benefits of existing drugs are generally modest. The role of PFS as an endpoint to support licensing approval varies in different cancer settings. Whether an improvement in PFS represents a direct clinical benefit or a surrogate for clinical benefit depends on the magnitude of the effect and the risk-benefit of the new treatment compared to available therapies.

"Time-to-treatment failure" (TTF) is defined as a composite endpoint measuring time from randomization to discontinuation of treatment for any reason, including disease progression, treatment toxicity, and death. TTF is not recommended as a regulatory endpoint for drug approval. TTF does not adequately distinguish efficacy from these additional variables. A regulatory endpoint should clearly distinguish the efficacy of the drug from toxicity, patient or physician withdrawal, or patient intolerance.

In some disclosures, treating a subject comprises an improvement in and/or the elimination of one or more symptoms of cancer in the subject. In some disclosures, treating a subject comprises an improvement in and/or the elimination of one or more symptoms of leukemia in the subject. The one or more symptoms of leukemia may include, but are not limited to, fever, chills, fatigue, weakness, loss of appetite, weight loss, night sweats, bone/joint pain, abdominal discomfort, headaches, shortness of breath, frequent infections, easy bruising or bleeding, petechia, anemia, leucopenia, thrombocytopenia, swollen lymph nodes, and enlarged liver or spleen.

In some disclosures, treating a subject comprises an improvement in and/or the elimination of one or more symptoms of lymphoma in the subject. The one or more symptoms of lymphoma may include, but are not limited to, fever, swollen lymph nodes in the neck, underarms and groin, night sweats, night chills, persistent fatigue, lethargy, feeling of tiredness, loss of appetite, nausea, vomiting, unexplained weight loss, abdominal pain or swelling, feeling of fullness, skin rash or itchy skin, coughing, shortness of breath, headaches, difficulty moving parts of the body, and pain in the chest, abdomen or bones for no known reason.

In some disclosures, treating a subject comprises an improvement in and/or the elimination of one or more symptoms of bone cancer in the subject. The one or more symptoms of bone cancer may include, but are not limited to, bone pain, swelling, fractures, decreased mobility, unintended weight loss, fatigue, and difficulty breathing.

In some disclosures, treating a subject comprises an improvement in and/or the elimination of one or more symptoms of brain cancer in the subject. The one or more symptoms of brain cancer may include, but are not limited to, headaches, vision changes, loss or motor skill, naseau, vomiting, seizures, speech problems, cognitive problems, weakness, and numbness.

In some disclosures, treating a subject comprises an improvement in and/or the elimination of one or more symptoms of breast cancer in the subject. The one or more symptoms of breast cancer may include, but are not limited to, skin changes, increase in size or change in shape of the breasts, changes in the appearance of one or both nipples, nipple discharge other than breast milk, general pain in/on any part of the breast, lumps or nodes felt on or inside of the breast, irritated or itchy breasts, change in breast color, and peeling or flaking of the nipple skin. Skin changes may include, but are not limited to, swelling, redness, and other visible differences in one or both breasts.

In some disclosures, treating a subject comprises an improvement in and/or the elimination of one or more symptoms of colorectal cancer in the subject. The one or more symptoms of colorectal cancer may include, but are not limited to, changes in bowel habits, constipation, diarrhea, alternating diarrhea and constipation, rectal bleeding or blood in stool, abdominal bloating, abdominal cramps, abdominal discomfort, gas pains, feeling of incomplete bowel emptying, thinner than normal stools, unexplained weight loss, unexplained loss of appetite, nausea, vomiting, anemia, jaundice, weakness, and fatigue or tiredness. Colorectal cancer symptoms may also include pain, fracture, constipation, decreased alertness, shortness of breath, difficulty breathing, coughing, chest wall pain, extreme fatigue, increased abdominal girth, swelling of the feet and hands, yellowing or itch skin, bloating, swollen belly, pain, confusion, memory loss, headache, blurred or double vision, difficulty with speech, difficulty with movement, and seizures.

In some disclosures, treating a subject comprises an improvement in and/or the elimination of one or more symptoms of liver cancer in the subject. The one or more symptoms of liver cancer may include, but are not limited to, weight loss, decrease in appetite, nausea and vomiting, general weakness and/or fatigue, fever, pain, enlarged liver, enlarged spleen, abdominal swelling, and jaundice.

In some disclosures, treating a subject comprises an improvement in and/or the elimination of one or more symptoms of lung cancer in the subject. The one or more symptoms of lung cancer may include, but are not limited to, coughing, coughing up blood, breathing difficulties, loss of appetite, fatigue, recurring infections, bone pain, swelling in face, arms or neck, headaches, dizziness, limbs that become weak or numb, jaundice, and lumps in the neck or collar bone region. Breathing difficulties may include, but are not limited to, shortness of breath, wheezing and noisy breathing.

In some disclosures, treating a subject comprises an improvement in and/or the elimination of one or more symptoms of pancreatic cancer in the subject. The one or more symptoms of pancreatic cancer may include, but are not limited to, jaundice, digestive problems, pain in the upper abdomen, loss of appetite, nausea, sudden weight loss, swollen gallbladder, blood clots, and diabetes. Digestive problems may include, but are not limited to, abnormal stools, nausea, and vomiting.

In some disclosures, treating a subject comprises an improvement in and/or the elimination of one or more symptoms of prostate cancer in the subject. The one or more symptoms of prostate cancer may include, but are not limited to, urinary symptoms, blood in semen, difficulty getting an erection, painful ejaculation, swelling in legs or pelvic area, numbness or pain in the hips, legs or feet, and bone pain that doesn't go away or leads to fractures. Urinary symptoms include, but are not limited to, burning or pain during urination, difficulty urinating, or trouble starting and stopping while urinating, more frequent urges to urinate at night, loss of bladder control, decreased flow or velocity of urine stream, and blood in urine.

In some disclosures, treating a subject comprises an improvement in and/or the elimination of one or more symptoms of stomach cancer in the subject. The one or more symptoms of stomach cancer may include, but are not limited to, unexplained weight loss, stomach pain, feeling full, heartburn, nausea, and vomiting.

In some disclosures, treating a subject comprises an improvement in and/or the elimination of one or more symptoms of oral cancer in the subject. The one or more symptoms of oral cancer may include, but are not limited to, persistent mouth sore, persistent mouth pain, lump formation or thickening in the cheek, white or red patch on the gums, tongue, tonsil, or lining of the mouth, sore throat or feeling that something is caught in the throat that does not go away, difficulty swallowing or chewing, difficulty moving the jaw or tongue, numbness of the tongue or elsewhere in the mouth, jaw swelling, pain in the teeth or jaw, voice changes, lump in the neck, weight loss, and persistent bad breath.

In some disclosures, treating a subject comprises an improvement in and/or the elimination of one or more symptoms of bladder cancer in the subject. The one or more symptoms of bladder cancer may include, but are not limited to, blood in the urine, changes in urination, and lower back pain.

In some disclosures, treating a subject comprises an improvement in and/or the elimination of one or more symptoms of ovarian cancer in the subject. The one or more symptoms of ovarian cancer may include, but are not limited to, persistent abdominal bloating, indigestion, nausea, changes in appetite, such loss of appetite or feeling full sooner, feelings of pressure in the pelvic or lower back, needing to urinate more frequently, changes in bowel movements, increased abdominal girth, and feeling tired or low energy.

In some disclosures, treating a subject comprises an improvement in and/or the elimination of one or more symptoms of uterine cancer in the subject. The one or more symptoms of uterine cancer may include, but are not limited to, vaginal bleeding or spotting, abnormal vaginal discharge, and pelvic pain or pressure.

In some disclosures, treating a subject comprises an improvement in and/or the elimination of one or more symptoms of throat cancer in the subject. The one or more symptoms of throat cancer may include, but are not limited to, difficulty swallowing, changes in voice, sore throat, unexplained weight loss, swelling of the eyes, jaw, throat or neck, bleeding in the mouth or through the nose, and chronic cough.

In some disclosures, treating a subject comprises an improvement in and/or the elimination of one or more symptoms of thyroid cancer in the subject. The one or more symptoms of thyroid cancer may include, but are not limited to, neck pain, voice changes, breathing problems, coughing, and trouble swallowing.

In some disclosures, treating a subject comprises an improvement in and/or the elimination of one or more symptoms of skin cancer in the subject. The one or more symptoms of skin cancer may include, but are not limited to, unusual skin growth, persistent bump or sore, new spots on the skin, change in size, shape, or color of an existing mole, sores that don't heal, pigment, redness or swelling that spreads outside the border of a spot to the surrounding skin, itchiness, tenderness or pain, and changes in texture or scales, oozing or bleeding from an existing mole.

### 6. EXAMPLE 1

This example describes the development of fly avatars based on individual cancer patient data, such as individual colorectal cancer patient data. In addition, this example describes the development of a robust platform for screening fly avatars for compound and drug therapeutics. The information from this screen can identify drug therapeutics to treat an individual patient's cancer.

### Identifying Candidate Tumor Drivers

**Pathology:** Tissue is removed from a tumor from a patient and immediately cooled or preserved. The tissue may be assessed for purity, which ideally exceeds 40% purity. Once the purity of the tissue is validated, DNA and RNA are obtained for genomic analysis and network pathway analysis, respectively.

**Genomics:** In order to capture the full cancer network, 200X/100X depth of coverage for DNA sequencing of tumor/normal is performed to permit mutations in tumor subpopulations to be captured with confidence.

**Variant calling:** To determine whether a variant (DNA change) in the tumor alters protein function, meta-analysis software is used, including MutationAssessor to query ten different computer programs that provide a "score" for each variant. In addition, each variant is examined against the literature and against other patients' mutation profiles to determine if it occurs in an above-random frequency, within functional domains, etc.

**RNA:** RNA-Seq is used to quantify RNA expression to permit modeling of the signaling networks that are active in a tumor cell. This information is used to determine if a pathway has been activated.

### Fly Construction

If a patient's genomic analysis results in ten or fewer "hits", a fly avatar (*e.g.,* a Drosophila avatar, such as a *Drosophila melanogaster* avatar) that includes all of the fly variant orthologs is constructed. If a patient has more than ten altered proteins, a 'base' cancer model is generated and the remaining candidates are tested using a "dominant genetic modifier" screen to identify those genes that are functional drivers of tumor progression. All variants are cloned into a transformation plasmid: oncogenes are overexpressed or activated, tumor suppressors are mildly knocked down with RNA-interference. Table 3 provides a list of a genes identified in a medullary thyroid carcinoma (MTC) patient and the fly orthology.

**Table 3**

| **Fly** | **Human** |
|---|---|
| 1.Ret | RET |
| 2.Sfl | NDST3 |
| 3.Atl | ATL3 |
| 4.Ki-2 | DNAH2 |
| 5.Magi | MAGI1 |
| 6.Arm | CTNNB1 |
| 7.CG9297 | SRL |
| 8.Cnk | CNKSR3 |
| 9.Ptper | PTPRR |
| 10.Tatl | TGS1 |

Flies are constructed by a combination of activated transgenes and shRNA-mediated knockdown. The approach comprises (i) creating a single plasmid encompassing all shRNA knockdown hairpins in a single sequence and (ii) cloning all plasmids into a single transformation vector will significantly speed the process. *De novo* polycistronic transcripts with verified shRNAs are established. A single genetic element can silence as many as ten (or more) genes at once and the vector allows for overexpression of three oncogenes (Figure 1).

Up to 15 different genes are inserted into a single stable transgenic line; in tumors with more than 15 variants that alter protein function-uncommon based on CRC patient experience- a passenger-vs.-driver genetic modifier screen is performed. Transgenes are fused to a *UAS* promoter that will remain silent until activated by a simple F1 cross to a 'driver' line containing, *e.g.,* the hindgut specific driver *byn-GAL4.* In this manner, the avatar set can be kept indefinitely as a stable and unique resource for a variety of cancer studies.

All *UAS* elements are placed in the same chromosomal location via *attb*/*attp* directed integration (Venken et al., (2006) Science 314: 1747-1751), eliminating local chromatin effects due to insertion site. Equal expression levels of different combinations are confirmed by qPCR and by fluorescence microscopy of *UAS-GFP* for multiple lines. Transgenes are targeted to the hindgut for CRC models using a *byn-GAL4* driver, introduced by standard crossing.

In particular, 300 fly avatars (*e.g., Drosophila melanogaster* avatars) based on 300 CRC patients are generated. The Cancer Genome Atlas (TCGA) (Cancer Genome Atlas Research, N. et al., (2013) Nat. Genet. 45: 1113-1120) has posted 461 sequenced CRC patients. Based on the analysis conducted, 224 of these are high quality based in part on containing at least one validated cancer driver. The Vogelstein group has posted an additional 68 sequenced samples (Sjoblom et al., (2006) Science 314: 268-274; Wood et al., (2007) Science 318: 1108-1113). The 300 samples are sequenced and 300 fly avatars are produced. As more sequence information becomes available, the more useful it is for 'best fit' analysis (see below), and the generation of additional fly avatars beyond 300.

### Best-Fit Analysis for New Patients

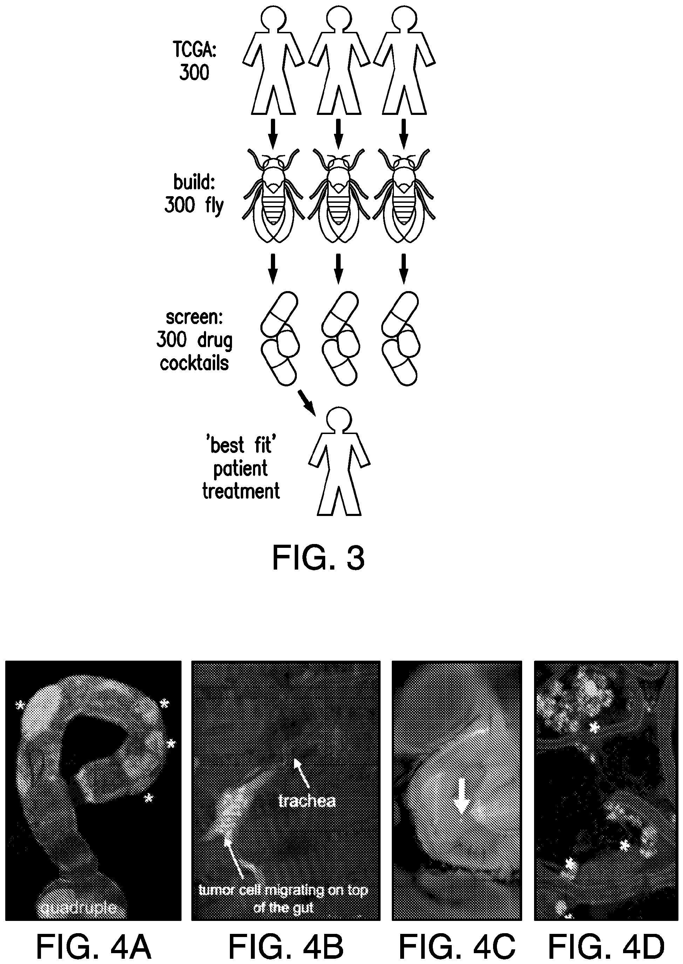

A multi-pronged approach based on the representation of each tumor in terms of its DNA variants is used; only variants modeled in the fly avatar (not all human genes have a fly ortholog) are considered. Similarity measures for used genetic variant data (Libiger et al., (2009) Human Biology 81: 389-406; Bansal et al., (2010) Nat. Rev. Genet. 11: 773-785) are used to identify the best matching fly line for a new patient. *See, e.g.,* Figure 3. Varying all the mutations may not be needed to be weighted identically in making predictions, suggesting that using a diverse set of predictors, each of which uses a different representation for the variant-to-treatment mapping/function, can improve prediction performance further. Approaches have been developed to construct ensemble predictors that can effectively combine many such diverse predictors, such as *k*-NN, decision trees, SVMs and neural networks (see DataSink tool at the following website: github.com/shwhalen/datasink) (Whalen & Pandey (2013) ICDM 807-816).

### Lethality Based Screening Method

Personalized fly avatars, such colorectal cancer (CRC) fly avatars, are built. Each avatar line to lethality is calibrated using the hindgut-specific driver *byn-GAL4*/*GAL80^{ts}* to develop adult specific models, *e.g.,* adult specific CRC models. Phenotypes are calibrated *en masse* by altering temperature; flies are isothermic and GAL4 drivers increase activity at higher temperatures. These drivers at 20-25°C are sufficient to direct lethality for most lines but other drivers or multiple transgene copies are considered if required. The goal is to establish >98% lethality for each line.

Once established, each of the avatars is screened against a library of 1200 FDA-approved drugs (Selleck Chemicals), which contains both cancer and non-cancer drugs. Each drug is dissolved into duplicate wells at 10 and 100 µM final food concentration, leading to hemolymph concentrations of ~5-500 nM in the animal. A robotics-based approach is used to screen each line rapidly and reproducibly. The robotics-based screening platform for screening flies uses a 96-well plate format (Figure 2). A PerkinElmer Janus liquid handler dispenses 150 µL of a specialized Drosophila food; dissolved drug is mixed in. A Union Biometrica COPAS embryo sorter then places 10 embryos from each patient-derived fly line into each well on top of solidified food/drug. The plate is covered with a breathable membrane and animals develop at the optimal lethality temperature. A minimum of 2 adults must emerge to mark for re-testing. Initial 'hits' are confirmed by a minimum of two additional repeats in which the drug must show consistent rescue activity.

For example for CRC models, fly avatars are screened 14 days after setup for candidate drugs' ability to rescue at least three larvae to adulthood from each of the five fly lines. Initial positives are re-screened twice. The goal is to identify lead drugs that improve animal survival. Once hits are identified, the remaining FDA drugs are re-screened through two additional rounds to identify 1-2 synergistic drugs. The aim is to identify the best drug or drug cocktail in respect to animal survival and, using GFP, tumor shrinkage.

In addition, an activity curve for each candidate is established to ensure its rescue shows a progressive survival curve with a clear ED50, and to identify optimal dosing. Drugs are ranked based on (i) percent adult rescue, (ii) ability to visibly decrease tumor progression as assessed by GFP, (iii) demonstration of a smooth concentration activity curve, (iv) likely disease utility based on its previous clinical history of efficacy and especially toxicity, and (v) reasonable cost and accessibility of the drug. For each avatar, 1-3 of the highest ranked drugs are fed at rescuing doses, then animals are re-screened for synergistic drugs. Avatars that cannot be rescued to at least 30% by at least one cocktail are removed from the set. By identifying unique 2-3 drug combinations that improve survival of the fly cancer model, a set of potentially useful drug cocktails are ranked.

### Proteomics

A simple proteomic analysis may be added to the model building and assessment. Patient stem cell samples may be tested with phospho-antibody-based human arrays (*RTK Signaling* and *Pathscan Intracellular Signaling* arrays, Cell Signaling Technologies). These arrays monitor activity of 57 human proteins using antibodies to components of the Ras, PI3K, Jnk, Src, etc. pathways. This provides an overview of the major pathways that are altered, in a pure population from each patient. Controls are included from, *e.g.,* control colorectal iPSCs (Ng et al., (2014) Cell Death Dis. 5: e1024). In addition, CyTOF Mass Cytometry may be evaluated to survey phospho-peptides. In short, cell lysates are harvested, lysed, and probed with the arrays by standard protocol. Signal intensity is quantitated and graphed to facilitate comparisons between patient and control. Differences of >30% are validated by western analysis with phospho-antibodies. These detailed biochemical results are compared with the biochemical data obtained in the Drosophila models, again to determine the predictive value of the fly platform.

A similar analysis on the fly avatars is performed. The goal is to add additional resolution and refinement to the matching of fly and patient.

### 7. EXAMPLE 2

This example describes the construction of fly avatars, specifically *Drosophila melanogaster* avatars, for human colorectal cancer and the use of those fly avatars to screen for drug therapeutics to treat colorectal cancer. Some of the techniques referenced in Section 6 were used to generate the fly avatars and to screen those fly avatars for drug therapeutics.

Mutational profiling studies of primary human colon tumor samples identified 280 candidate genes from 38 tumors as potential drivers of tumorigenesis (Leary et al., (2008) Proceedings of the National Academy of Sciences of the United States of America 105: 16224-16229). Analysis of the most frequently mutated genes in these tumors revealed frequent deregulation of Wnt, RTK/Ras, p53, TGF-beta and PI3K pathways, all of which have been previously linked to colon cancer (Takayma et al., (2006) J. Gastroenterol. 41: 185-192) *See* Table 4, *infra,* for the human data and fly constructs used to model each pathway. The number of human tumors that carried double, triple and quadruple combinations of mutations of these five pathways were determined. Transgenic lines representing the most frequently observed mutations in these tumors were used to model the most common human quadruple combinations in Drosophila. In addition, the full 15 single, double and triple sub-combinations for each quadruple were generated; a total of 29 unique transgenic lines were generated since many combinations overlap.

*Drosophila* CRC fly avatar models were built from several four-hit and five-hit cancer gene combinations reported for human CRC patients For example, the tumorigenic potential of a quadruple combination of Wnt activation, RTK/Ras activation, P53 inactivation, and PI3K activation was generated by targeting them to the adult *Drosophila* hindgut, the structure most analogous to the human colon (genotype: *UAS-dcr2; +; byn-gal4 UAS-GFP tub-gal80^{ts}, UAS-ras^{V12}, UAS-p53(RNAi), UAS-pten(RNAi), UAS-apc1(RNAi)).* The *Drosophila* hindgut shows fundamental similarities to the human colon including the signaling pathways that regulate its maintenance (Fox et al., (2009) Stem Cell 5: 290-297; Takashima et al., (2008) Nature 454: 651-655).

**Table 4: Pathways frequently deregulated in human CRC and matching Drosophila transgenic 1 lines**

| **Pathway** | **Frequency (%)** | **genes (mutated, deleted/amplified)** | **transgenic lines in Drosophila** |
|---|---|---|---|
| Wnt activation | 66 | APC | dAPC^{RNAi} (UAS-dAPC1^{RNAi-}+ UASdAPC2^{RNAi}) |
| RTK/Ras activation | 63 | EGFR, other RTKs, RAS | UAS-dRas^{G12V}, UAS-dEGFR |
| p53 loss of function | 45 | P53 | UAS-dP53^{RNAi} |
| TGF-beta loss of function | 32 | SMAD2, SMAD3, SMAD4, TBRII | UAS-dSmad4^{RNAi} |
| PI3K activation | 24 | PIK3CA, PTEN | UAS-dPTEN^{RNAi} |

The Gal4/Gal80^{ts}/UAS system (Brand et al., (1993) Development 118: 401-415; McGuire et al., Sci. STKE 2004, pl6 (2004) was used to specifically target the quadruple combinations, along with a UAS-GFP transgene, to all epithelial cells of the adult colon; a temperature sensitive *Gal80^{ts}* kept transgenes silent during developmental stages (Takashima et al., (2008) Nature 454: 651-655). Targeting these quadruple combinations to the adult hindgut recapitulated several key aspects of tumorigenesis and metastasis. Example phenotypes are shown in Figure 4. Briefly, the following was observed: 1) disruption of the normal epithelial architecture and multilayering of the hindgut epithelium, 2) hyperproliferation, 3) evasion of apoptosis and senescence, two key cellular defense systems against tumorigenesis and 4) activation of metastasis-related factors and migration of hindgut cells out of the epithelium to distant sites within the body. This extensive migratory behavior was accompanied by expression of Matrix Metalloproteinase 1 (MMP1), degradation of the basement membrane, and an increase and membrane localization of phosphorylated-Src levels. Cells migrating on top of the gut were often associated with trachea, a tubular network that provides oxygen to *Drosophila* tissues (Figure 4). In addition, cells at earlier stages of basal delamination and migration were observed within the gut epithelium (Figure 4).

After migrating out of the gut epithelium, these cells go on to colonize distant sites within the body: GFP positive tumor cells can be found within the abdominal cavity, usually near the body wall, migrating along tracheal branches (Figure 4). The fact that individual migrating cells survived in these foreign environments and formed small GFP+ foci throughout the animal (Figure 4) indicate that this 'distant migration' phenotype recapitulates key early aspects of metastasis.

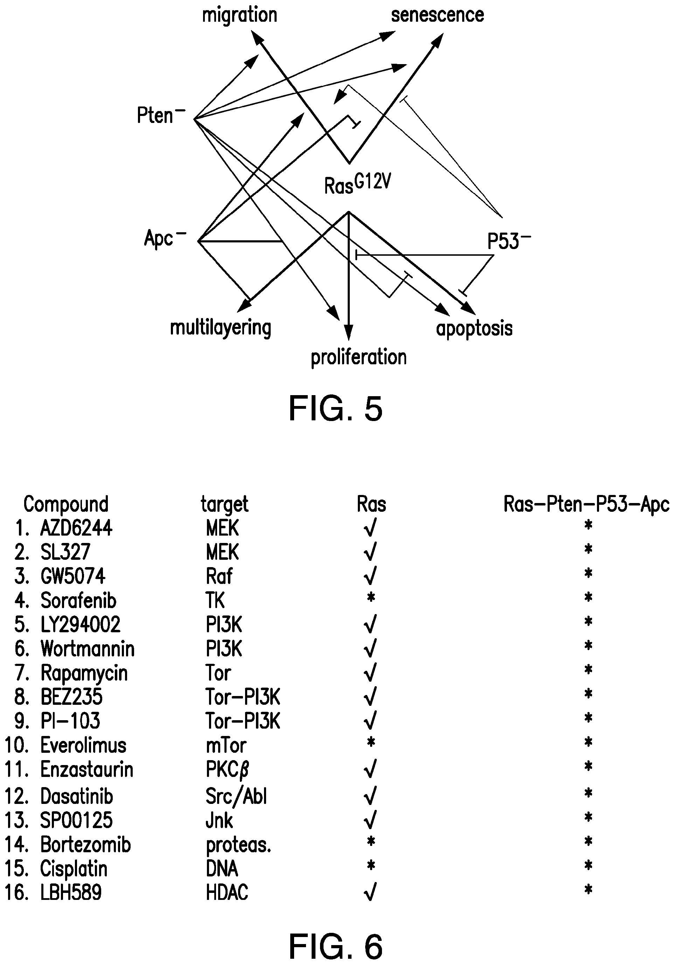

The relative contributions of individual mutations to the final tumor were investigated. Each of the sub-combinations that make up the quadruples as well as each individual transgene alone was examined. This analysis provided a detailed map of interactions between individual mutations underlying each tumor phenotype (Figure 5). The fact that many of the tumor phenotypes were emergent features of multiple mutations emphasizes the importance of using complex, multigenic models to study cancer.

Increasingly complex fly tumors led to increasing drug resistance. For example, flies were fed 16 clinically relevant drugs. For scoring, distant migration (*i.e.,* fly 'metastasis') was used as a quantitative read-out. Distant migration was quantitated by counting the number of migrating GFP+ foci (*i.e.,* 2° tumors) present within the abdominal cavity of dissected individuals. Animals were assigned into one of four categories: no migration, weak migration (1-3 GFP+ foci in the abdominal cavity), moderate migration (4-10 GFP+ foci) and strong migration (>10 GFP+ foci). 12/16 of these drugs significantly reduced cell dissemination in a one-hit dRas^{G12V} fly, mirroring results in mice. However, 0/16 of these same drugs showed detectable effects on the dRas-Pten-Apc-P53 flies (Figure 6), mirroring results in human clinical trials. This points to the key observation that, while targeted therapies work well against genetically simple models, in general it was found that complex models do not respond to most single targeted therapeutics.

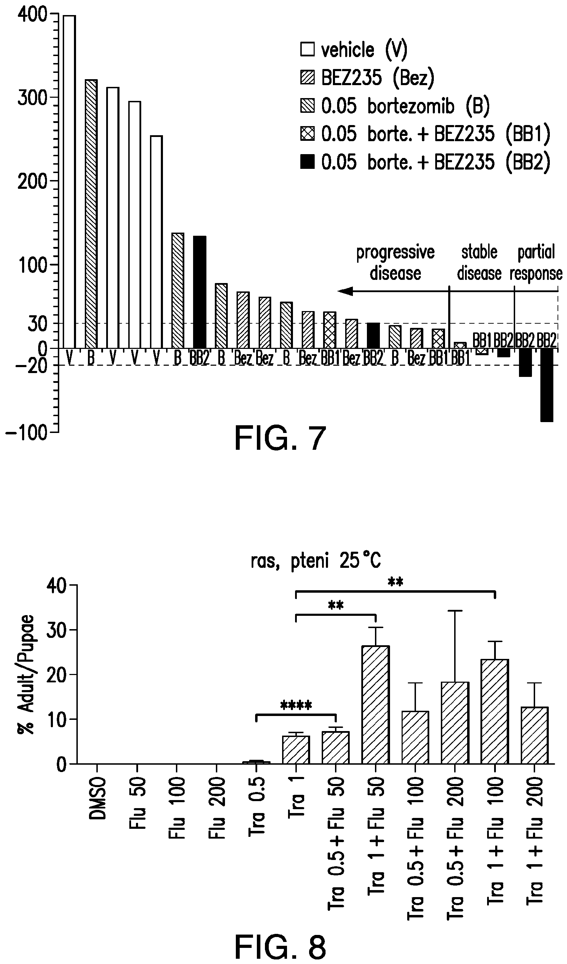

Using sub-combinations, the source of resistance for several drugs was mapped. These data were used to identify bortezomib-plus-BEZ235 as useful in reducing tumor progression in flies, mouse KRas-Pten-Apc transformed colon cell colografts and allografts, and DLD-1 cell culture and xenografts. All responded through the same biochemical mechanism that was identified in Drosophila (Figure 7).

Based upon this work, it was discovered that complex tumor models show greater resistance to single agents, and likely more closely model patients. Second, drug cocktails identified in empirical screens can be used to override this complexity and provide therapeutic efficacy. That is, complex tumors often require polypharmacological therapeutics.

### 8. EXAMPLE 3

This example describes the construction of fly avatars, specifically *Drosophila melanogaster* avatars, for human lung cancer and the use of those fly avatars to screen for drug therapeutics to treat lung cancer. Some of the techniques referenced in Section 6 were used to generate the fly avatars and to screen those fly avatars for drug therapeutics.

To model lung cancer a *Drosophila* generic avatar line was created expressing transgenes that directed expression of the oncogenic Ras1 isoform Ras1^{G12V} and the RNA interference-mediated knockdown of the PI3K pathway inhibitor PTEN (*PTENi*), addressing two pathways commonly activated in non-small cell lung cancer (NSCLC) patients. Transgenes were expressed specifically in the *Drosophila* trachea with the *btl-GAL4* driver *btl>Ras1, PTENi.* Animals died as larvae at 29°C. A library of 1192 FDA approved drugs was screened for drugs that rescued animals to pupariation. As shown in Figure 8, a drug combination comprising 50 µM fluvastatin with 0.5 µM trametinib significantly enhanced rescue of pupal lethality in the lung cancer avatars.

### 9. EXAMPLE 4

This example describes additional assays that may be conducted to assess the drug therapeutics identified using the fly avatars. In addition, this assay describes how the information produced from the fly avatar screens and potentially other assays may be used to provide a recommendation to a patient regarding treatment options for his/her cancer.

### Additional Assays

Candidate compounds identified using the fly avatars (see Sections 6, 7, and 8, *supra*) may be tested in mouse xenograft tumor models. Cell line-based xenograft models, patient tumor implants (patient-derived xenograft models), and/or stem cell based xenograft models may be used to test the candidate compounds identified using the fly avatars. In addition, swine toxicology studies may be conducted to assess toxicity. Novel drug cocktails may be tested for two weeks in pigs and liver enzymes/metabolites measured to determine both whole body toxicity and dosing.

### Treatment Recommendations

Based upon the results of the fly avatar screen (see Sections 6, 7, and 8, *supra*), potentially other additional assays noted above, and consultations with oncologists, a recommendation for treatment options are provided to patients. Those patients wishing to receive the recommended treatment option may be enrolled in a clinical trial comparing the recommended treatment option to the standard of care treatment.

Those skilled in the art will recognize or be able to ascertain, using no more than routine experimentation, many equivalents to the specific embodiments of the invention described herein. Such equivalents are intended to be encompassed by the following claims.

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims.

## Claims

1. A method for screening/selecting a therapeutically effective drug or combination of drugs for treating a subject diagnosed with cancer, wherein cancer cells from the subject exhibit increased activity of one or more oncogenes and/or reduced activity of one or more tumor suppressors, said method comprising:
providing a transgenic *Drosophila* larva avatar having an ortholog of an oncogene or a tumor suppressor of cancer cells of a subject diagnosed with cancer, the ortholog designed to be under inducible control such that, upon induction of the ortholog through an external factor, the activity of the ortholog is altered to correspond to the increased activity of the oncogene or reduced activity of the tumor suppressor, wherein the activity of the ortholog is in a larval tissue of the transgenic Drosophila larva avatar selected from the group consisting of the hindgut, midgut, imaginal disc, fat body, nephrocyte, heart, trachea, thorax, abdomen and malphigian tubules and the activity of the ortholog prevents the *Drosophila* larva avatar from surviving to pupation; and
screening a library of drugs, wherein said screening comprises feeding a culture of the transgenic *Drosophila* larva avatar a drug or combination of drugs,
wherein a therapeutically effective drug or combination of drugs is selected from the library of drugs when the transgenic *Drosophila* larva avatar survives to pupation.

2. The method of claim 1, wherein the cancer is:
(i) a solid tumor; or
(ii) selected from the group consisting of bladder cancer, bone cancer, breast cancer, cervical cancer, colorectal cancer, esophageal cancer, gallbladder cancer, head and neck cancer, kidney cancer, lung cancer, ocular cancer, oral cancer, ovarian cancer, pancreatic cancer, pelvic cancer, penile cancer, prostate cancer, skin cancer, throat cancer, thyroid cancer, such as medullary thyroid cancer, and uterine cancer.

3. The method of any one of claims 1 or 2, wherein the oncogene is selected from the group consisting of abl, Af4/hrx, akt-2, alk, alk/npm, amll, amll/mtg8, axl, bcl-2, bcl-3, bcl-6, bcr/abl, c-myc, dbl, dek/can E2A/pbxl, EGFR, enl/hrx, erg/TLS, erbB, erbB-2, ets-1, ews/fli-1, fins, fos, fps, gli, gsp, Her2/neu, hoxll, hst, IL-3, int-2, jun, kit, KS3, K-sam, Lbc, lck, lmol, lmo2, Lmyc, lyl-1, lyt-10/C alphal, mas, mdm-2, mll, mos, mtg8/amll, myb, MYHl 1/CBFB, neu, N-myc, ost, pax-5, pbxl/E2A, pim-1, PRAD-1, raf, RAR/PML, rash, rasK, rasN, rel/nrg, ret, rhoml, rhom2, ros, ski, sis, set/can, src, tall, tal2, tan-I, Tiaml, TSC2, and trk.

4. The method of any one of claims 1 to 3, wherein the activity of the oncogene is increased by genetically engineering the *Drosophila* to express cDNAs encoding the orthologs, which expression of the cDNAs are regulated by exposure to the external factor.

5. The method of any one of claims 1 to 4, wherein the tumor suppressor is selected from the group consisting of APC, BRCAI, BRCA2, CDKN2A, DCC, DPC4 (SMAD4), MADR2/N18 (SMAD2), MENI, MTSI, NFI, NF2, p53, PTEN, Rb, VHL, WRN, and WTI.

6. The method of any one of claims 1 to 5, wherein the tumor suppressor is inhibited by genetically engineering the *Drosophila* larva to express interfering RNAs which expression of the interfering RNAs are regulated by exposure to the external factor.

7. The method of any one of claims 1 to 6, wherein:
(i) the activity of the oncogene is increased by at least 10%, 20%, 30%, 40%, 50%, 60%, 70%, 80%, 90%, 100%, 125%, 150%, 175%, 200%, 250%, or 300% or more as compared to the activity of a corresponding oncogene in a non-modified *Drosophila;* or
(ii) the oncogene is overexpressed in the *Drosophila* larva avatar as compared to a corresponding oncogene in a non-modified *Drosophila.*

8. The method of any one of claims 1 to 7, wherein:
(i) the activity the tumor suppressor is decreased by at least 10%, 20%, 30%, 40%, 50%, 60%, 70%, 80%, 90%, 100%, 125%, 150%, 175%, 200%, 250%, or 300% or more as compared to the activity of a corresponding tumor suppressor in a non-modified *Drosophila.*
(ii) the tumor suppressor is underexpressed in the *Drosophila* larva avatar as compared to the expression of a corresponding tumor suppressor in a non-modified *Drosophila;* or
(iii) expression of the tumor suppressor is inhibited in the *Drosophila* larva avatar.

9. The method of any one of claims 1 to 8, wherein:
(i) the subject has failed standard of care therapy;
(ii) there is no standard of care therapy for the cancer; or
(iii) the subject is a treatment naive subject.

10. The method of any one of claims 1 to 9, wherein the treatment of the *Drosophila* larva avatar with the drug or combination of drug increases survival of the avatar to adulthood.

11. The method of any one of claims 1 to 10, wherein the larval tissue that is necessary for survival is a hindgut or an imaginal disc.

12. The method of any one of claims 1 to 11, wherein the drug is, or the combination of drugs comprises an anti-cancer therapy, preferably wherein the anti-cancer therapy is selected from the group consisting of alkylating agents, anthracyclines, cytoskeletal disruptors, hi stone deacetylase inhibitors, inhibitors of topoisomerase I, inhibitors of topoisomerase II, kinase inhibitors, nucleotide analogs and precursor analogs, peptide antibiotics, platinum-based agents, retinoids, and vinca alkaloids and derivatives thereof.

13. The method of any one of claims 1 to 12, wherein the external factor is temperature.

14. The method of any one of claims 1 to 13, wherein the subject is a human.

## Patentansprüche

1. Verfahren zum Screenen/Auswählen eines therapeutisch wirksamen Arzneimittels oder einer Kombination von Arzneimitteln zum Behandeln eines Subjekts, bei dem Krebs diagnostiziert wurde, wobei Krebszellen von dem Subjekt eine erhöhte Aktivität eines oder mehrerer Onkogene und/oder eine verringerte Aktivität eines oder mehrerer Tumorsuppressoren vorweisen, wobei das Verfahren Folgendes umfasst:
Bereitstellen eines transgenen Drosophila-Larven-Avatars, der ein Ortholog eines Onkogens oder eines Tumorsuppressors von Krebszellen eines Subjekts, bei dem Krebs diagnostiziert wurde, aufweist, wobei das Ortholog aufgebaut ist, um unter induzierbarer Kontrolle derart zu sein, dass bei Induktion des Orthologs durch einen externen Faktor, die Aktivität des Orthologs verändert wird, um der erhöhten Aktivität des Onkogens oder der verringerten Aktivität des Tumorsuppressors zu entsprechen, wobei die Aktivität des Orthologs in einem Larvengewebe des transgenen Drosophila-Larven-Avatars vorliegt, das aus der Gruppe ausgewählt ist, die aus Enddarm, Mitteldarm, Imaginalscheibe, Fettkörper, Nephrozyten, Herz, Luftröhre, Thorax, Abdomen und malphigische Tubuli besteht und die Aktivität des Orthologs den *Drosophila*-Larven-Avatar davon abhält, bis zu einer Verpuppung zu überleben; und
Screenen einer Arzneimittelbibliothek, wobei das Screenen ein Füttern einer Kultur des transgenen Drosophila-Larven-Avatars mit einem Arzneimittel oder einer Kombination von Arzneimitteln umfasst,
wobei ein therapeutisch wirksames Arzneimittel oder eine Kombination von Arzneimitteln aus der Arzneimittelbibliothek ausgewählt wird, wenn der transgene *Drosophila*-Larven-Avatar bis zu der Verpuppung überlebt.

2. Verfahren nach Anspruch 1, wobei der Krebs Folgendes ist:
(i) ein solider Tumor; oder
(ii) aus der Gruppe ausgewählt ist, die aus Blasenkrebs, Knochenkrebs, Brustkrebs, Gebärmutterhalskrebs, Dickdarmkrebs, Speiseröhrenkrebs, Gallenblasenkrebs, Kopf-Hals-Krebs, Nierenkrebs, Lungenkrebs, Augenkrebs, Mundkrebs, Eierstockkrebs, Bauchspeicheldrüsenkrebs, Beckenkrebs, Peniskrebs, Prostatakrebs, Hautkrebs, Kehlkopfkrebs, Schilddrüsenkrebs, wie etwa medullärer Schilddrüsenkrebs, und Gebärmutterkrebs besteht.

3. Verfahren nach einem der Ansprüche 1 oder 2, wobei das Onkogen aus der Gruppe ausgewählt ist, die aus abl, Af4/hrx, akt-2, alk, alk/npm, amll, amll/mtg8, axl, bcl-2, bcl-3, bcl-6, bcr/abl, c-myc, dbl, dek/can E2A/pbxl, EGFR, enl/hrx, erg/TLS, erbB, erbB-2, ets-1, ews/fli-1, fins, fos, fps, gli, gsp, Her2/neu, hoxll, hst, IL-3, int-2, jun, kit, KS3, K-sam, Lbc, Ick, Imol, Imo2, Lmyc, lyl-1, lyt-10/C alphal, mas, mdm-2, mll, mos, mtg8/amll, myb, MYHI 1/CBFB, neu, N-myc, ost, pax-5, pbxl/E2A, pim-1, PRAD-1, raf, RAR/PML, rash, rasK, rasN, rel/nrg, ret, rhoml, rhom2, ros, ski, sis, set/can, src, tall, tal2, tan-I, Tiaml, TSC2 und trk besteht.

4. Verfahren nach einem der Ansprüche 1 bis 3, wobei die Aktivität des Onkogens mittels gentechnischen Manipulierens der *Drosophila* erhöht wird, um cDNAs zu exprimieren, die die Orthologe codieren, wobei die Expression der cDNAs mittels Exposition gegenüber dem externen Faktor reguliert wird.

5. Verfahren nach einem der Ansprüche 1 bis 4, wobei der Tumorsuppressor aus der Gruppe ausgewählt ist, die aus APC, BRCAI, BRCA2, CDKN2A, DCC, DPC4 (SMAD4), MADR2/N18 (SMAD2), MENI, MTSI, NFI, NF2, p53, PTEN, Rb, VHL, WRN und WTI besteht.

6. Verfahren nach einem der Ansprüche 1 bis 5, wobei der Tumorsuppressor mittels gentechnischen Manipulierens der *Drosophila*-Larve inhibiert wird, um interferierende RNAs zu exprimieren, wobei die Expression der interferierenden RNAs mittels Exposition gegenüber dem externen Faktor reguliert wird.

7. Verfahren nach einem der Ansprüche 1 bis 6, wobei:
(i) die Aktivität des Onkogens um wenigstens 10 %, 20 %, 30 %, 40 %, 50 %, 60 %, 70 %, 80 %, 90 %, 100 %, 125 %, 150 %, 175 %, 200 %, 250 % oder 300 % oder mehr im Vergleich zu der Aktivität eines entsprechenden Onkogens in einer nicht modifizierten *Drosophila* erhöht ist; oder
(ii) das Onkogen in dem *Drosophila*-Larven-Avatar im Vergleich zu einem entsprechenden Onkogen in einer nicht modifizierten *Drosophila* überexprimiert ist.

8. Verfahren nach einem der Ansprüche 1 bis 7, wobei:
(i) die Aktivität des Tumorsuppressors um wenigstens 10 %, 20 %, 30 %, 40 %, 50 %, 60 %, 70 %, 80 %, 90 %, 100 %, 125 %, 150 %, 175 %, 200 %, 250 % oder 300 % oder mehr im Vergleich zu der Aktivität eines entsprechenden Tumorsuppressors in einer nicht modifizierten *Drosophila* vermindert ist.
(ii) der Tumorsuppressor in dem *Drosophila*-Larven-Avatar im Vergleich zu der Expression eines entsprechenden Tumorsuppressors in einer nicht modifizierten *Drosophila* unterexprimiert ist; oder
(iii) die Expression des Tumorsuppressors in dem *Drosophila*-Larven-Avatar inhibiert wird.

9. Verfahren nach einem der Ansprüche 1 bis 8, wobei:
(i) das Subjekt die Standardtherapie nicht bestanden hat;
(ii) es keine Standardtherapie für den Krebs gibt; oder
(iii) das Subjekt ein behandlungsnaives Subjekt ist.

10. Verfahren nach einem der Ansprüche 1 bis 9, wobei die Behandlung des *Drosophila-*Larven-Avatars mit dem Arzneimittel oder einer Kombination von Arzneimitteln das Überleben des Avatars bis zum Erwachsenenalter erhöht.

11. Verfahren nach einem der Ansprüche 1 bis 10, wobei das überlebensnotwendige Larvengewebe ein Enddarm oder eine Imaginalscheibe ist.

12. Verfahren nach einem der Ansprüche 1 bis 11, wobei das Arzneimittel oder die Kombination von Arzneimitteln eine Anti-Krebs-Therapie umfasst, bevorzugt wobei die Anti-Krebs-Therapie aus der Gruppe ausgewählt ist, die aus Alkylierungsmitteln, Anthracyclinen, Cytoskelett-Disruptoren, Hi-Stone-Deacetylase-Inhibitoren, Inhibitoren von Topoisomerase I, Inhibitoren von Topoisomerase II, Kinase-Inhibitoren, Nukleotid-Analoga und Vorläufer-Analoga, Peptid-Antibiotika, Mitteln auf Platinbasis, Retinoiden und Vinca-Alkaloiden und Derivaten davon besteht.

13. Verfahren nach einem der Ansprüche 1 bis 12, wobei der externe Faktor Temperatur ist.

14. Verfahren nach einem der Ansprüche 1 bis 13, wobei das Subjekt ein Mensch ist.

## Revendications

1. Procédé de criblage/sélection d'un médicament ou d'une combinaison de médicaments efficace sur le plan thérapeutique pour le traitement d'un sujet diagnostiqué comme étant atteint d'un cancer, dans lequel les cellules cancéreuses du sujet présentent une activité accrue d'un ou de plusieurs oncogènes et/ou une activité réduite d'un ou de plusieurs suppresseurs de tumeurs, ledit procédé comprenant :
la fourniture d'un avatar de larve de *Drosophila* transgénique ayant un orthologue d'un oncogène ou un suppresseur de tumeur de cellules cancéreuses d'un sujet diagnostiqué comme étant atteint d'un cancer, l'orthologue étant conçu pour être sous contrôle inductible de telle sorte que, lors de l'induction de l'orthologue par un facteur externe, l'activité de l'orthologue est modifié pour correspondre à l'activité accrue de l'oncogène ou à l'activité réduite du suppresseur de tumeur, l'activité de l'orthologue étant dans un tissu larvaire de l'avatar de larve de drosophile transgénique sélectionné dans le groupe constitué par l'intestin postérieur, l'intestin moyen, le disque imaginai, le corps gras, le néphrocyte, le cœur, la trachée, le thorax, l'abdomen et les tubes de Malpighi et l'activité de l'orthologue empêche l'avatar de la larve de *Drosophila* de survivre jusqu'à la nymphose ; et
le criblage d'une bibliothèque de médicaments, dans lequel ledit criblage comprend l'alimentation d'une culture de l'avatar de larve de *Drosophila* transgénique avec un médicament ou une combinaison de médicaments,
dans lequel un médicament ou une combinaison de médicaments efficace sur le plan thérapeutique est sélectionné(e) dans la bibliothèque de médicaments lorsque l'avatar de larve de *Drosophila* transgénique survit jusqu'à la nymphose.

2. Procédé selon la revendication 1, le cancer étant :
(i) une tumeur solide ; ou
(ii) sélectionné dans le groupe comprenant le cancer de la vessie, le cancer des os, le cancer du sein, le cancer du col de l'utérus, le cancer colorectal, le cancer de l'œsophage, le cancer de la vésicule biliaire, le cancer de la tête et du cou, le cancer du rein, le cancer des poumon, le cancer des yeux, le cancer de la bouche, le cancer des ovaires, le cancer du pancréas, le cancer pelvien, le cancer du pénis, le cancer de la prostate, le cancer de la peau, le cancer de la gorge, le cancer de la thyroïde, tel que le cancer médullaire de la thyroïde et le cancer de l'utérus.

3. Procédé selon l'une quelconque des revendications 1 ou 2, dans lequel l'oncogène est choisi dans le groupe consistant en abl, Af4/hrx, akt-2, alk, alk/npm, amll, amll/mtg8, axl, bcl-2, bcl-3, bcl-6, bcr/abl, c-myc, dbl, dek/can E2A/pbxl, EGFR, enl/hrx, erg/TLS, erbB, erbB-2, ets-1, ews/fli-1, fins, fos, fps, gli, gsp, Her2/neu, hoxll, hst, IL-3, int-2, jun, kit, KS3, K-sam, Lbc, Ick, Imol, Imo2, Lmyc, Iyl-1, lyt-10/C alphal, mas, mdm-2, mll, mos, mtg8/amll, myb, MYHI 1/CBFB, neu, N-myc, ost, pax-5, pbxl/E2A, pim-1, PRAD-1, raf, RAR/PML, rash, rasK, rasN, rel/nrg, ret, rhoml, rhom2, ros, ski, sis, set/can, src, tall, tal2, tan-I, Tiaml, TSC2, et trk.

4. Procédé selon l'une quelconque des revendications 1 à 3, dans lequel l'activité de l'oncogène est augmentée en modifiant génétiquement la *Drosophila* pour exprimer des ADNc codant les orthologues, laquelle expression des ADNc est régulée par exposition au facteur externe.

5. Procédé selon l'une quelconque des revendications 1 à 4, dans lequel le suppresseur de tumeur est choisi dans le groupe consistant en APC, BRCAI, BRCA2, CDKN2A, DCC, DPC4 (SMAD4), MADR2/N18 (SMAD2), MENI, MTSI, NFI, NF2, p53, PTEN, Rb, VHL, WRN, et WTI.

6. Procédé selon l'une quelconque des revendications 1 à 5, dans lequel le suppresseur de tumeur est inhibé par la modification génétique de la larve de *Drosophila* pour exprimer des ARN interférents, laquelle expression des ARN interférents est régulée par exposition au facteur externe.

7. Procédé selon l'une quelconque des revendications 1 à 6, dans lequel :
(i) l'activité de l'oncogène est augmentée d'au moins 10 %, 20 %, 30 %, 40 %, 50 %, 60 %, 70 %, 80 %, 90 %, 100 %, 125 %, 150 %, 175 %, 200 %, 250 % ou 300 % ou plus par rapport à l'activité d'un oncogène correspondant dans une *Drosophila* non modifiée ; ou
(ii) l'oncogène est surexprimé dans l'avatar de larve de *Drosophila* par rapport à un oncogène correspondant dans une *Drosophila* non modifiée.

8. Procédé selon l'une quelconque des revendications 1 à 7, dans lequel :
(i) l'activité du suppresseur de tumeur est diminuée d'au moins 10 %, 20 %, 30 %, 40 %, 50 %, 60 %, 70 %, 80 %, 90 %, 100 %, 125 %, 150 %, 175 %, 200 %, 250 % ou 300 % ou plus par rapport à l'activité d'un suppresseur de tumeur correspondant dans une *Drosophila* non modifiée.
(ii) le suppresseur de tumeur est sous-exprimé dans l'avatar de larve de *Drosophila* par rapport à l'expression d'un suppresseur de tumeur correspondant dans une *Drosophila* non modifiée ; ou
(iii)l'expression du suppresseur de tumeur est inhibée dans l'avatar de larve de *Drosophila.*

9. Procédé selon l'une quelconque des revendications 1 à 8, dans lequel :
(i) le sujet a échoué à la traitement de soins standard ;
(ii) il n'y a pas de traitement de soins standard pour le cancer ; ou
(iii)le sujet est un sujet n'ayant jamais reçu de traitement.

10. Procédé selon l'une quelconque des revendications 1 à 9, dans lequel le traitement de l'avatar de larve de *Drosophila* avec le médicament ou la combinaison de médicaments augmente la survie de l'avatar jusqu'à l'âge adulte.

11. Procédé selon l'une quelconque des revendications 1 à 10, dans lequel le tissu larvaire nécessaire à la survie est un intestin postérieur ou un disque imaginal.

12. Procédé selon l'une quelconque des revendications 1 à 11, dans lequel le médicament est, ou la combinaison de médicaments comprend, un traitement anticancéreux, de préférence dans laquelle le traitement anticancéreux est choisi dans le groupe constitué d'agents alkylants, d'anthracyclines, de perturbateurs du cytosquelette, d'inhibiteurs des histone-désacétylases, d'inhibiteurs de la topoisomérase I, d'inhibiteurs de la topoisomérase II, d'inhibiteurs de kinases, d'analogues de nucléotides et d'analogues précurseurs, d'antibiotiques peptidiques, d'agents à base de platine, de rétinoïdes et d'alcaloïdes de la pervenche et de leurs dérivés.

13. Procédé selon l'une quelconque des revendications 1 à 12, dans lequel le facteur externe est la température.

14. Procédé selon l'une quelconque des revendications 1 à 13, dans lequel le sujet est un humain.
